# EUROPEAN PATENT SPECIFICATION

(11) **EP 2 689 053 B1**
(45) Date of publication and mention of the grant of the patent: **14.11.2018**
(21) Application number: 12760047.6
(22) Date of filing: 23.03.2012
(51) Int. Cl.: G01N 33/566, G01N 33/574, G01N 33/68, C07K 1/04, C07K 7/06, C40B 40/04, C40B 40/10, C40B 60/12

(54) **DIAGNOSTIC AND TREATMENT METHODS USING A LIGAND LIBRARY**
DIAGNOSE- UND BEHANDLUNGSVERFAHREN UNTER VERWENDUNG EINER LIGANDENBIBLIOTHEK
MÉTHODES DE DIAGNOSTIQUE ET DE TRAITEMENT UTILISANT UNE BANQUE DE LIGANDS

(30) Priority: 24.03.2011 US 201161467256 P; 31.05.2011 US 201161491717 P; 06.01.2012 US 201261583881 P
(43) Date of publication of application: 29.01.2014
(73) Proprietor: OPKO Pharmaceuticals, LLC, Miami, FL 33137 (US)
(72) Inventor: MOOLA, Muralidhar, Reddy, Jupiter FL 33458 (US); SCHILKE, Jessica, Jupiter FL 33458 (US)
(74) Representative: Pearl Cohen Zedek Latzer Baratz UK LLP
(86) International application number: PCT/US2012/030223
(87) International publication number: WO 2012/129457

(56) References cited:
- WO-A1-2009/077763
- US-A- 5 834 318
- US-A1- 2002 018 778
- US-A1- 2007 054 847
- US-A1- 2007 105 152
- US-A1- 2009 111 102
- US-A1- 2009 246 124
- US-A1- 2009 246 124
- US-A1- 2010 266 610
- US-A1- 2010 303 805
- US-A1- 2010 303 805
- US-A1- 2010 303 835
- US-B2- 7 354 584
- US-B2- 7 776 313
- MURALIDHAR REDDY M ET AL: "Identification of Candidate IgG Biomarkers for Alzheimer's Disease via Combinatorial Library Screening", CELL, CELL PRESS, US, vol. 144, no. 1, 19 November 2010 (2010-11-19), pages 132-142, XP028151095, ISSN: 0092-8674, DOI: 10.1016/J.CELL.2010.11.054 [retrieved on 2010-12-03]
- GARSKE ADAM L ET AL: "SIRT1 top 40 hits: Use of one-bead, one-compound acetyl-peptide libraries and quantum dots to probe deacetylase specificity", BIOCHEMISTRY, AMERICAN CHEMICAL SOCIETY, US, vol. 45, no. 1, 1 January 2006 (2006-01-01), pages 94-101, XP002429823, ISSN: 0006-2960, DOI: 10.1021/BI052015L
- JOO SANG HOON ET AL: "Synthesis and screening of support-bound combinatorial peptide libraries with free C-termini: determination of the sequence specificity of PDZ domains", BIOCHEMISTRY, AMERICAN CHEMICAL SOCIETY, US, vol. 47, no. 9, 4 March 2008 (2008-03-04), pages 3061-3072, XP002563256, ISSN: 0006-2960, DOI: 10.1021/BI7023628 [retrieved on 2008-01-31]
- RYAN L. HARD ET AL: "HDAC6 and Ubp-M BUZ Domains Recognize Specific C-Terminal Sequences of Proteins", BIOCHEMISTRY, vol. 49, no. 50, 21 December 2010 (2010-12-21), pages 10737-10746, XP55178545, ISSN: 0006-2960, DOI: 10.1021/bi101014s
- GARCIA-ARRARAS ET AL.: 'Enteric nerve fibers of holothurians are recognized by an antibody to acetylated alpha-tubulin.' NEUROSCIENCE LETTERS vol. 157, 23 July 1993, pages 153 - 156, XP026039552
- NEUMANN ET AL.: 'SPR-based Fragment Screening: Advantages and Applications.' CURRENT TOPICS IN MEDICINAL CHEMISTRY vol. 7, 2007, pages 1630 - 1642, XP055124034

## Description

### FIELD OF THE INVENTION

The invention relates to diagnostic methods using a ligand library and active compounds derived therefrom. Specifically, the invention relates to using a ligand library to find ligands that are used to determine disease biomarker profiles and to diagnose or detect a drug induced response, including drug adverse reaction, side effects, drug resistance, and therapeutic efficacy.

### BACKGROUND OF THE INVENTION

Small-molecule microarrays are becoming increasingly important tools in combinatorial chemistry. The arrays are generally produced by first synthesizing a combinatorial library on a suitable bead resin, separating the beads into the wells of microtiter plates, and then releasing the compounds from the beads. The resulting solutions then are spotted robotically onto a chemically modified glass slide such that the library-derived molecule is attached covalently to the surface. Alternatively, methods exist for the synthesis of certain classes of compounds *in situ* on the array surface. By far the most common application of small-molecule microarrays has been as a versatile platform for library screening, usually with the goal of identifying small-molecule ligands for a given protein of interest.

M. Muralidhar Reddy et al., 2011, Cell 144(1): 132-142 discloses the identification of candidate IgG biomarkers for Alzheimer's Disease *via* combinatorial library screening. U.S. patent publication 2007/0003954 discloses protein and antibody profiling using small molecule microarrays. The application discloses ligands, which bind to ligand binding moieties wherein the ligands are arranged in arrays of synthetic molecules, which are used to screen for biomarkers and molecular fingerprints. The specific arrays described therein include, for example, a peptoid microarray having 7680 different compounds bound to the array. In that disclosure, bead based libraries were utilized as the initial means to make peptoids which were then transferred to microarrays with addressable locations on the microarray to screen biological fluids. The screening results in a unique pattern or molecular fingerprint on the array for any-particular protein in a complex biological mixture. U.S. patent application 2010/0303805, discloses certain peptoids and diagnostic arrays useful in screening biological fluids for biomarkers associated with central nervous system disorders. The specific monomers disclosed therein utilized to form the arrays therein may also be utilized in the new screening methodology of the present invention provided the libraries are enlarged to a much greater number of beads/peptoids or beads/ligands, e.g., between greater than 100K to 150MM.

The inventors of the instant application have found that significantly larger bead based libraries (relative to microarray based screens for antibody biomarkers or bead based screens for cells) can, under the right conditions, be used to directly screen complex biological samples to find drug response associated biomarkers as well as a significantly larger pool of ligands which bind to such ligand-binding moieties. This significantly larger pool includes a significantly improved number of high affinity ligands that serve as diagnostic tools as well as potential therapeutics.

### SUMMARY OF THE INVENTION

The present invention provides a method for diagnosing a drug induced response in a subject, the method comprising:
screening a ligand library against a biological sample previously obtained from said subject; identifying binding characteristics of one or more markers in said sample with one or more ligands in the library; and determining whether said one or more markers are associated with said drug induced response,
wherein the library comprises a compound of formula I, 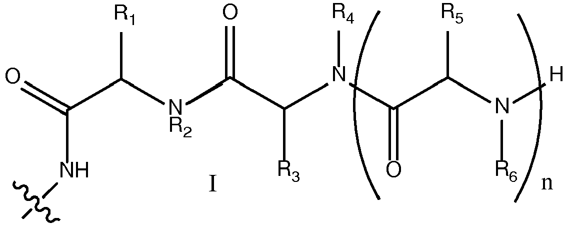 wherein
   R₁ is -(C₁-C₆)SCH_{3;}
   R₂ is H; and
   R₃-R₆ are independently selected from the groups consisting of H, -C₁-C₆alkyl, -C₁-C₆alkylSCH₃, -C₀-C₆alkylC₂-C₆alkenyl, -C₀-C₆alkyl C₂-C₆alkynyl, -C₁-C₆ COOH, -C₁-C6alkylOH, -C₁-C₆alkylN(R)₂, -C₃-C₈cycloalkyl, -C₁-C₆alkylaryl, -C₁-C₆alkylheteroaryl, - C₁-C₆a1ky1NC(O)C₁-C₆alkyl, -C₁-C₆alkylcycloamide wherein any of the aryl or heteroaryl groups may be independently substituted with -OH, Cl, F, Br, -OCH₃, -SO₂NH₂ or -O-CH₂-O-,
wherein said drug induced response is an adverse reaction to said drug, a side effect of said drug, or a resistance to said drug, thereby diagnosing said drug induced response in said subject, and
wherein n is 3-11.

In an embodiment of this method, the library comprise a compound of formula I, 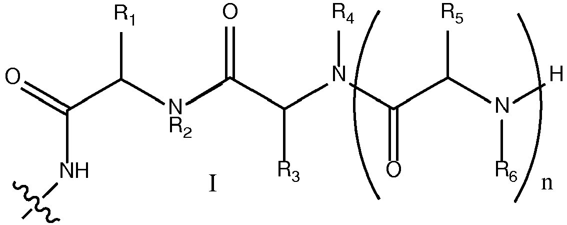 wherein the compounds are produced by a process which comprises use of a reactant selected from the group consisting of
(A) furfurylamine; 3,4-dimethoxyethanolamine; benzylamine; N-(2-aminoethyl)acetamide; N-(3-aminopropyl)-2-pyrrolidinone; ethanolamine; glycine; diaminobutane; allylamine; piperonylamine; methylbenzylamine; isobutylamine;4-(2-aminoethyl)benzenesulfonamide or cyclohexylamine; or
(B) methoxyethylamine; piperonylamine; cyclohexylamine; diaminobutane; methylbenzylamine; isobutylamine; furfurylamine or 4-(2-aminoethyl)benzenesulfonamide; or
(C) furfurylamine, ethanolamine; glycine; diaminobutane; allylamine; piperonylamine; methylbenzylamine; isobutylamine or 4-(2-aminoethyl)benzenesulfonamide; or
(D) furfurylamine, N-(2-aminoethyl)acetamide; N-(3-aminoethyl)-2-pyrrolidinone; ethanolamine; glycine; diaminobutane; allylamine; piperonylamine; methylbenzylamine; isobutylamine; 4-(2-aminoethyl)benzenesulfonamide; or
(E) cysteine, glycine, allylamine, ethanolamine, isobutylamine, methylbenzylamine, piperonylamine, methionine, cyclohexylamine, 3,4-dimethoxyphenethylamine, benzylamine, N-(2-aminoethyl)acetamide, N-(3-aminopropyl)-2-pyrrolidone, 4-(2-aminoethyl)benzenesulfonamide and furfurylamine; and
   wherein,
   R₁ is -(C₁-C₆)SCH₃;
   R₂ is H;
   R₃ and R₅ are independently selected from the groups consisting of H, -C₁-C₆alkyl, - C₁-C₆alkylSCH₃, -C₀-C₆alkylC₂-C₆alkenyl, -C₀-C₆alkyl C₂-C₆alkynyl, -C₁-C₆ COOH, -C₁-C₆alkylOH, -C₁-C₆alkylN(R)₂, -C₃-C₈cycloalkyl, -C₁-C₆alkylaryl, -C₁-C₆alkylheteroaryl, - C₁-C₆alkylNC(O)C₁-C₆alkyl, -C₁-C₆alkylcycloamide wherein any of the aryl or heteroaryl groups may be independently substituted with -OH, Cl, F, Br, -OCH₃, -SO₂NH₂ or -O-CH₂-O-;
   R₄ is selected from the group consisting of furfuryl or -(C₁-C₆alkyl)NR₇R₈, and
   R6 is selected from the group consisting of H, 1-yl-allyl, 1-yl-2-hydroxyethyl, isobutyl, 1-yl-n-butylamine, methylbenzyl, piperonyl, cyclohexyl, 1-yl-2-(3,4-dimethoxyphenyl)ethyl, benzyl, 1-yl-2-(acetamide)ethyl, 1-yl-3-2-pyrrolidinone, 1-yl-2-(4-benzenesulfonamide)ethyl or furfuryl.

In an embodiment of the method, the library comprises a compound having a formula Ia 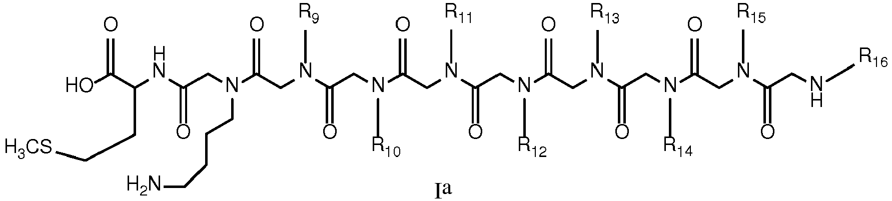 wherein the compound is selected from the group consisting of a compound of formula Ia wherein,
(a) R₉ is n-butylamine; R₁₀ is 1-yl-n-butylamine; R₁₁ is piperonyl; R₁₂ is methylbenzyl; R₁₃ is piperonyl; R₁₄ is methylbenzyl; R₁₅ is 1-yl-2-(4(benzenesulfonamide)ethyl and R₁₆ is 1-yl-2-methoxyethyl;
(b) R₉ is 1-yl-n-butylamine; R₁₀ is 1-yl-2-(4(benzenesulfonamide)ethyl; R₁₁ is 1-yl-n-butylamine; R₁₂ is cyclohexyl; R₁₃ is 1-yl-2-methoxyethyl; R₁₄ is 1-yl-2,2-dimethylethyl (isobutyl); R₁₅ is 1-yl-2-(4(benzenesulfonamide)ethyl and R₁₆ is methylbenzyl;
(c) R₉ is 1-yl-n-butylamine; R₁₀ is 1-yl-n-butylamine; R₁₁ is piperonyl; R₁₂ is 1-yl-2-(4(benzenesulfonamide)ethyl; R₁₃ is 1-yl-n-butylamine; R₁₄ is methylbenzyl; R₁₅ is methylbenzyl and R₁₆ is cyclohexyl;
(d) R₉ is piperonyl; R₁₀ is 1-yl-n-butylamine; R₁₁ is isobutyl; R₁₂ is 1-yl-2-(4(benzenesulfonamide)ethyl; R₁₃ is methylbenzyl; R₁₄ is cyclohexyl; R₁₅ is isobutyl and R₁₆ is 1-yl-n-butylamine;
(e) R₉ is 1-yl-n-butylamine; R₁₀ is 1-yl-n-butylamine; R₁₁ is methylbenzyl; R₁₂ is 1-yl-2-methoxyethyl; R₁₃ is cyclohexyl; R₁₄ is cyclohexyl; R₁₅ is methylbenzyl and R₁₆ is piperonyl;
(f) R₉ is piperonyl; R₁₀ is 1-yl-n-butylamine; R₁₁ is isopropyl; R₁₂ is isopropyl; R₁₃ is 1-yl-2-methoxyethyl; R₁₄ is cyclohexyl; R₁₅ is 1-yl-2-(4(benzenesulfonamide)ethyl and R₁₆ is 1-yl-2-(4(benzenesulfonamide)ethyl;
(g) R₉ is 1-yl-n-butylamine; R₁₀ is 1-yl-n-butylamine; R₁₁ is piperonyl; R₁₂ is methylbenzyl; R₁₃ is piperonyl; R₁₄ is methylbenzyl; R₁₅ is 1-yl-2-(4(benzenesulfonamide)ethyl and R₁₆ is cyclohexyl;
(h) R₉ is 1-yl-n-butylamine; R₁₀ is 1-yl-n-butylamine; R₁₁ is methylbenzyl; R₁₂ is 1-yl-2-methoxyethyl; R₁₃ is cyclohexyl; R₁₄ is cyclohexyl; R₁₅ is methylbenzyl and R₁₆ is piperonyl;
(i) R₉ is 1-yl-n-butylamine; R₁₀ is methylbenzyl; R₁₁ is methylbenzyl; R₁₂ is 1-yl--n-butylamine; R₁₃ is 1-yl-n-butylamine; R¹⁴ is cyclohexyl; R¹⁵ is 1-yl-2-(4(benzenesulfonamide)ethyl and R¹⁶ is cyclohexyl;
(j) R₉ is 1-yl-2-(4(benzenesulfonamide)ethyl; R₁₀ is methylbenzyl; R₁₁ is methylbenzyl; R₁₂ is cyclohexyl; R₁₃ is 1-yl-n-butylamine; R₁₄ is methylbenzyl; R₁₅ is isobutyl and R₁₆ is 1-yl-n-butylamine;
(k) R₉ is 1-yl-2-methoxyethyl; R₁₀ is isobutyl; R₁₁ is 1-yl-2-(4(benzenesulfonamide)ethyl; R₁₂ is piperonyl; R₁₃ is 1-yl-n-butylamine; R₁₄ is cyclohexyl; R₁₅ is methylbenzyl and R₁₆ is 1-yl-2-methoxyethyl;
(1) R₉ is 1-yl-n-butylamine; R₁₀ is 1-yl-2-(4(benzenesulfonamide)ethyl; R₁₁ is 1-yl-n-butylamine; R₁₂ is cyclohexyl; R₁₃ is 1-yl-2-methoxyethyl; R₁₄ is isobutyl; R₁₅ is 1-yl-2-(4(benzenesulfonamide)ethyl and R₁₆ is methylbenzyl;
(m) R9 is 1-yl-2-methoxyethyl; RIO is 1-yl-n-butylamine; R11 is 1-yl-n-butylamine; R12 is 1-yl-2-methoxyethyl; R13 is methylbenzyl; R14 is 1-yl-n-butylamine; R15 is furfuryl and R16 is furfuryl;
(n) R9 is cyclohexyl; R10 is cyclohexyl; R11 is 1-yl-n-butylamine; R12 is furfuryl; R13 is 1-yl-2-methoxyethyl; R14 is 1-yl-2-methoxyethyl; R15 is 1-yl-2-(4(benzenesulfonamide)ethyl and R16 is furfuryl;
(o) R9 is 1-yl-n-butylamine; R10 is piperonyl; R11 is 1-yl-2-(4(benzenesulfonamide)ethyl; R12 is 1-yl-2-methoxyethyl; R13 is methylbenzyl; R14 is 1-yl-n-butylamine; R15 is 1-yl-2-methoxyethyl and R16 is methylbenzyl;
(p) R9 is cyclohexyl; R10 is cyclohexyl; R11 is piperonyl; R12 is 1-yl-n-butylamine; R13 is 1-yl-n-butylamine; R14 is 1-yl-n-butylamine; R15 is 1-yl-n-butylamine and R16 is isobutyl;
(q) R9 is piperonyl; RIO is 1-yl-n-butylamine; R11 is 1-yl-2-methoxyethyl; R12 is 1-yl-2-(4(benzenesulfonamide)ethyl; R13 is piperonyl; R14 is 1-yl-n-butylamine; R15 is methylbenzyl and R16 is methylbenzyl;
(r) R9 is methylbenzyl; RIO is methylbenzyl; R11 is methylbenzyl; R12 is 1-yl-n-butylamine; R13 is piperonyl; R14 is 1-yl-n-butylamine; R15 is piperonyl and R₁₆ is 1-yl-n-butylamine;
(s) R₉ is 1-yl-n-butylamine; R₁₀ is 1-yl-n-butylamine; R₁₁ is methylbenzyl; R₁₂ is 1-yl-n-butylamine; R₁₃ is methylbenzyl; R₁₄ is methylbenzyl; R₁₅ is 1-yl-2-methoxyethyl and R₁₆ is piperonyl;
(t) R₉ is methylbenzyl; R₁₀ is 1-yl-2-(4(benzenesulfonamide)ethyl; R₁₁ is 1-yl-n-butylamine; R₁₂ is 1-yl-2-(4(benzenesulfonamide)ethyl; R₁₃ is isobutyl; R₁₄ is 1-yl-n-butylamine; R₁₅ is methylbenzyl and R₁₆ is 1-yl-2-methoxyethyl;
(u) R₉ is 1-yl-2-methoxyethyl; R₁₀ is 1-yl-n-butylamine; R₁₁ is isobutyl; R₁₂ is isobutyl; R₁₃ is cyclohexyl; R₁₄ is 1-yl-n-butylamine; R₁₅ is 1-yl-2-(4(benzenesulfonamide)ethyl and R₁₆ is cyclohexyl;
(v) R₉ is isobutyl; R₁₀ is 1-yl-n-butylamine; R₁₁ is 1-yl-n-butylamine; R₁₂ is methylbenzyl; R₁₃ is 1-yl-n-butylamine; R₁₄ is 1-yl-n-butylamine; R₁₅ is piperonyl and R₁₆ is piperonyl;
(w) R₉ is 1-yl-2-(4(benzenesulfonamide)ethyl; R₁₀ is isobutyl; R₁₁ is methylbenzyl; R₁₂ is 1-yl-2-methoxyethyl; R₁₃ is 1-yl-2-(4(benzenesulfonamide)ethyl; R₁₄ is isobutyl; R₁₅ is 1-yl-2-methoxyethyl and R₁₆ is cyclohexyl;
(x) R₉ is furfuryl; R₁₀ is furfuryl; R₁₁ is piperonyl; R₁₂ is cyclohexyl; R₁₃ is piperonyl; R₁₄ is 1-yl-n-butylamine; R₁₅ is 1-yl-2-(4(benzenesulfonamide)ethyl and R₁₆ is cyclohexyl;
(y) R₉ is piperonyl; R₁₀ is piperonyl; R₁₁ is 1-yl-2-methoxyethyl; R₁₂ is 1-yl-2-methoxyethyl; R₁₃ is 1-yl-n-butylamine; R₁₄ is 1-yl-n-butylamine; R₁₅ is 1-yl-n-butylamine and R₁₆ is 1-yl-2-methoxyethyl;
(z) R₉ is piperonyl; R₁₀ is 1-yl-n-butylamine; R₁₁ is isobutyl; R₁₂ is 1-yl-2-(4(benzenesulfonamide)ethyl; R₁₃ is methylbenzyl; R₁₄ is cyclohexyl; R₁₅ is isobutyl and R₁₆ is 1-yl-n-butylamine;
(aa) R₉ is 1-yl-2-(4(benzenesulfonamide)ethyl; R₁₀ is 1-yl-2-(4(benzenesulfonamide)ethyl; R₁₁ is methylbenzyl; R₁₂ is methylbenzyl; R₁₃ is 1-yl-n-butylamine; R₁₄ is 1-yl-n-butylamine; R₁₅ is 1-yl-2-(4(benzenesulfonamide)ethyl and R₁₆ is 1-yl-2-(4(benzenesulfonamide)ethyl;
(bb) R₉ is 1-yl-n-butylamine; R₁₀ is 1-yl-2-methoxyethyl; R₁₁ is 1-yl-n-butylamine; R₁₂ is isobutyl; R₁₃ is cyclohexyl; R₁₄ is 1-yl-n-butylamine; R₁₅ is 1-yl-n-butylamine and R₁₆ is piperonyl;
(cc) R₉ is cyclohexyl; R₁₀ is methylbenzyl; R₁₁ is cyclohexyl; R₁₂ is piperonyl; R₁₃ is 1-yl-n-butylamine; R₁₄ is 1-yl-2-(4(benzenesulfonamide)ethyl; R₁₅ is 1-yl-n-butylamine and R₁₆ is 1-yl-2-methoxyethyl;
(dd) R₉ is 1-yl-2-methoxyethyl; R₁₀ is 1-yl-2-(4(benzenesulfonamide)ethyl; R₁₁ is 1-yl-n-butylamine; R₁₂ is 1-yl-2-methoxyethyl; R₁₃ is 1-yl-2-(4(benzenesulfonamide)ethyl; R₁₄ is 1-yl-2-methoxyethyl; R₁₅ is isobutyl and R₁₆ is cyclohexyl;
(ee) R₉ is 1-yl-2-methoxyethyl; R₁₀ is methylbenzyl; R₁₁ is 1-yl-n-butylamine; R₁₂ is 1-yl-n-butylamine; R₁₃ is piperonyl; R₁₄ is isobutyl; R₁₅ is 1-yl-2-(4(benzenesulfonamide)ethyl and R₁₆ is 1-yl-n-butylamine;
(ff) R₉ is 1-yl-n-butylamine; R₁₀ is methylbenzyl; R₁₁ is 1-yl-2-(4(benzenesulfonamide)ethyl; R₁₂ is methylbenzyl; R₁₃ is 1-yl-n-butylamine; R₁₄ is 1-yl-2-(4(benzenesulfonamide)ethyl; R₁₅ is 1-yl-2-(4(benzenesulfonamide)ethyl and R₁₆ is cyclohexyl;
(gg) R₉ is 1-yl-n-butylamine; R₁₀ is 1-yl-2-methoxyethyl; R₁₁ is 1-yl-n-butylamine; R₁₂ is 1-yl-2-(4(benzenesulfonamide)ethyl; R₁₃ is 1-yl-2-methoxyethyl; R₁₄ is 1-yl-2-methoxyethyl; R₁₅ is 1-yl-n-butylamine and R₁₆ is methylbenzyl;
(hh) R₉ is cyclohexyl; R₁₀ is cyclohexyl; R₁₁ is methylbenzyl; R₁₂ is 1-yl-n-butylamine; R₁₃ is methylbenzyl; R₁₄ is cyclohexyl; R₁₅ is methylbenzyl and R₁₆ is 1-yl-n-butylamine;
(ii) R₉ is 1-yl-n-butylamine; R₁₀ is furfuryl; R₁₁ is methylbenzyl; R₁₂ is 1-yl-2-(4(benzenesulfonamide)ethyl; R₁₃ is furfuryl; R₁₄ is cyclohexyl; R₁₅ is methylbenzyl and R₁₆ is cyclohexyl;
(jj) R₉ is 1-yl-n-butylamine; R₁₀ is methylbenzyl; R₁₁ is 1-yl-n-butylamine; R₁₂ is 1-yl-2-(4(benzenesulfonamide)ethyl; R₁₃ is 1-yl-2-methoxyethyl; R₁₄ is methylbenzyl; R₁₅ is 1-yl-2-methoxyethyl and R₁₆ is isobutyl;
(kk) R₉ is 1-yl-2-(4(benzenesulfonamide)ethyl; R₁₀ is 1-yl-n-butylamine; R₁₁ is 1-yl-n-butylamine; R₁₂ is methylbenzyl; R₁₃ is methylbenzyl; R₁₄ is cyclohexyl; R₁₅ is 1-yl-2-(4(benzenesulfonamide)ethyl and R₁₆ is methylbenzyl;
(11) R₉ is 1-yl-2-(4(benzenesulfonamide)ethyl; R₁₀ is 1-yl-2-(4(benzenesulfonamide)ethyl; R₁₁ is 1-yl-n-butylamine; R₁₂ is 1-yl-n-butylamine; R₁₃ is methylbenzyl; R₁₄ is 1-yl-n-butylamine; R₁₅ is methylbenzyl and R₁₆ is 1-yl-n-butylamine;
(mm)R₉ is 1-yl-n-butylamine; R₁₀ is 1-yl-2-(4(benzenesulfonamide)ethyl; R₁₁ is 1-yl-2-(4(benzenesulfonamide)ethyl; R₁₂ is 1-yl-2-methoxyethyl; R₁₃ is 1-yl-n-butylamine; R₁₄ is cyclohexyl; R₁₅ is 1-yl-2-(4(benzenesulfonamide)ethyl and R₁₆ is methylbenzyl;
(nn) R₉ is 1-yl-n-butylamine; R₁₀ is 1-yl-n-butylamine; R₁₁ is piperonyl; R₁₂ is 1-yl-2-(4(benzenesulfonamide)ethyl; R₁₃ is 1-yl-n-butylamine; R₁₄ is methylbenzyl; R₁₅ is methylbenzyl and R₁₆ is cyclohexyl;
(oo) R₉ is 1-yl-2-(4(benzenesulfonamide)ethyl; R₁₀ is methylbenzyl; R₁₁ is methylbenzyl; R₁₂ is 1-yl-n-butylamine; R₁₃ is methylbenzyl; R₁₄ is piperonyl; R₁₅ is 1-yl-n-butylamine and R₁₆ is 1-yl-2-methoxyethyl;
(pp) R₉ is piperonyl; R₁₀ is 1-yl-n-butylamine; R₁₁ is 1-yl-n-butylamine; R₁₂ is methylamine; R₁₃ is piperonyl; R₁₄ is 1-yl-n-butylamine; R₁₅ is piperonyl and R₁₆ is 1-yl-2-methoxyethyl;
(qq) R₉ is cyclohexyl; R₁₀ is cyclohexyl; R₁₁ is furfuryl; R₁₂ is 1-yl-2-methoxyethyl; R₁₃ is isobutyl; R₁₄ is cyclohexyl; R₁₅ is methylbenzyl and R₁₆ is methylbenzyl;
(rr) R₉ is piperonyl; R₁₀ is 1-yl-n-butylamine; R₁₁ is isobutyl; R₁₂ is isobutyl; R₁₃ is 1-yl-2-methoxyethyl; R₁₄ is cyclohexyl; R₁₅ is 1-yl-2-(4(benzenesulfonamide)ethyl and R₁₆ is 1-yl-2-(4(benzenesulfonamide)ethyl;
(ss) R₉ is cyclohexyl; R₁₀ is cyclohexyl; R₁₁ is 1-yl-n-butylamine; R₁₂ is methylbenzyl; R₁₃ is 1-yl-n-butylamine; R₁₄ is methylbenzyl; R₁₅ is cyclohexyl and R₁₆ is piperonyl;
and pharmaceutically acceptable salts thereof.

In another embodiment of the method, the library comprises a compound of formula II: 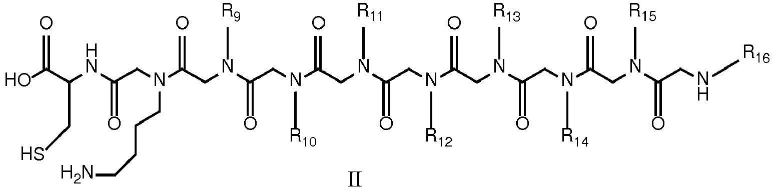 wherein the compound is selected from the group consisting of a compound of formula II wherein,
(a) R₉ is n-butylamine; R₁₀ is 1-yl-n-butylamine; R₁₁ is piperonyl; R₁₂ is methylbenzyl; R₁₃ is piperonyl; R₁₄ is methylbenzyl; R₁₅ is 1-yl-2-(4(benzenesulfonamide)ethyl and R₁₆ is 1-yl-2-methoxyethyl;
(b) R₉ is 1-yl-n-butylamine; R₁₀ is 1-yl-2-(4(benzenesulfonamide)ethyl; R₁₁ is 1-yl-n-butylamine; R₁₂ is cyclohexyl; R₁₃ is 1-yl-2-methoxyethyl; R₁₄ is 1-yl-2,2-dimethylethyl (isobutyl); R₁₅ is 1-yl-2-(4(benzenesulfonamide)ethyl and R₁₆ is methylbenzyl;
(c) R₉ is 1-yl-n-butylamine; R₁₀ is 1-yl-n-butylamine; R₁₁ is piperonyl; R₁₂ is 1-yl-2-(4(benzenesulfonamide)ethyl; R₁₃ is 1-yl-n-butylamine; R₁₄ is methylbenzyl; R₁₅ is methylbenzyl and R₁₆ is cyclohexyl;
(d) R₉ is piperonyl; R₁₀ is 1-yl-n-butylamine; R₁₁ is isobutyl; R₁₂ is 1-yl-2-(4(benzenesulfonamide)ethyl; R₁₃ is methylbenzyl; R₁₄ is cyclohexyl; R₁₅ is isobutyl and R₁₆ is 1-yl-n-butylamine;
(e) R₉ is 1-yl-n-butylamine; R₁₀ is 1-yl-n-butylamine; R₁₁ is methylbenzyl; R₁₂ is 1-yl-2-methoxyethyl; R₁₃ is cyclohexyl; R₁₄ is cyclohexyl; R₁₅ is methylbenzyl and R₁₆ is piperonyl;
(f) R₉ is piperonyl; R₁₀ is 1-yl-n-butylamine; R₁₁ is isopropyl; R₁₂ is isopropyl; R₁₃ is 1-yl-2-methoxyethyl; R₁₄ is cyclohexyl; R₁₅ is 1-yl-2-(4(benzenesulfonamide)ethyl and R₁₆ is 1-yl-2-(4(benzenesulfonamide)ethyl;
(g) R₉ is 1-yl-n-butylamine; R₁₀ is 1-yl-n-butylamine; R₁₁ is piperonyl; R₁₂ is methylbenzyl; R₁₃ is piperonyl; R₁₄ is methylbenzyl; R₁₅ is 1-yl-2-(4(benzenesulfonamide)ethyl and R₁₆ is cyclohexyl;
(h) R₉ is 1-yl-n-butylamine; R₁₀ is 1-yl-n-butylamine; R₁₁ is methylbenzyl; R₁₂ is 1-yl-2-methoxyethyl; R₁₃ is cyclohexyl; R₁₄ is cyclohexyl; R₁₅ is methylbenzyl and R₁₆ is piperonyl;
(i) R₉ is 1-yl-n-butylamine; R₁₀ is methylbenzyl; R₁₁ is methylbenzyl; R₁₂ is 1-yl--n-butylamine; R₁₃ is 1-yl-n-butylamine; R¹⁴ is cyclohexyl; R¹⁵ is 1-yl-2-(4(benzenesulfonamide)ethyl and R¹⁶ is cyclohexyl;
(j) R₉ is 1-yl-2-(4(benzenesulfonamide)ethyl; R₁₀ is methylbenzyl; R₁₁ is methylbenzyl; R₁₂ is cyclohexyl; R₁₃ is 1-yl-n-butylamine; R₁₄ is methylbenzyl; R₁₅ is isobutyl and R₁₆ is 1-yl-n-butylamine;
(k) R₉ is 1-yl-2-methoxyethyl; R₁₀ is isobutyl; R₁₁ is 1-yl-2-(4(benzenesulfonamide)ethyl; R₁₂ is piperonyl; R₁₃ is 1-yl-n-butylamine; R₁₄ is cyclohexyl; R₁₅ is methylbenzyl and R₁₆ is 1-yl-2-methoxyethyl;
(1) R₉ is 1-yl-n-butylamine; R₁₀ is 1-yl-2-(4(benzenesulfonamide)ethyl; R₁₁ is 1-yl-n-butylamine; R₁₂ is cyclohexyl; R₁₃ is 1-yl-2-methoxyethyl; R₁₄ is isobutyl; R₁₅ is 1-yl-2-(4(benzenesulfonamide)ethyl and R₁₆ is methylbenzyl;
(m) R₉ is 1-yl-2-methoxyethyl; R₁₀ is 1-yl-n-butylamine; R₁₁ is 1-yl-n-butylamine; R₁₂ is 1-yl-2-methoxyethyl; R₁₃ is methylbenzyl; R₁₄ is 1-yl-n-butylamine; R₁₅ is furfuryl and R₁₆ is furfuryl;
(n) R₉ is cyclohexyl; R₁₀ is cyclohexyl; R₁₁ is 1-yl-n-butylamine; R₁₂ is furfuryl; R₁₃ is 1-yl-2-methoxyethyl; R₁₄ is 1-yl-2-methoxyethyl; R₁₅ is 1-yl-2-(4(benzenesulfonamide)ethyl and R₁₆ is furfuryl;
(o) R₉ is 1-yl-n-butylamine; R₁₀ is piperonyl; R₁₁ is 1-yl-2-(4(benzenesulfonamide)ethyl; R₁₂ is 1-yl-2-methoxyethyl; R₁₃ is methylbenzyl; R₁₄ is 1-yl-n-butylamine; R₁₅ is 1-yl-2-methoxyethyl and R₁₆ is methylbenzyl;
(p) R₉ is cyclohexyl; R₁₀ is cyclohexyl; R₁₁ is piperonyl; R₁₂ is 1-yl-n-butylamine; R₁₃ is 1-yl-n-butylamine; R₁₄ is 1-yl-n-butylamine; R₁₅ is 1-yl-n-butylamine and R₁₆ is isobutyl;
(q) R₉ is piperonyl; R₁₀ is 1-yl-n-butylamine; R₁₁ is 1-yl-2-methoxyethyl; R₁₂ is 1-yl-2-(4(benzenesulfonamide)ethyl; R₁₃ is piperonyl; R₁₄ is 1-yl-n-butylamine; R₁₅ is methylbenzyl and R₁₆ is methylbenzyl;
(r) R₉ is methylbenzyl; R₁₀ is methylbenzyl; R₁₁ is methylbenzyl; R₁₂ is 1-yl-n-butylamine; R₁₃ is piperonyl; R₁₄ is 1-yl-n-butylamine; R₁₅ is piperonyl and R₁₆ is 1-yl-n-butylamine;
(s) R₉ is 1-yl-n-butylamine; R₁₀ is 1-yl-n-butylamine; R₁₁ is methylbenzyl; R₁₂ is 1-yl-n-butylamine; R₁₃ is methylbenzyl; R₁₄ is methylbenzyl; R₁₅ is 1-yl-2-methoxyethyl and R₁₆ is piperonyl;
(t) R₉ is methylbenzyl; R₁₀ is 1-yl-2-(4(benzenesulfonamide)ethyl; R₁₁ is 1-yl-n-butylamine; R₁₂ is 1-yl-2-(4(benzenesulfonamide)ethyl; R₁₃ is isobutyl; R₁₄ is 1-yl-n-butylamine; R₁₅ is methylbenzyl and R₁₆ is 1-yl-2-methoxyethyl;
(u) R₉ is 1-yl-2-methoxyethyl; R₁₀ is 1-yl-n-butylamine; R₁₁ is isobutyl; R₁₂ is isobutyl; R₁₃ is cyclohexyl; R₁₄ is 1-yl-n-butylamine; R₁₅ is 1-yl-2-(4(benzenesulfonamide)ethyl and R₁₆ is cyclohexyl;
(v) R₉ is isobutyl; R₁₀ is 1-yl-n-butylamine; R₁₁ is 1-yl-n-butylamine; R₁₂ is methylbenzyl; R₁₃ is 1-yl-n-butylamine; R₁₄ is 1-yl-n-butylamine; R₁₅ is piperonyl and R₁₆ is piperonyl;
(w) R₉ is 1-yl-2-(4(benzenesulfonamide)ethyl; R₁₀ is isobutyl; R₁₁ is methylbenzyl; R₁₂ is 1-yl-2-methoxyethyl; R₁₃ is 1-yl-2-(4(benzenesulfonamide)ethyl; R₁₄ is isobutyl; R₁₅ is 1-yl-2-methoxyethyl and R₁₆ is cyclohexyl;
(x) R₉ is furfuryl; R₁₀ is furfuryl; R₁₁ is piperonyl; R₁₂ is cyclohexyl; R₁₃ is piperonyl; R₁₄ is 1-yl-n-butylamine; R₁₅ is 1-yl-2-(4(benzenesulfonamide)ethyl and R₁₆ is cyclohexyl;
(y) R₉ is piperonyl; R₁₀ is piperonyl; R₁₁ is 1-yl-2-methoxyethyl; R₁₂ is 1-yl-2-methoxyethyl; R₁₃ is 1-yl-n-butylamine; R₁₄ is 1-yl-n-butylamine; R₁₅ is 1-yl-n-butylamine and R₁₆ is 1-yl-2-methoxyethyl;
(z) R₉ is piperonyl; R₁₀ is 1-yl-n-butylamine; R₁₁ is isobutyl; R₁₂ is 1-yl-2-(4(benzenesulfonamide)ethyl; R₁₃ is methylbenzyl; R₁₄ is cyclohexyl; R₁₅ is isobutyl and R₁₆ is 1-yl-n-butylamine;
(aa) R₉ is 1-yl-2-(4(benzenesulfonamide)ethyl; R₁₀ is 1-yl-2-(4(benzenesulfonamide)ethyl; R₁₁ is methylbenzyl; R₁₂ is methylbenzyl; R₁₃ is 1-yl-n-butylamine; R₁₄ is 1-yl-n-butylamine; R₁₅ is 1-yl-2-(4(benzenesulfonamide)ethyl and R₁₆ is 1-yl-2-(4(benzenesulfonamide)ethyl; (bb) R₉ is 1-yl-n-butylamine; R₁₀ is 1-yl-2-methoxyethyl; R₁₁ is 1-yl-n-butylamine; R₁₂ is isobutyl; R₁₃ is cyclohexyl; R₁₄ is 1-yl-n-butylamine; R₁₅ is 1-yl-n-butylamine and R₁₆ is piperonyl;
(cc) R₉ is cyclohexyl; R₁₀ is methylbenzyl; R₁₁ is cyclohexyl; R₁₂ is piperonyl; R₁₃ is 1-yl-n-butylamine; R₁₄ is 1-yl-2-(4(benzenesulfonamide)ethyl; R₁₅ is 1-yl-n-butylamine and R₁₆ is 1-yl-2-methoxyethyl;
(dd) R₉ is 1-yl-2-methoxyethyl; R₁₀ is 1-yl-2-(4(benzenesulfonamide)ethyl; R₁₁ is 1-yl-n-butylamine; R₁₂ is 1-yl-2-methoxyethyl; R₁₃ is 1-yl-2-(4(benzenesulfonamide)ethyl; R₁₄ is 1-yl-2-methoxyethyl; R₁₅ is isobutyl and R₁₆ is cyclohexyl;
(ee) R₉ is 1-yl-2-methoxyethyl; R₁₀ is methylbenzyl; R₁₁ is 1-yl-n-butylamine; R₁₂ is 1-yl-n-butylamine; R₁₃ is piperonyl; R₁₄ is isobutyl; R₁₅ is 1-yl-2-(4(benzenesulfonamide)ethyl and R₁₆ is 1-yl-n-butylamine;
(ff) R₉ is 1-yl-n-butylamine; R₁₀ is methylbenzyl; R₁₁ is 1-yl-2-(4(benzenesulfonamide)ethyl; R₁₂ is methylbenzyl; R₁₃ is 1-yl-n-butylamine; R₁₄ is 1-yl-2-(4(benzenesulfonamide)ethyl; R₁₅ is 1-yl-2-(4(benzenesulfonamide)ethyl and R₁₆ is cyclohexyl;
(gg) R₉ is 1-yl-n-butylamine; R₁₀ is 1-yl-2-methoxyethyl; R₁₁ is 1-yl-n-butylamine; R₁₂ is 1-yl-2-(4(benzenesulfonamide)ethyl; R₁₃ is 1-yl-2-methoxyethyl; R₁₄ is 1-yl-2-methoxyethyl; R₁₅ is 1-yl-n-butylamine and R₁₆ is methylbenzyl;
(hh) R₉ is cyclohexyl; R₁₀ is cyclohexyl; R₁₁ is methylbenzyl; R₁₂ is 1-yl-n-butylamine; R₁₃ is methylbenzyl; R₁₄ is cyclohexyl; R₁₅ is methylbenzyl and R₁₆ is 1-yl-n-butylamine;
(ii) R₉ is 1-yl-n-butylamine; R₁₀ is furfuryl; R₁₁ is methylbenzyl; R₁₂ is 1-yl-2-(4(benzenesulfonamide)ethyl; R₁₃ is furfuryl; R₁₄ is cyclohexyl; R₁₅ is methylbenzyl and R₁₆ is cyclohexyl;
(jj) R₉ is 1-yl-n-butylamine; R₁₀ is methylbenzyl; R₁₁ is 1-yl-n-butylamine; R₁₂ is 1-yl-2-(4(benzenesulfonamide)ethyl; R₁₃ is 1-yl-2-methoxyethyl; R₁₄ is methylbenzyl; R₁₅ is 1-yl-2-methoxyethyl and R₁₆ is isobutyl;
(kk) R₉ is 1-yl-2-(4(benzenesulfonamide)ethyl; R₁₀ is 1-yl-n-butylamine; R₁₁ is 1-yl-n-butylamine; R₁₂ is methylbenzyl; R₁₃ is methylbenzyl; R₁₄ is cyclohexyl; R₁₅ is 1-yl-2-(4(benzenesulfonamide)ethyl and R₁₆ is methylbenzyl;
(11) R₉ is 1-yl-2-(4(benzenesulfonamide)ethyl; R₁₀ is 1-yl-2-(4(benzenesulfonamide)ethyl; R₁₁ is 1-yl-n-butylamine; R₁₂ is 1-yl-n-butylamine; R₁₃ is methylbenzyl; R₁₄ is 1-yl-n-butylamine; R₁₅ is methylbenzyl and R₁₆ is 1-yl-n-butylamine;
(mm)R₉ is 1-yl-n-butylamine; R₁₀ is 1-yl-2-(4(benzenesulfonamide)ethyl; R₁₁ is 1-yl-2-(4(benzenesulfonamide)ethyl; R₁₂ is 1-yl-2-methoxyethyl; R₁₃ is 1-yl-n-butylamine; R₁₄ is cyclohexyl; R₁₅ is 1-yl-2-(4(benzenesulfonamide)ethyl and R₁₆ is methylbenzyl;
(nn) R₉ is 1-yl-n-butylamine; R₁₀ is 1-yl-n-butylamine; R₁₁ is piperonyl; R₁₂ is 1-yl-2-(4(benzenesulfonamide)ethyl; R₁₃ is 1-yl-n-butylamine; R₁₄ is methylbenzyl; R₁₅ is methylbenzyl and R₁₆ is cyclohexyl;
(oo) R₉ is 1-yl-2-(4(benzenesulfonamide)ethyl; R₁₀ is methylbenzyl; R₁₁ is methylbenzyl; R₁₂ is 1-yl-n-butylamine; R₁₃ is methylbenzyl; R₁₄ is piperonyl; R₁₅ is 1-yl-n-butylamine and R₁₆ is 1-yl-2-methoxyethyl;
(pp) R₉ is piperonyl; R₁₀ is 1-yl-n-butylamine; R₁₁ is 1-yl-n-butylamine; R₁₂ is methylamine; R₁₃ is piperonyl; R₁₄ is 1-yl-n-butylamine; R₁₅ is piperonyl and R₁₆ is 1-yl-2-methoxyethyl;
(qq) R₉ is cyclohexyl; R₁₀ is cyclohexyl; R₁₁ is furfuryl; R₁₂ is 1-yl-2-methoxyethyl; R₁₃ is isobutyl; R₁₄ is cyclohexyl; R₁₅ is methylbenzyl and R₁₆ is methylbenzyl;
(rr) R₉ is piperonyl; R₁₀ is 1-yl-n-butylamine; R₁₁ is isobutyl; R₁₂ is isobutyl; R₁₃ is 1-yl-2-methoxyethyl; R₁₄ is cyclohexyl; R₁₅ is 1-yl-2-(4(benzenesulfonamide)ethyl and R₁₆ is 1-yl-2-(4(benzenesulfonamide)ethyl;
(ss) R₉ is cyclohexyl; R₁₀ is cyclohexyl; R₁₁ is 1-yl-n-butylamine; R₁₂ is methylbenzyl; R₁₃ is 1-yl-n-butylamine; R₁₄ is methylbenzyl; R₁₅ is cyclohexyl and R₁₆ is piperonyl;
and pharmaceutically acceptable salts thereof.

In an embodiment of the method, the drug is at least one of the drugs listed in Table 1.

In an embodiment, the method further comprises the step of determining whether said subject is responsive or non-responsive to a treatment or therapy by said drug.

In an embodiment, the drug induced response is associated with a disease, or a stage of said disease, wherein said disease is optionally a cancer, an autoimmune disease, an inflammatory disease, an infectious disease, a neurodegenerative disease, or a cardiovascular disease.

In an embodiment, the cancer is breast cancer, lung cancer, prostate cancer, cervical cancer, head and neck cancer, testicular cancer, ovarian cancer, skin cancer, brain cancer, pancreatic cancer, liver cancer, stomach cancer, colon cancer, rectal cancer, esophageal cancer, lymphoma, or leukemia; the autoimmune disease is lupus, myestenia gravis, multiple sclerosis, narcolepsy, rheumatoid arthritis, nephritis, Chagas disease, scleroderma, or Sjogren's disease; the infectious disease is a result of infection with viruses, bacteria or fungi; or the neurodegenerative disease is Alzheimer's disease, dementia, or Creutzfeld-Jacob disease.

In an embodiment of the method, the ligand library comprises a plurality of ligands identified in an earlier initial screening or preselected based on known reactivity to said ligand binding markers.

In an embodiment of the method, the ligand library is a bead-based ligand library comprising a plurality of beads having ligands bound to the beads.

In an embodiment of the method, the screening is performed by an array, and wherein said array is optionally a microarray, a microscope slide, a plate, a chip, or a population of beads. In an embodiment, the array comprises a solid substrate having a surface; a plurality of ligands bound to said substrate, each of said ligand comprising an anchor segment stably bound to the substrate surface, a peptoid segment, and a linker segment connecting and separating the anchor and peptoid segments, or wherein said array comprises between about 1000 and 100,000 distinct ligands.

### BRIEF DESCRIPTION OF THE DRAWINGS

Figure 1 shows a basic chemical schematic of the preparation of a library of Tentagel. beads (KN1B) used to screen Alzheimer's serum samples. Figure IA shows starting from a polystyrene bead having an amino group as the reactant (a PEG or equivalent or alternative linker may be formed between the bead and the terminal amino group). Figure 1B shows the starting amino acid on the bead as methionine and which is then reacted to form, the compound shown in B. Figure 1C shows the submonomers (monomeric amines and haloacetic acids) utilized to form the oligomeric library of compounds.

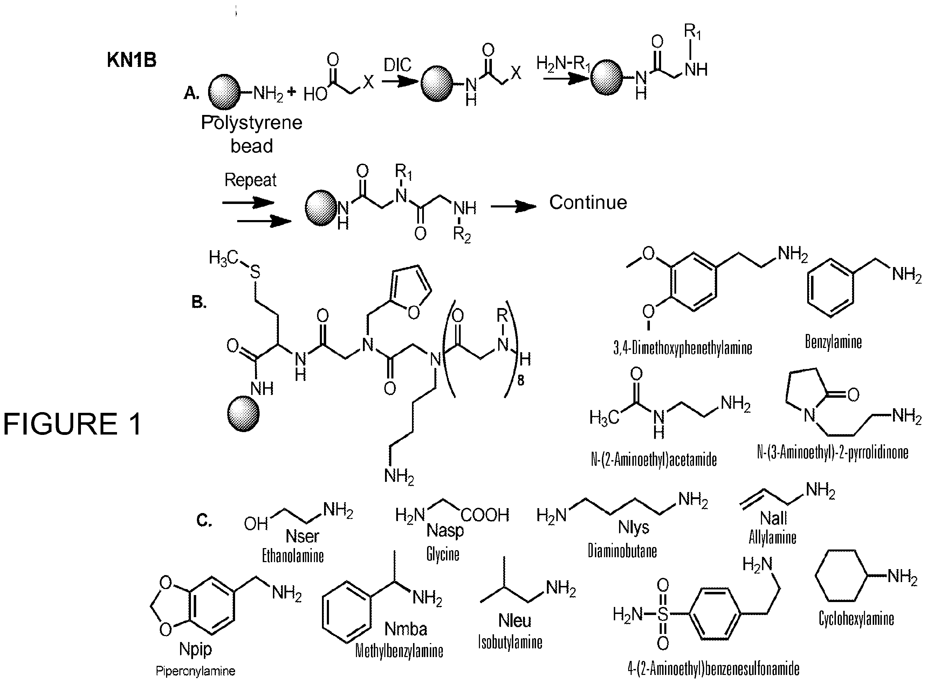

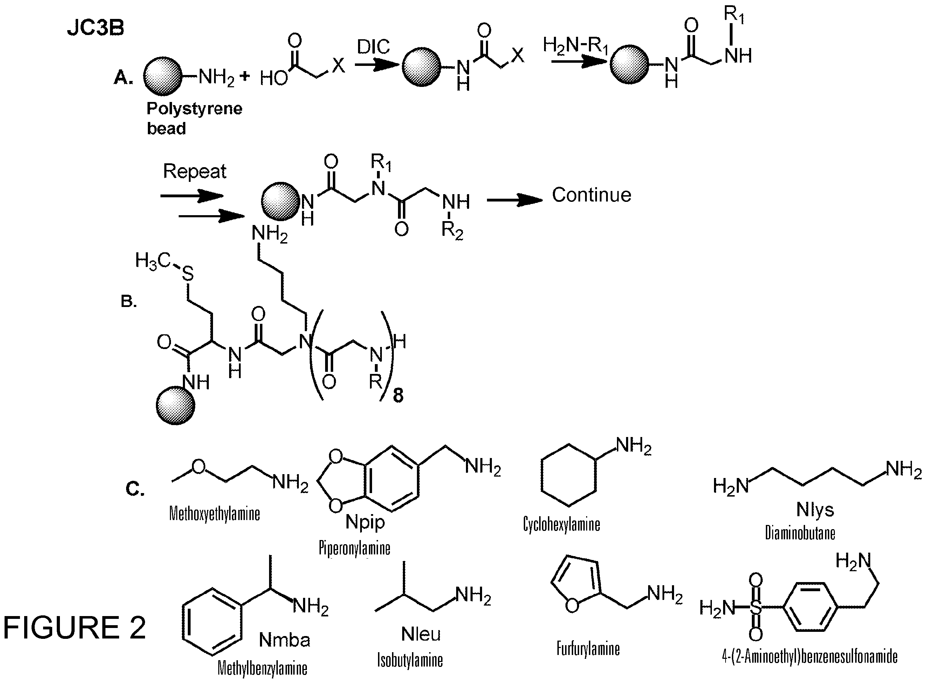

Figure 2 shows a basic chemical schematic of the preparation of a library of Tentagel beads (JC3B) also used to screen Alzheimer's serum samples. Figure 1A shows starting from a polystyrene bead having an amino group as the reactant (a PEG or equivalent or alternative linker may be formed between the bead and the terminal amino group). Figure 1B shows the starting amino acid on the bead as methionine and which is then reacted to form the compound shown in B, Figure 1C shows the submonomers (monomeric amines and haloacetic acids) utilized to form the oligomeric library of compounds. JC3B was also used to screen pancreatic cancer serum.
Figure 3 shows a basic chemical schematic of the preparation of a library of Tentagel beads (JC4B) used to screen Alzheimer's serum samples. Figure 1A shows starting from a polystyrene bead having an amino group as the reactant (a PEG or equivalent or alternative linker may be formed between the bead and the terminal amino group). Figure 1B shows the starting amino acid on the bead as methonine and which is then reacted to form the compound shown in B, Figure 1C shows the submonomers (monomenc amines and haloacetic acids) utilized to form the oligomeric library of compounds.

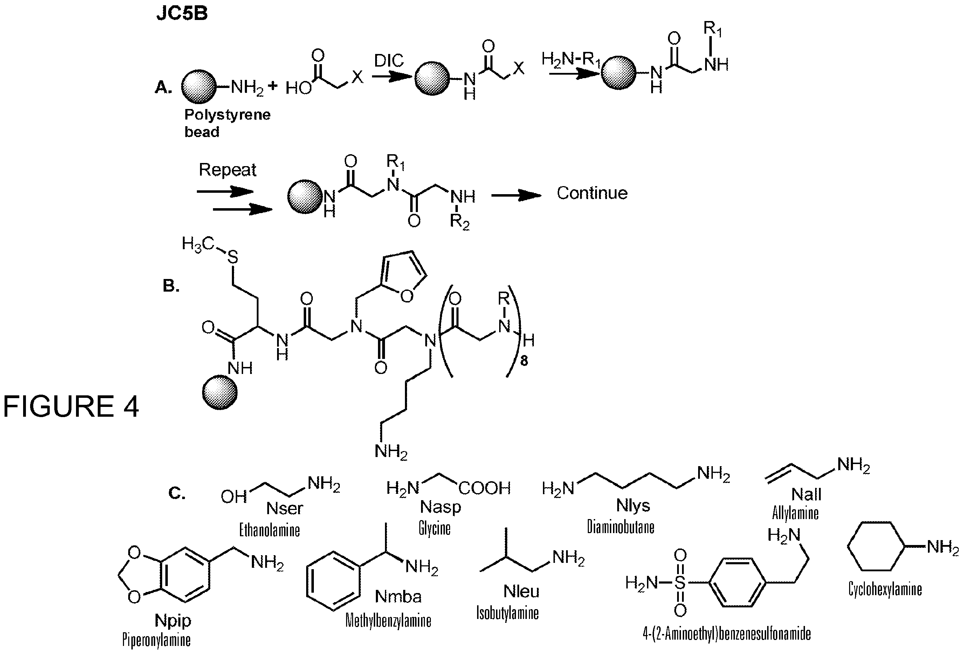

Figure 4 shows a basic chemical schematic of the preparation of a library of Tentagel beads (JC5B) used to screen Alzheimer's serum samples. Figure 1A shows starting from a polystyrene bead having an amino group as the reactant (a PEG or equivalent or alternative linker may be formed between die bead and the terminal amino group). Figure 1B shows the starting amino acid on the bead as methionine and which is then reacted to form the compound shown in B. Figure 1C shows the submonomers (monomeric amines and haloacetic acids) utilized to form the oligomeric library of compounds. JC5B monomers included Isobutylamine, 2-Methoxyethytamine, Diaminobutane, Furfurylamine, Cyclohexylamine, R-Methylbenzylamine, Piperonylamine and 4-(Aminoethyl) Benzenesulfonamide.
Figure 5 shows a basic chemical schematic of the preparation of a library of Tentagel beads (JC7B) used to screen serum samples. Figure 3A shows starting from a polystyrene bead having an amino group as the reactant (a PEG or equivalent or alternative linker may be formed between the bead and the terminal amino group). Figure 1B shows the starting amino acid on the bead as methionine and which is then reacted to form the compound shown in B. Figure IC shows the submonomers (monomeric amines and haloacetic acids) utilized to form the oligomeric library of compounds.
Figure 6 shows a schematic of the process of the invention to screen a complex biological sample using head based libraries of peptoid ligands.

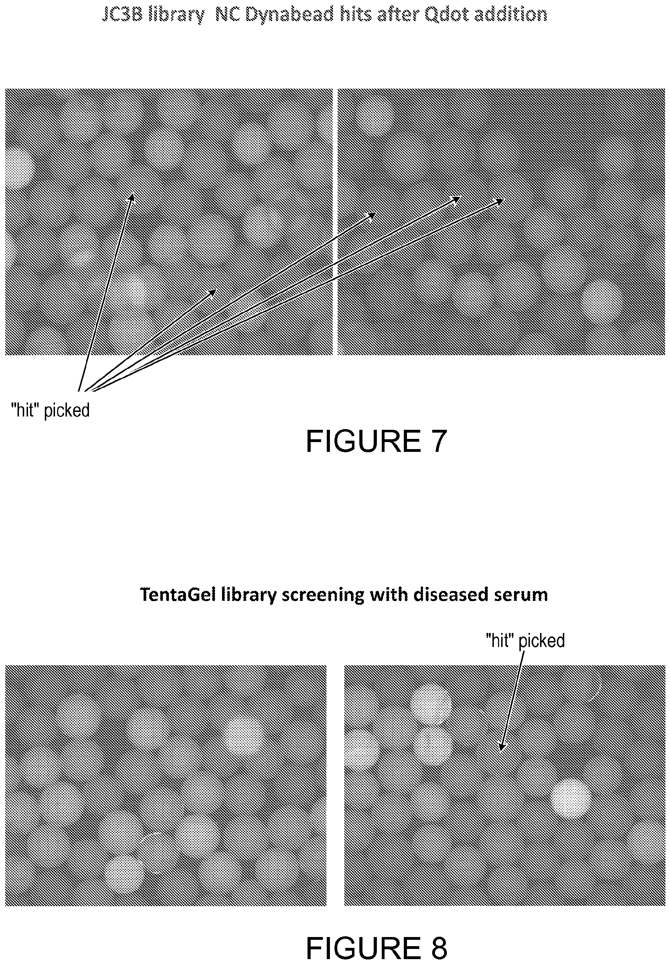

Figure 7 shows normal control (NC) Dynabead hits after QDot addition in a peptoid library (JOB) prepared to screen against an Alzheimer's normal control serum sample and Alzheimer's diseased serum sample. The hits were picked out and the remaining ligand bound beads were used in the disease based screen.
Figure 8 shows the Tentagel bead screening of diseased serum from Alzheimer's patient blood samples after the NC hits were removed. The hits are shown in red, which is the Qdot secondary antibody bound to the disease associated biomarker (antibody) in the serum which is bound to a peptoid linked through a PEG linker to the bead.

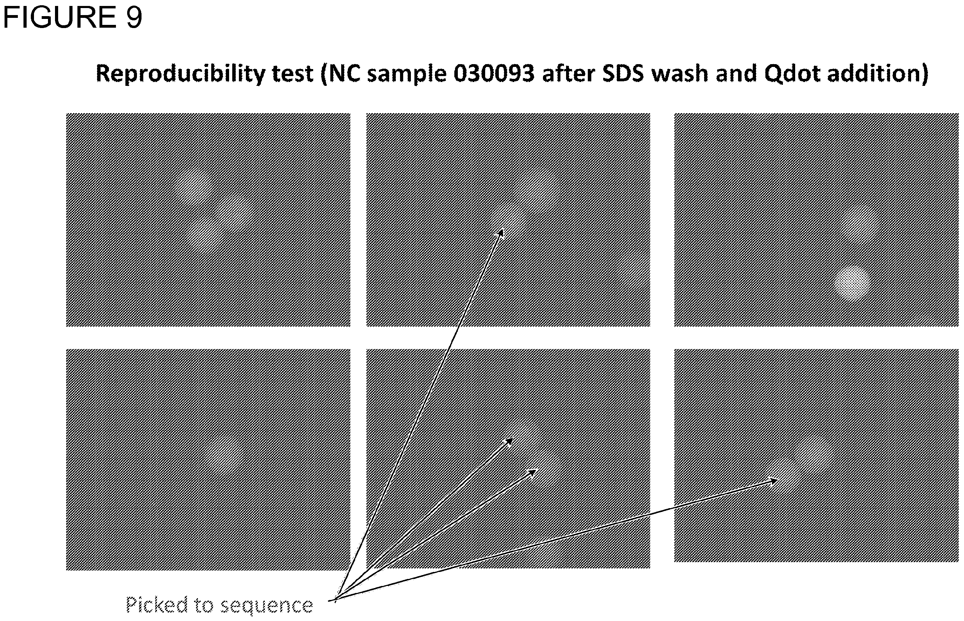

Figure 9 shows a reproducibility test which uses a normal control sample (NC 030093) after SDS wash and QDOT addition. The arrow shows which NC peptoid hits were picked to sequence.

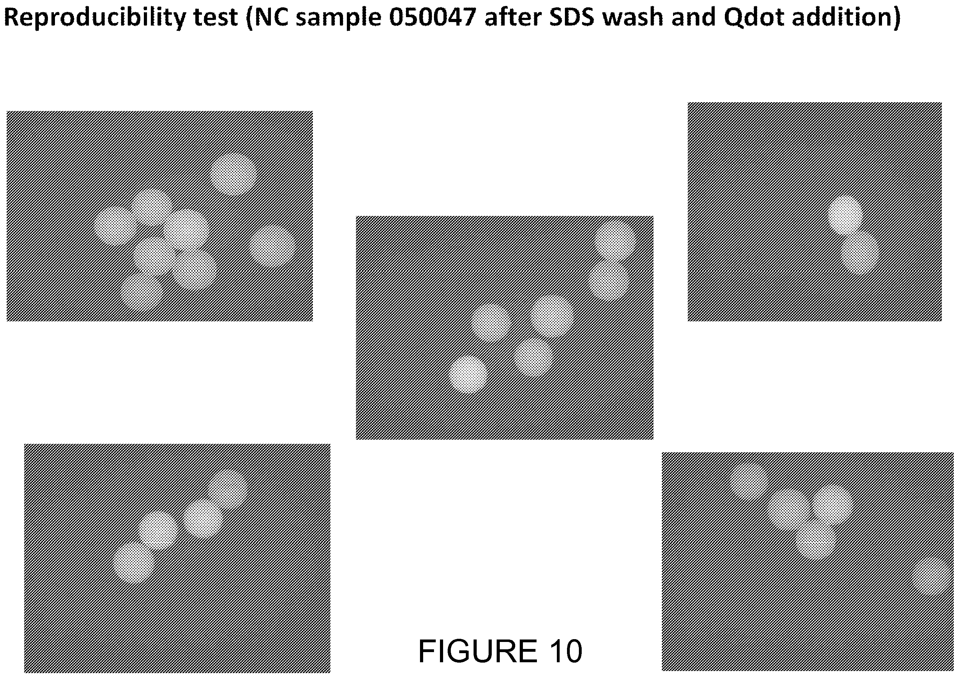

Figure 10 shows a reproducibility test which uses a normal control sample (NC 050047) after SDS wash and QDOT addition.

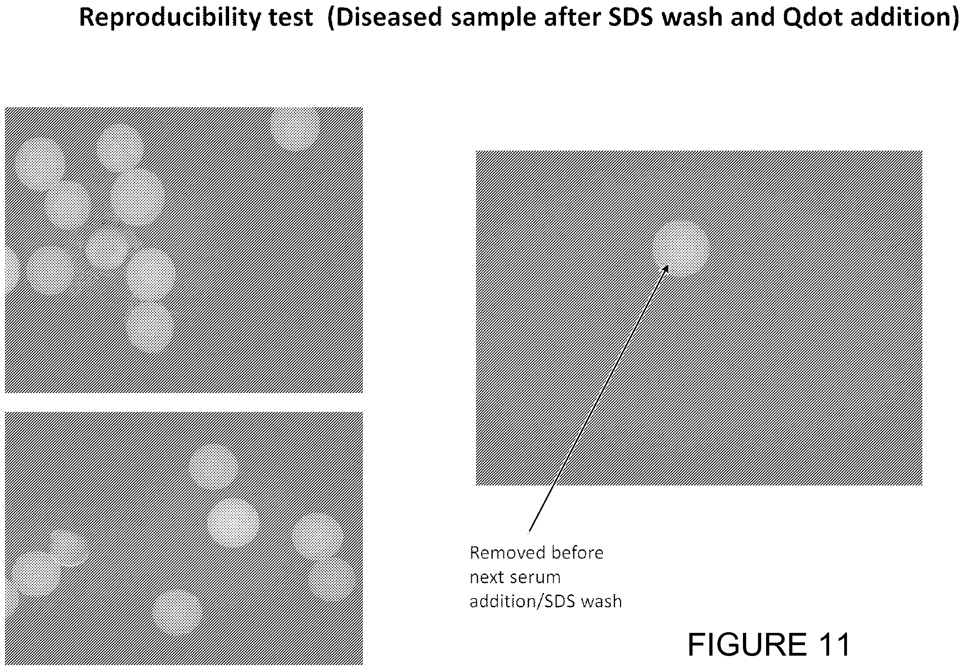

Figure 11 shows a reproducibility test which uses a diseased sample after SDS wash and QDOT addition.

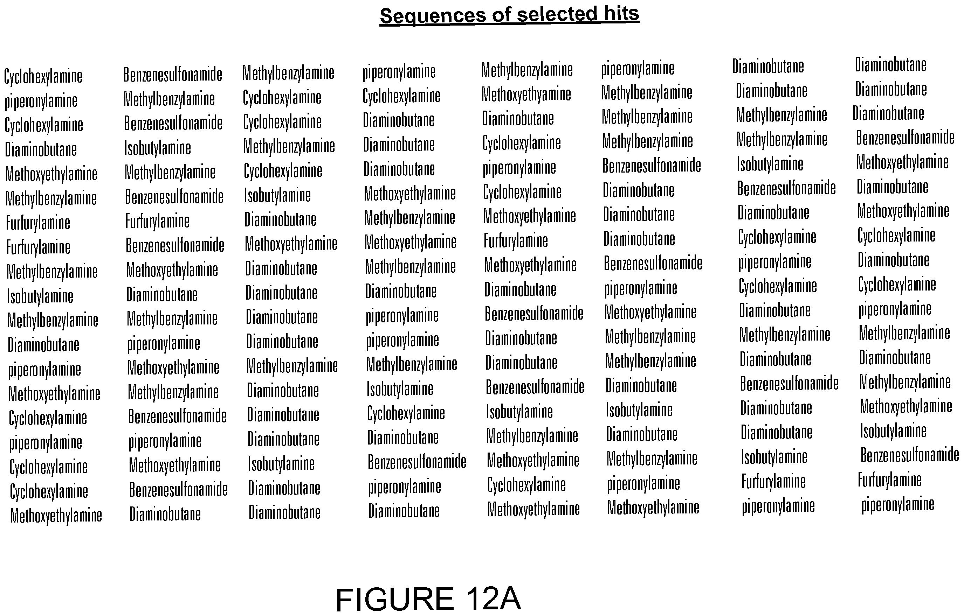

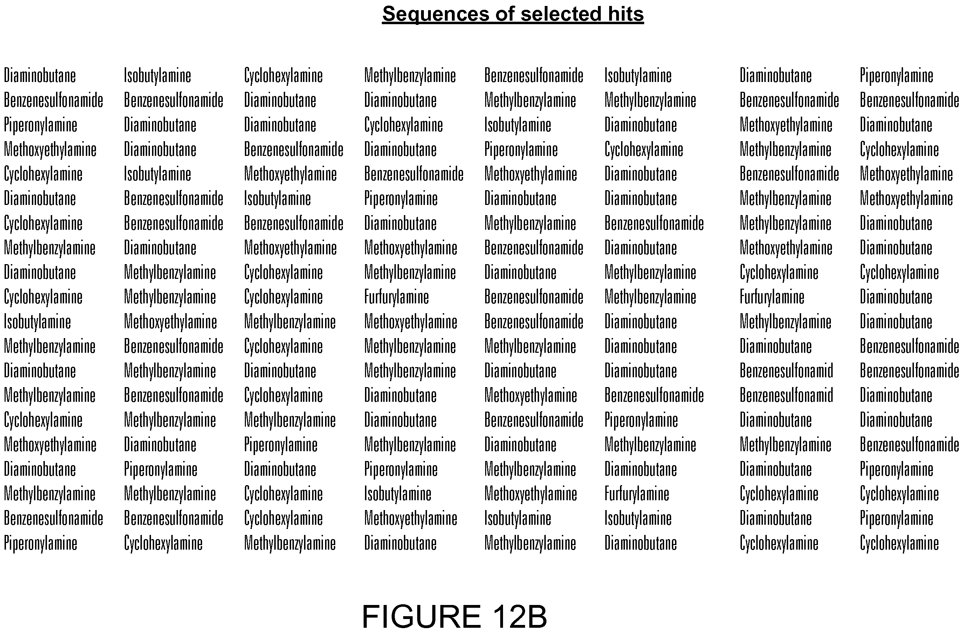

Figure 12 shows the peptoid sequences of the putative hits selected from the Alzheimer's screen from the JC3B library. The C-terminus is on the right side of the sheet and the N-terminus is on the left side.

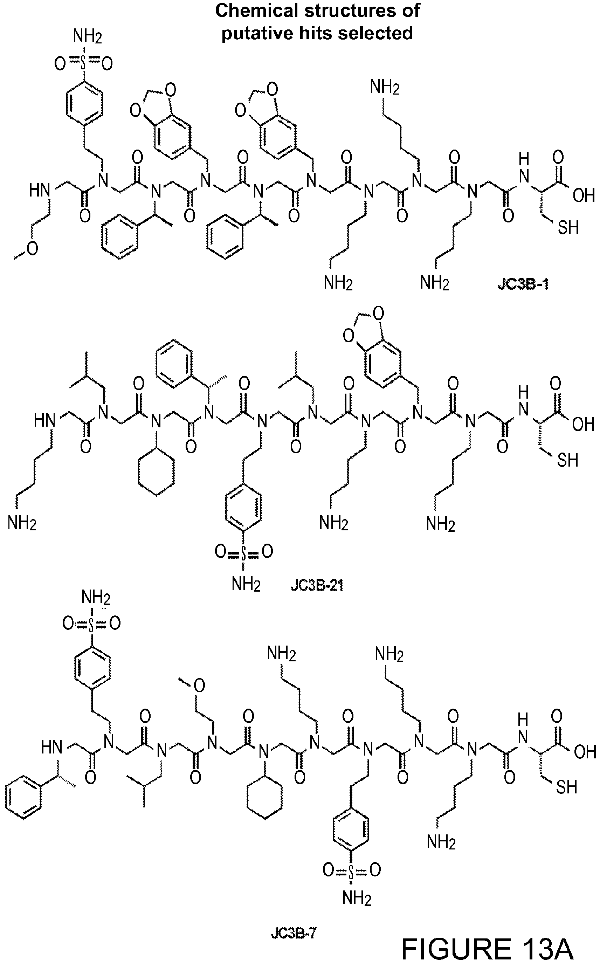

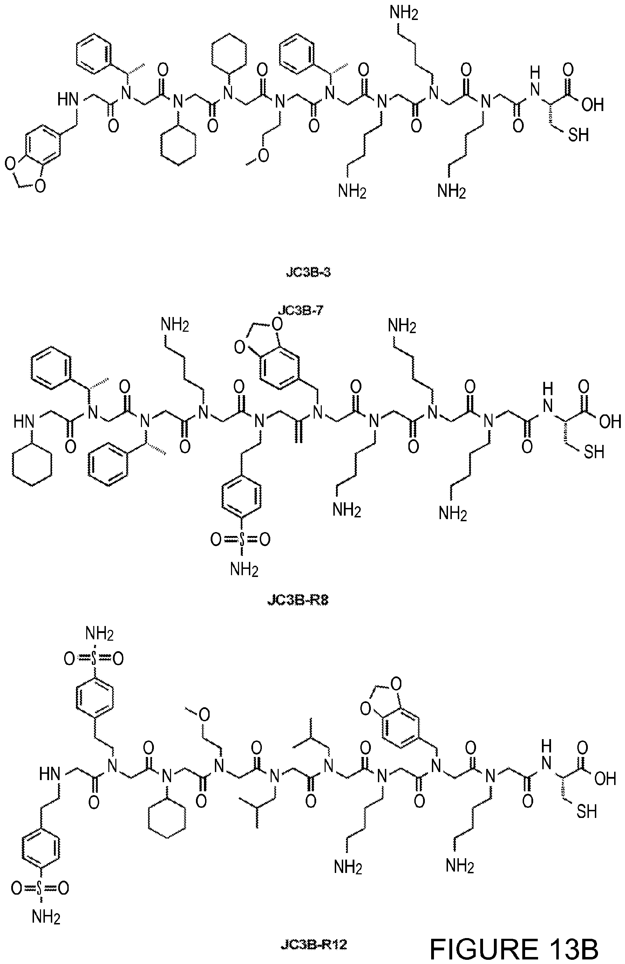

Figure 13 shows the chemical structures of the preferred high affinity hits from the Alzheimer's screen from the JC3B library, in this example, the structures shown have a cysteine residue and were resynthesized after determining the structure of the initial hit in the preliminary screen. The JC3B library contained an analous peptoid but which had a methionine residue on the C-terminus and not a cysteine residue.

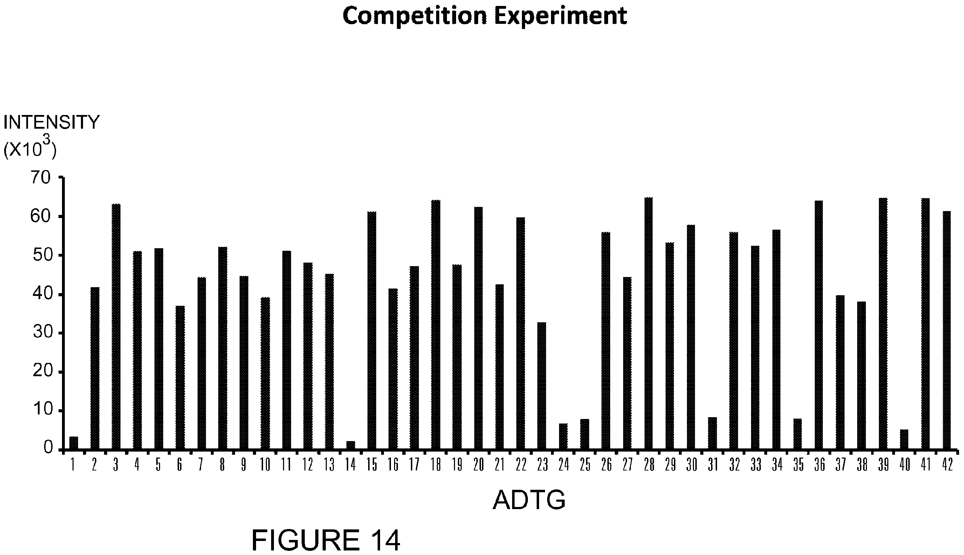

Figure 14 shows a competition experiment between a high affinity ligand (ADTGI) in solution versus ADTG-1-ADTG-42 on a microarray support. The competition experiment shows that ADTGI in solution bonded to the same antibody that would have bound to peptoids ADTG-1, ADTG14, ADTG24, ADTG25, ADTG3I, ADTG35 and ADTG40 on the microarray. Similar experiments were conducted on each of the peptoids to find four sets of peptoids, which bound to four distinct Alzheimer's autoantibodies.

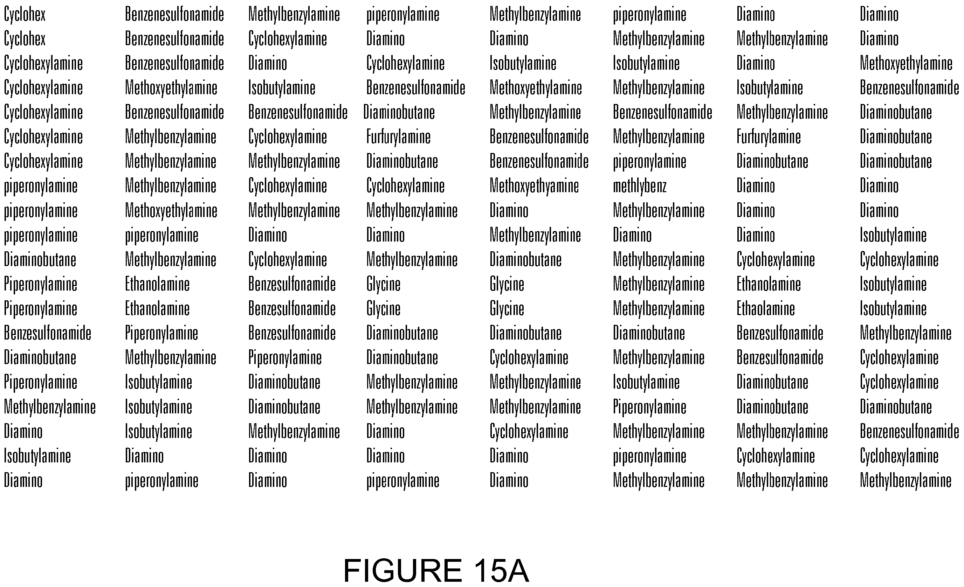

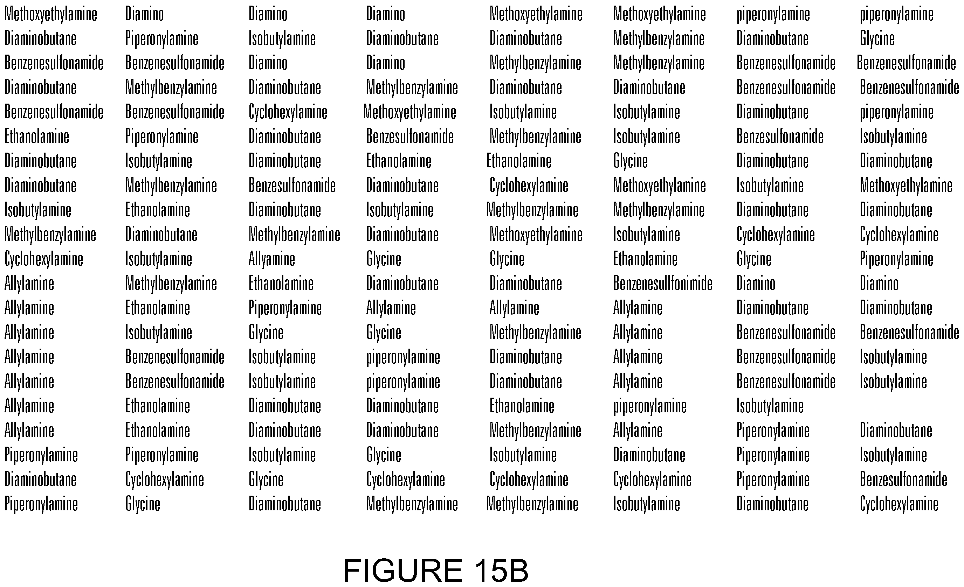

Figure 15 shows the four groups of distinct peptoids, which bind to different autoantibodies in the Alzheimer's screen. Each group on the figure has the higher affinity binder at the top.

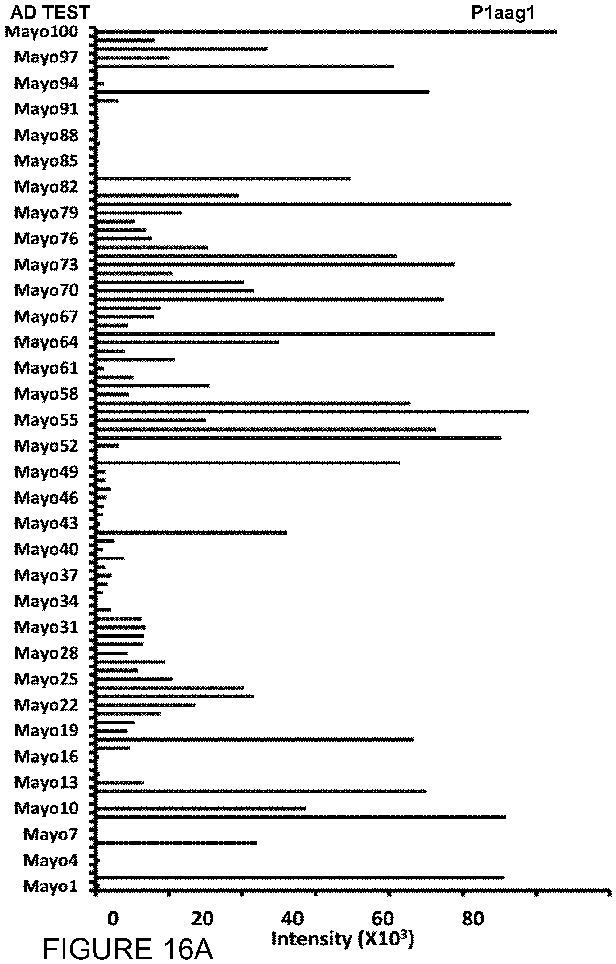

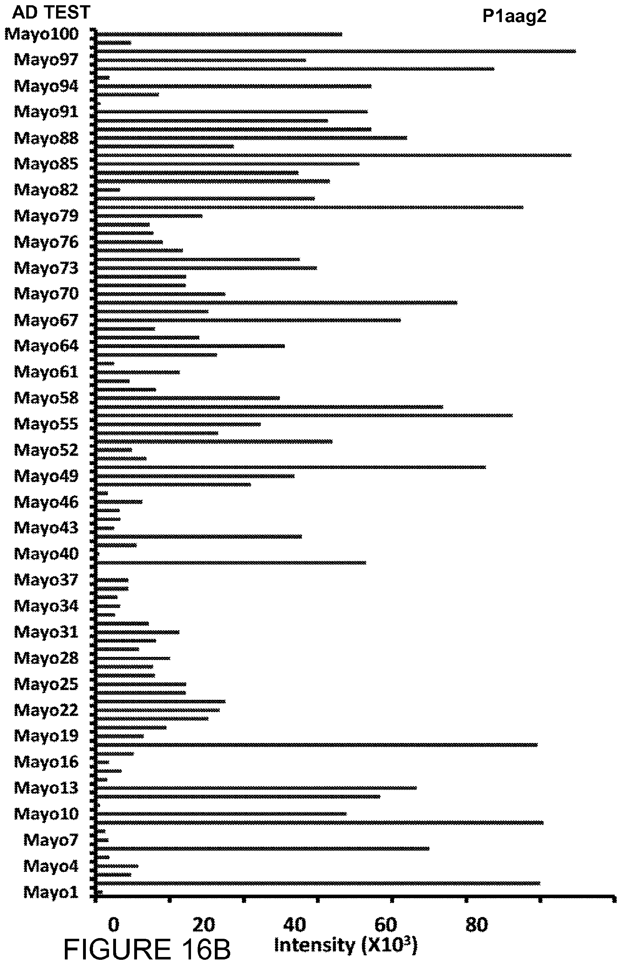

Figure 16A shows AD test data (blinded) for a pool of patients using PlaagI (JC3B-1) peptoid and Figure 16B shows test data (blinded) for the same pool of AD patients using Plaag2 (JC3B-21). Each peptoid is presented on a microarray.

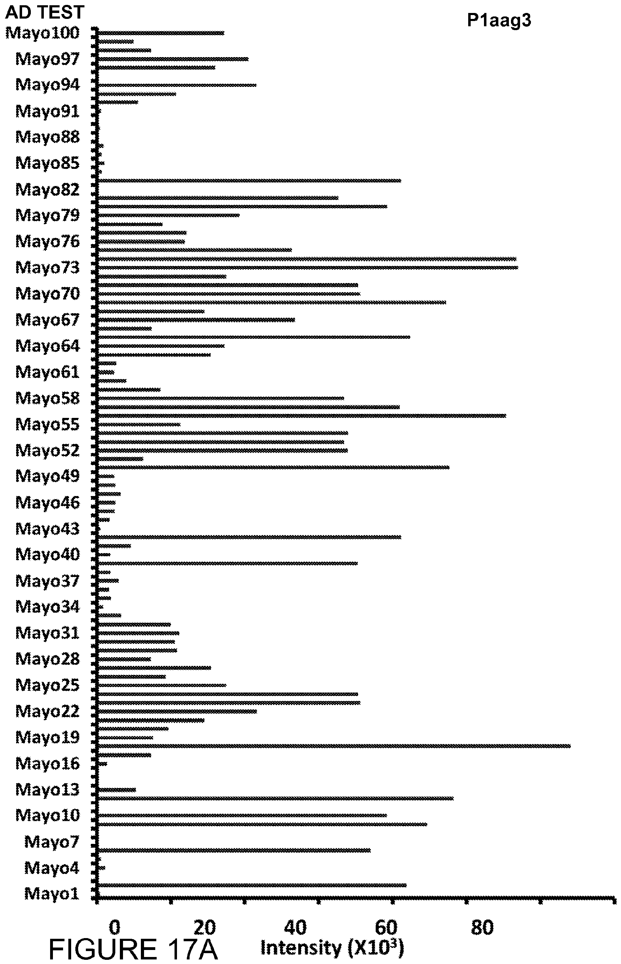

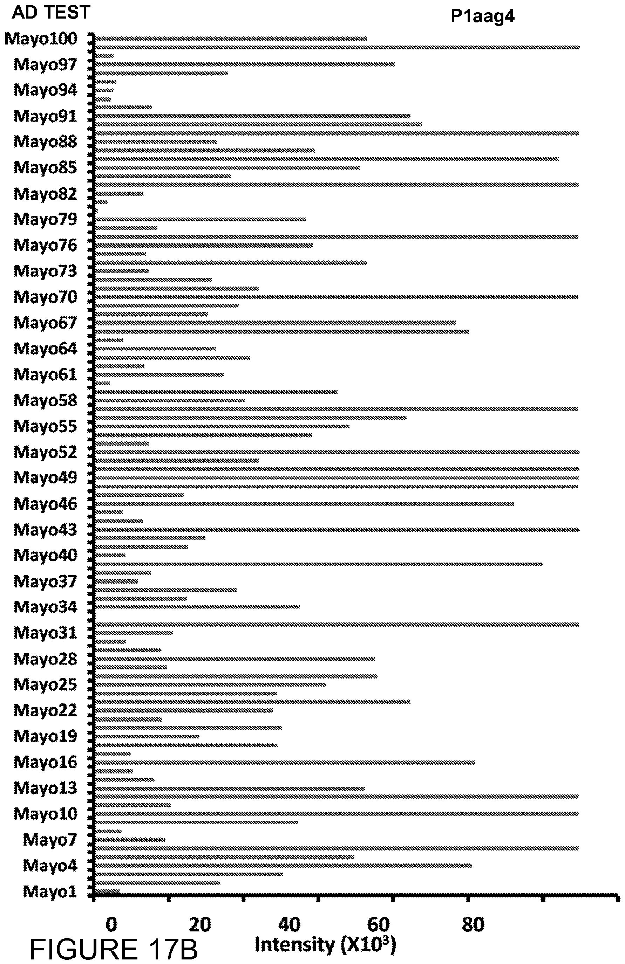

Figure 17A shows AD test data (blinded) for a pool of patients using Plaag3 (JC3B-7) peptoid and Figure 17B shows test data (blinded) for the same pool of AD patients using Plaag4 (3C3B-5). Each peptoid is presented on a microarray.

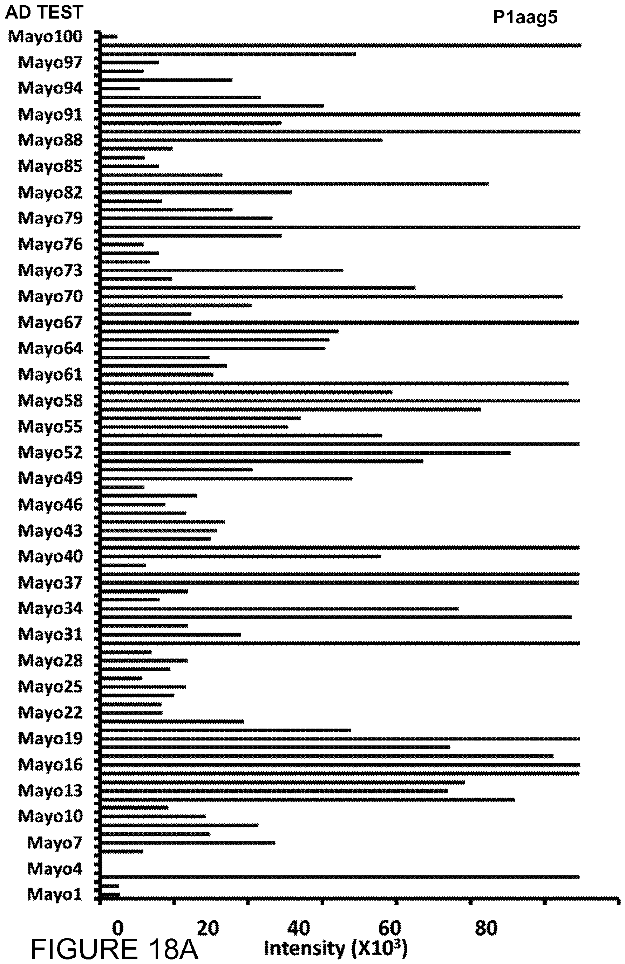

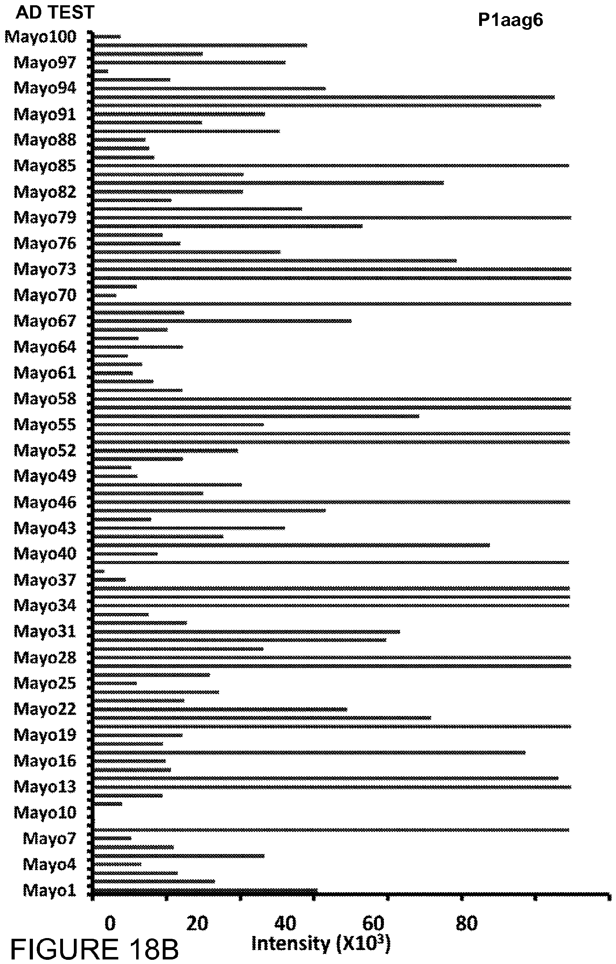

Figure 18A. shows AD test data (blinded) for a pool of patients using Plaag5 (JC3B-R8) peptoid and Figure 18B shows test data (blinded) for the same pool of AD patients using Plaag6 (JC3B-R 12). Each peptoid is presented on a microarray.

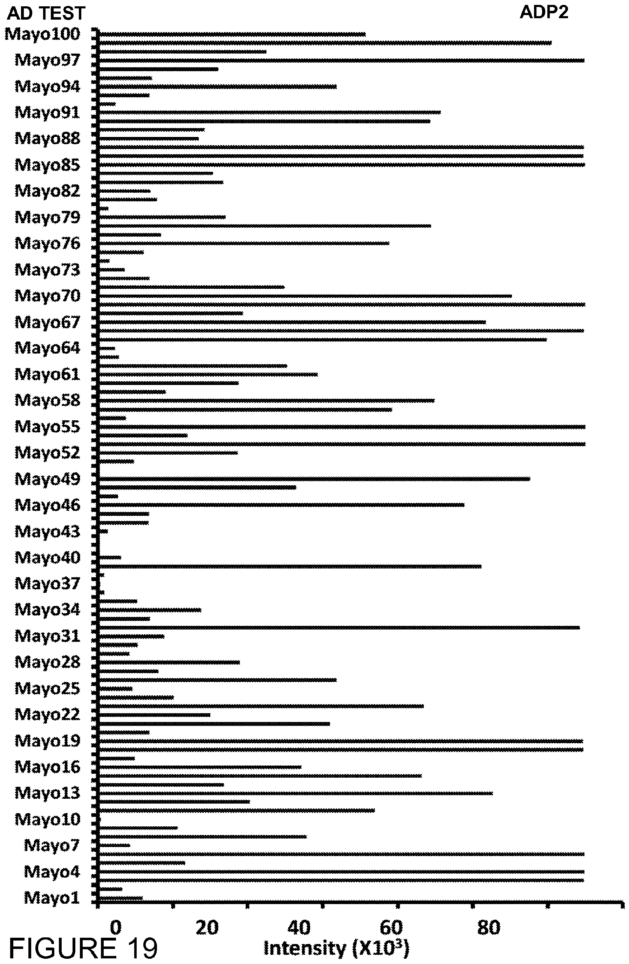

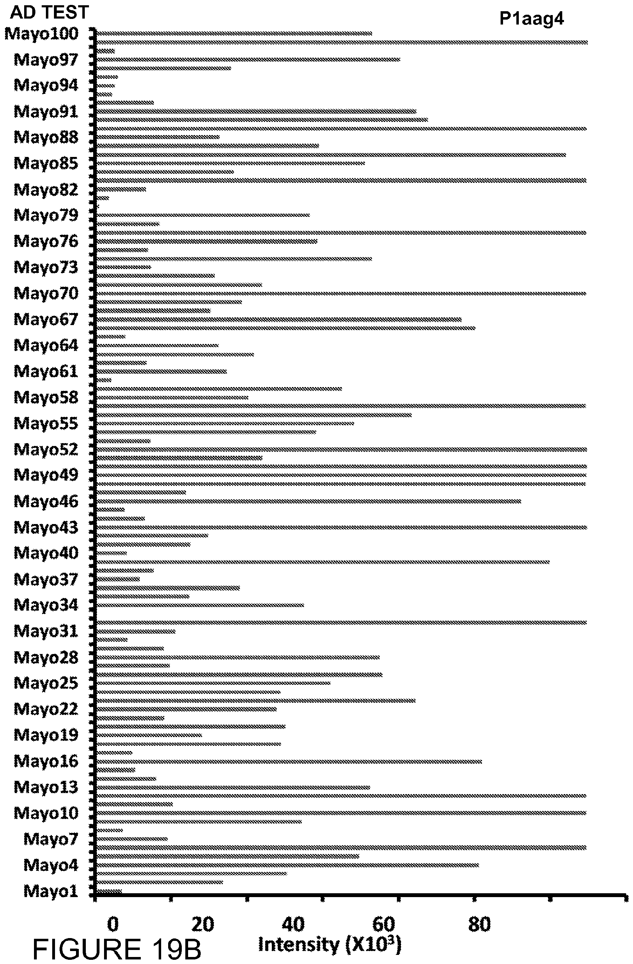

Figure 19A shows microarray data for ADP2 in the same pool of patients for the tests conducted using Plaag1-6. Figure 19B shows comparative data using Plaag4 with the same set of patients. The data shows a clear correlation between the results achieved with a previously identified ADP2 and the newly identified Plaag4 in the same patient pool.

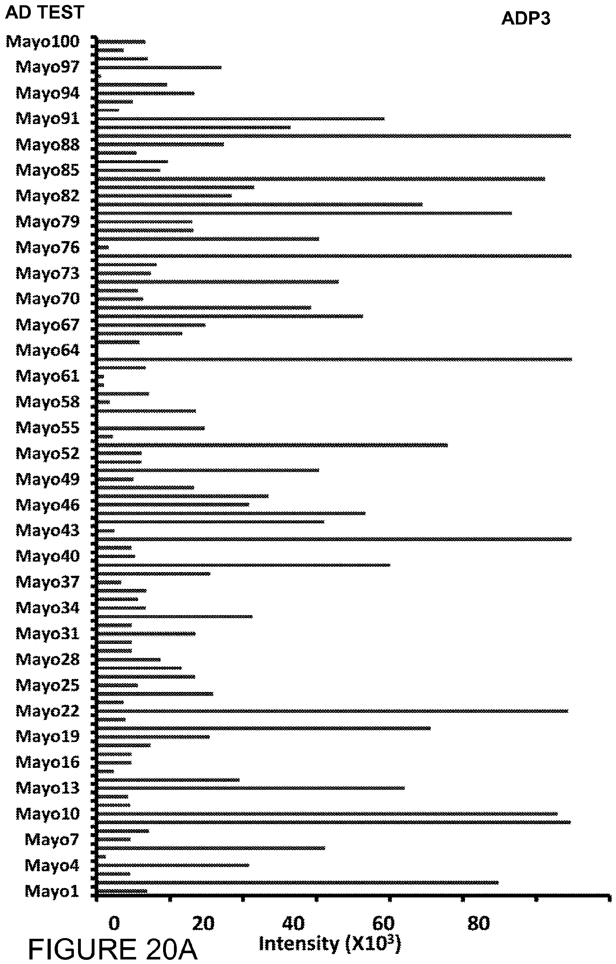

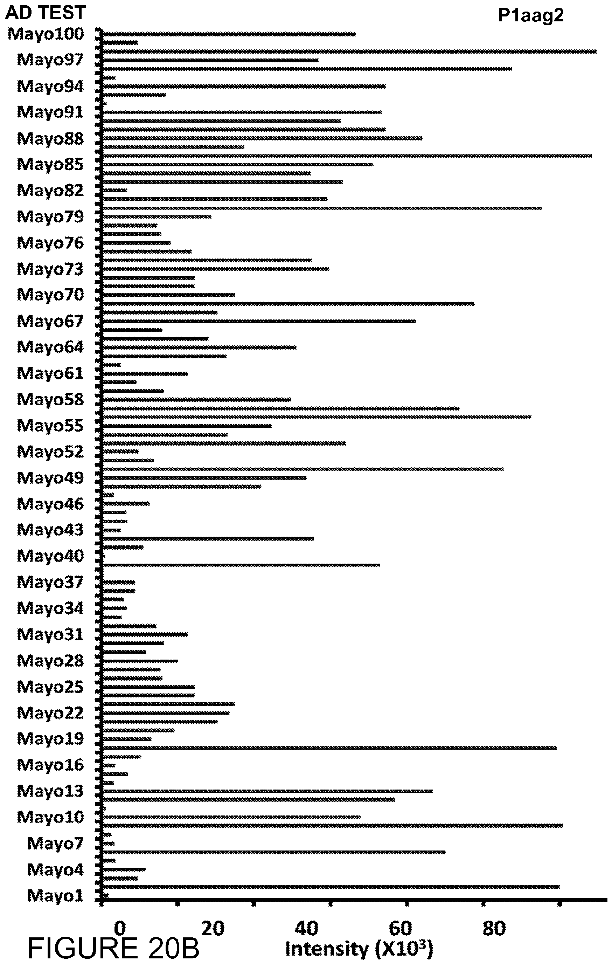

Figure 20A shows microarray data for ADP3 in the same pool of patients for the tests conducted using Plaag1-6, Figure 20B shows comparative data using Plaag2 with the same set of patients. The data shows a clear correlation between the results achieved with a previously identified ADP3 and the newly identified Plaag2 in the same patient pool.

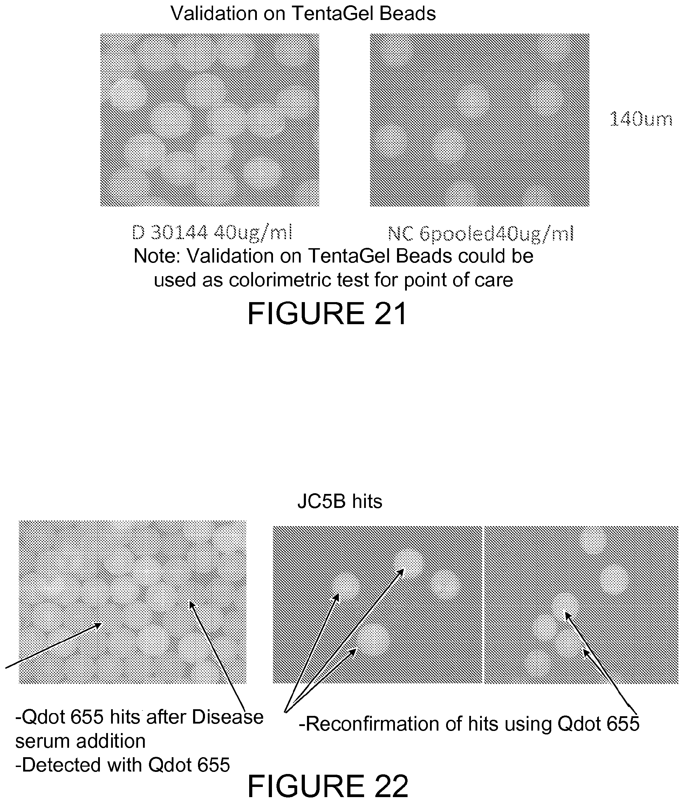

Figure 21 shows a validation of Plaag5 (putative hit 5 orJC3B-R8) on TentaGel beads in a comparison of diseased AD serum versus healthy control (pooled) at 40 ug/mL.
Figure 22A. shows the peptoid hits in the pancreatic cancer screen using QDot 655 and using the JC5B library. Figures 22B and C show confirmation of hits using QDot 655 (arrows point to hits).

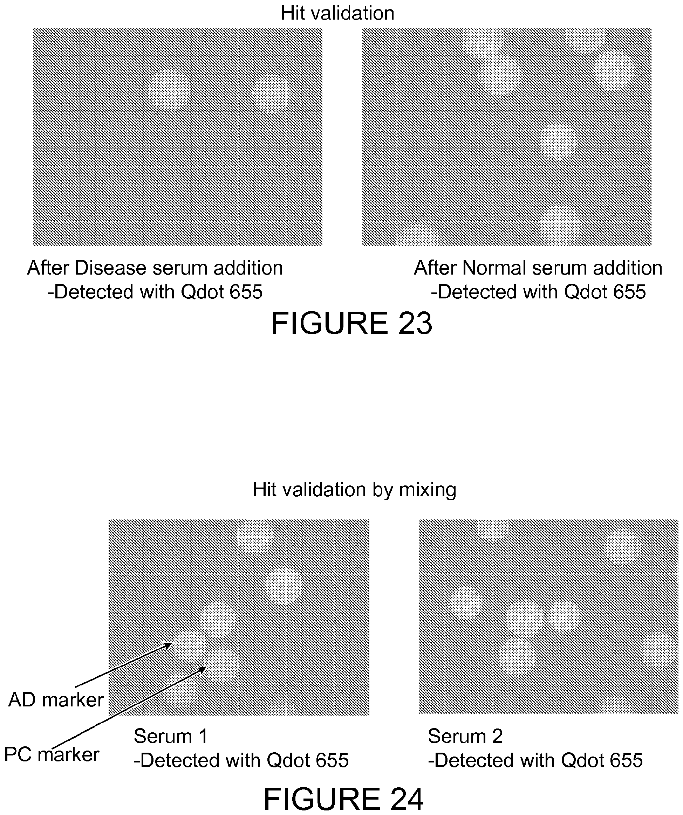

Figure 23 shows pancreatic peptoid hit validation and compares disease serum addition and detection with QDot 655 versus normal serum addition.
Figure 24 shows hit validation by mixing AD markers and PC markers. The data shows that, the PC marker was detected while there was no detectable antibody on the AD peptoid bead in the pancreatic cancer serum (Serum 1).

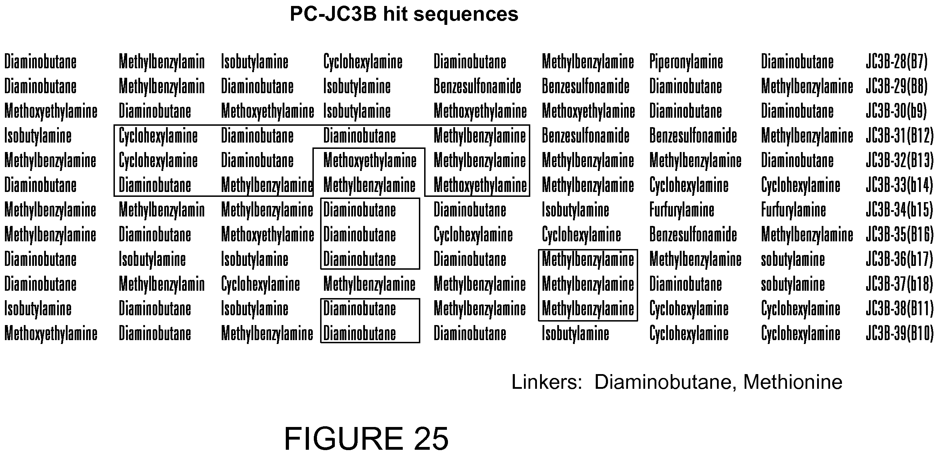

Figure 25 shows the pancreatic cancer screen hit sequences from the JC3B library.

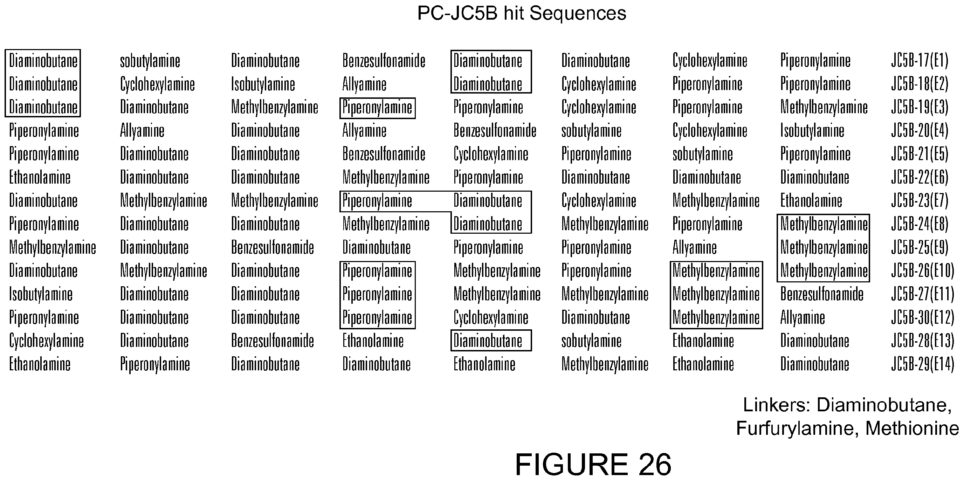

Figure 26 shows the pancreatic cancer screen hit sequences from the JC5B library.

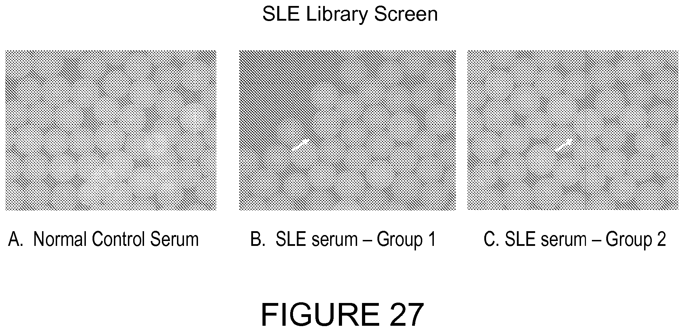

Figure 27A, B and C show the results of an SLE (Lupus) screen. A is normal control and B and C are SLE serum from two different groups 1 and 2. The arrows point to the hits.

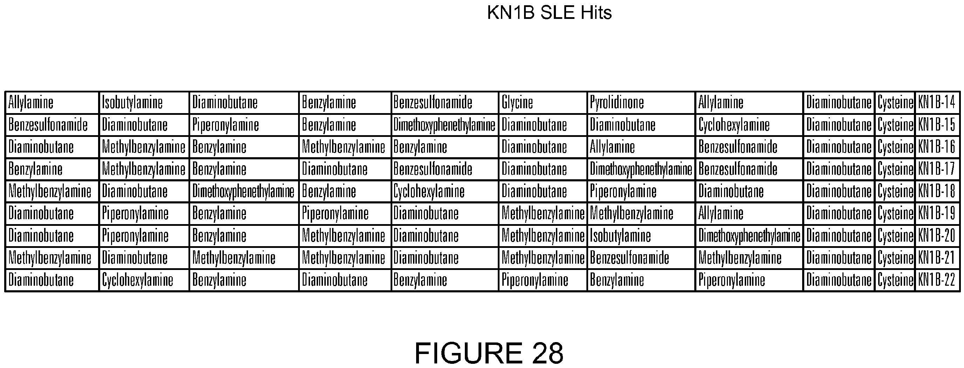

Figure 28 shows the SLE hits from the KN1B library. The C-terminus is on the right side of the sheet.

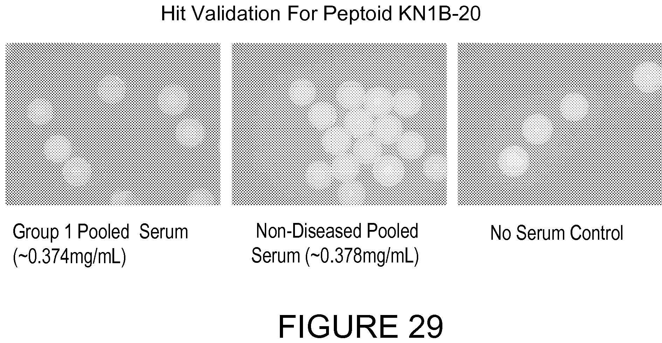

Figure 29 shows a hit validation for peptoid KN1B-20. Group 1 is pooled diseased serum at a concentration of about 0.374 mg/mL (left picture)(the hits are shown with a red tinge on the bead). Non-diseased pooled serum (center picture) is provided at a concentration of about 0.378 mg/mL and the far right picture shows a no serum control.

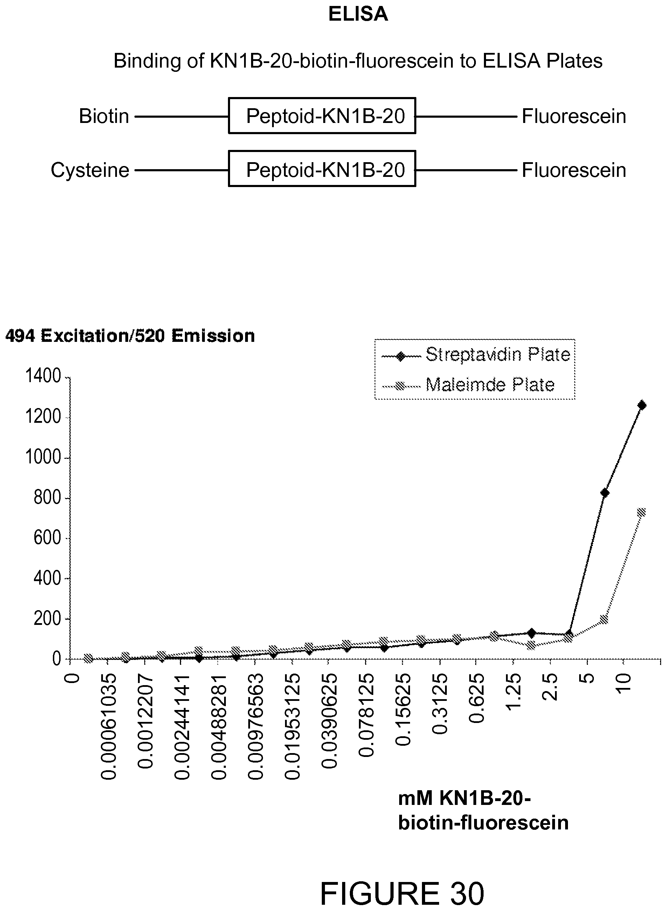

Figure 30 shows the binding/detection of one of the SLE (lupus) peptoids to ELISA plates using two different binding methods at different concentrations of peptoid using a fluorescein tag.

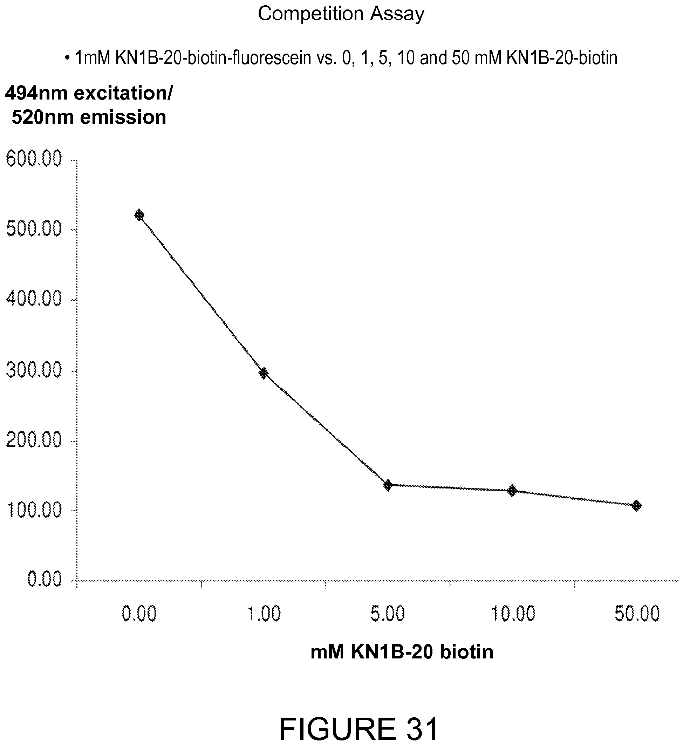

Figure 31 shows a competition assay between plate bound KN1B-20-biotin-fiuorescein versus free KN1B-20-biotin in solution at various concentrations. Signal dampening occurs as the concentration of free KN1B-20-biotin increases from equimolar concentrations of bound versus free.

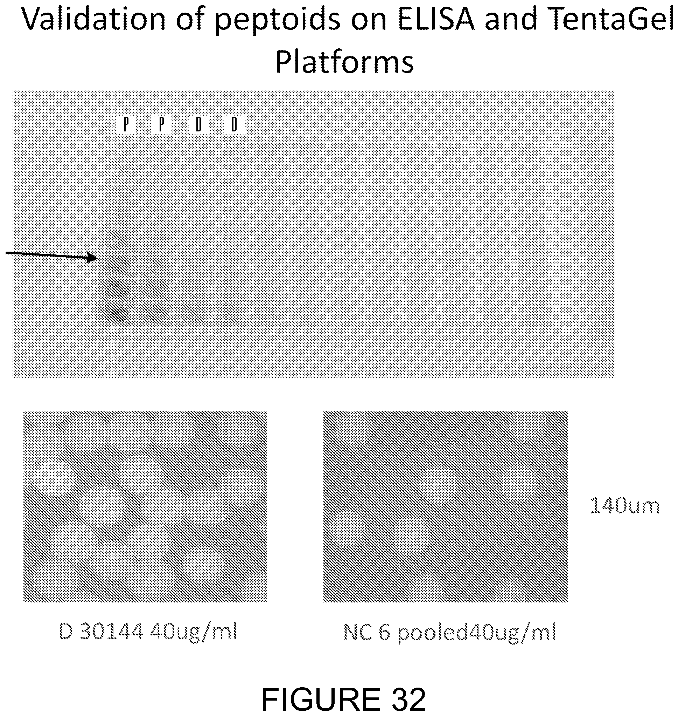

Figure 32 shows an ELISA plate having peptoid at various concentrations and clearly shows a difference between diseased serum (AD)(P column 1) and normal control serum (column 3) [1:200 doubling each well to 1:400,1:800, I; 1,600, 1:3,200, 1:6,400, 1:12,800]. The arrow points to the 1:800 dilution in 1XTBST buffer. The peptoid concentration in the wells is 10 mM. Figure 32 also shows validation of the TentaGel bead platform to distinguish between diseased and control sera.

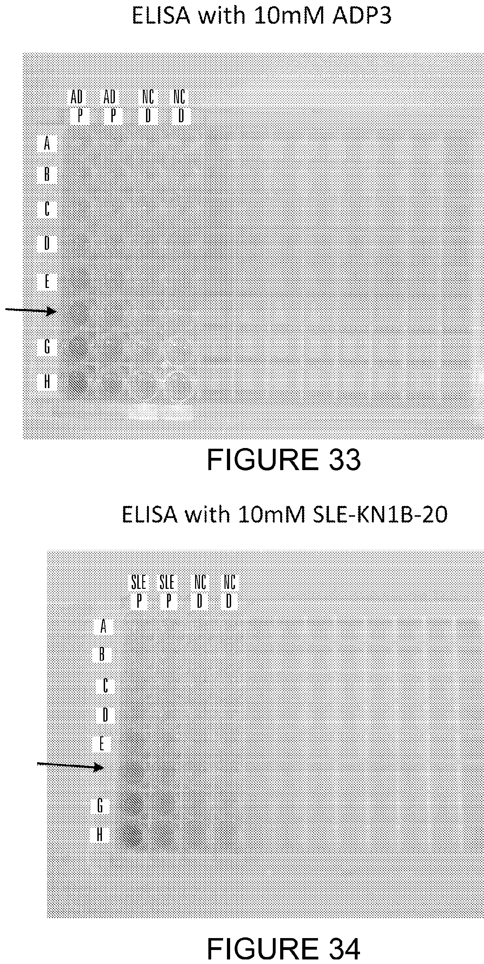

Figure 33 shows an ELISA plate with 10 mM ADP3 and at various dilutions of AD sera versus control sera. The arrow points to the 1:800 dilution.
Figure 34 shows an ELISA plate with 10 mM SLE-KNJ B-20 and at various dilutions of AD sera versus control sera. The arrow points to the 1:800 dilution.

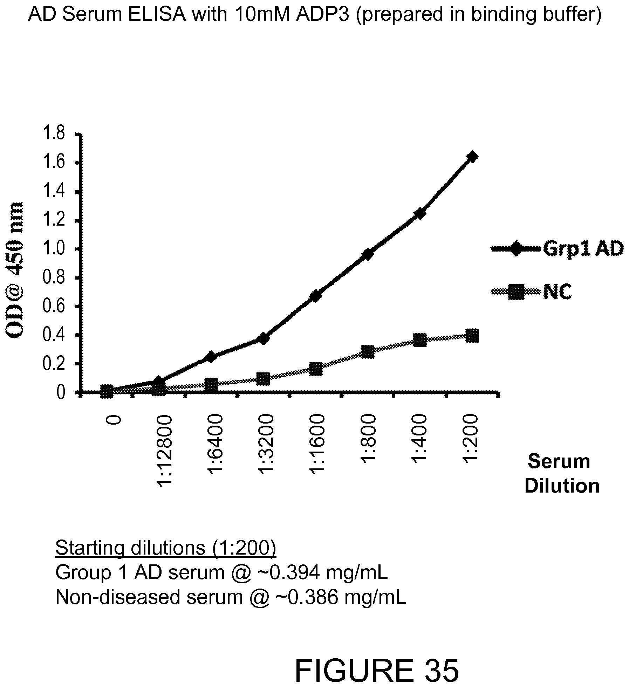

Figure 35 shows an AD serum ELISA graph using 10 mM ADP3 prepared in binding buffer at various serum dilutions. Separation between normal and diseased serum occurred, over the dilution range of 1:200 through approximately 1:10,000. The starting dilutions were .1:200 (Group 1 AD serum .394 mg/mL and non-diseased serum at .386 mg/mL).

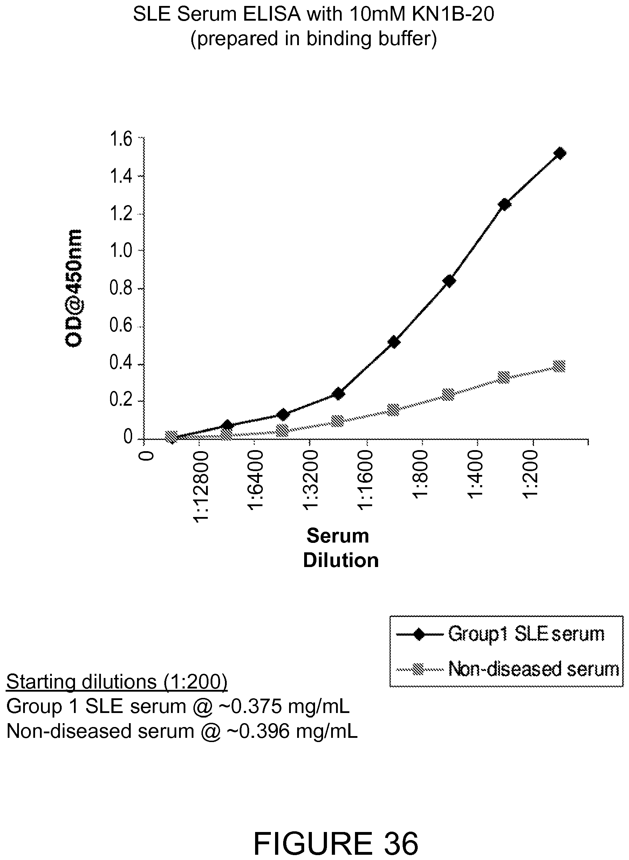

Figure 36 shows an SLE serum ELISA graph using 10 mM KN1B-20 prepared in binding buffer at various serum dilutions. Separation between normal and diseased serum occurred over the dilution range of 1:200 through approximately 1;10,000. The starting dilutions were 1:200 (Group 1 SLE serum .375 mg/rnL and non-diseased serum at .396 mg/mL).

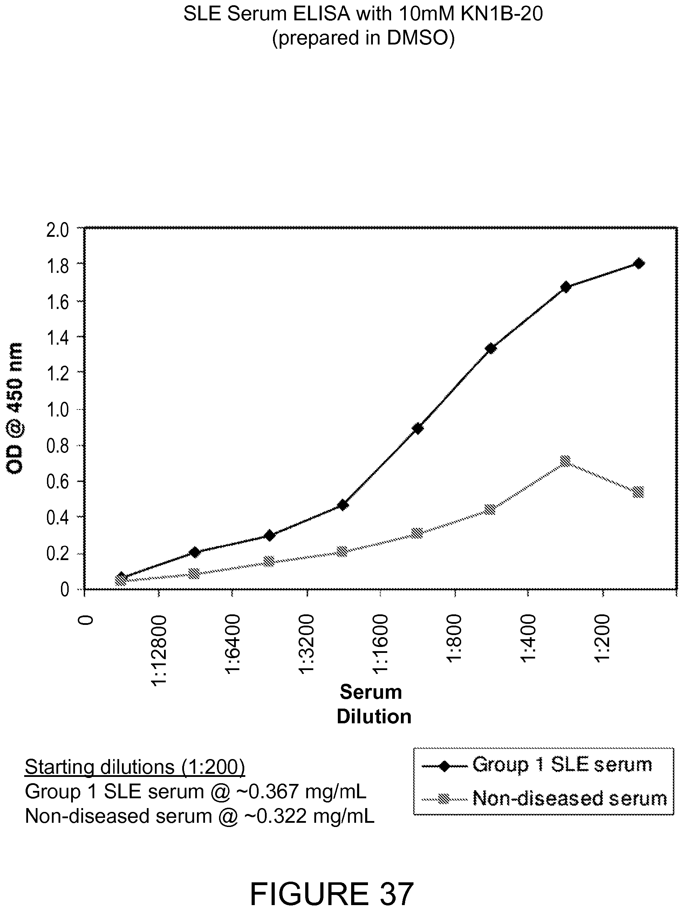

Figure 37 shows an SLE serum ELISA graph using 1.0 mM KN1B-20 prepared in DMSO at various serum dilutions. Separation between normal and diseased serum occurred over the dilution range of 1:200 through approximately 1:10,000. The starting dilutions were 1:200 (Group 1 SLE serum .367 mg/mL and non-diseased serum at .322 mg/mL).

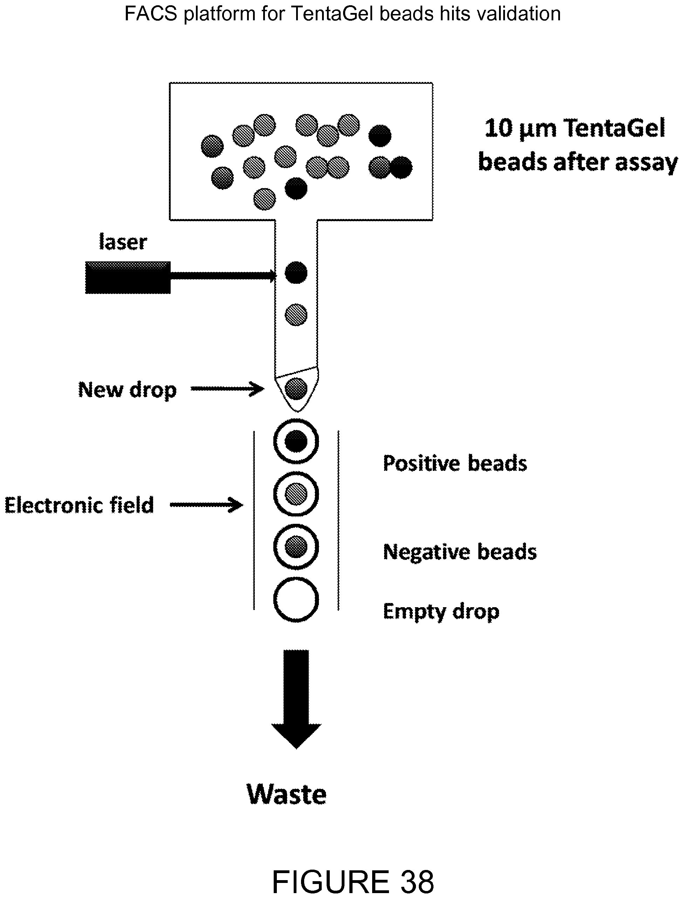

Figure 38 shows a FAGS platform for Tentagel beads hits validation.

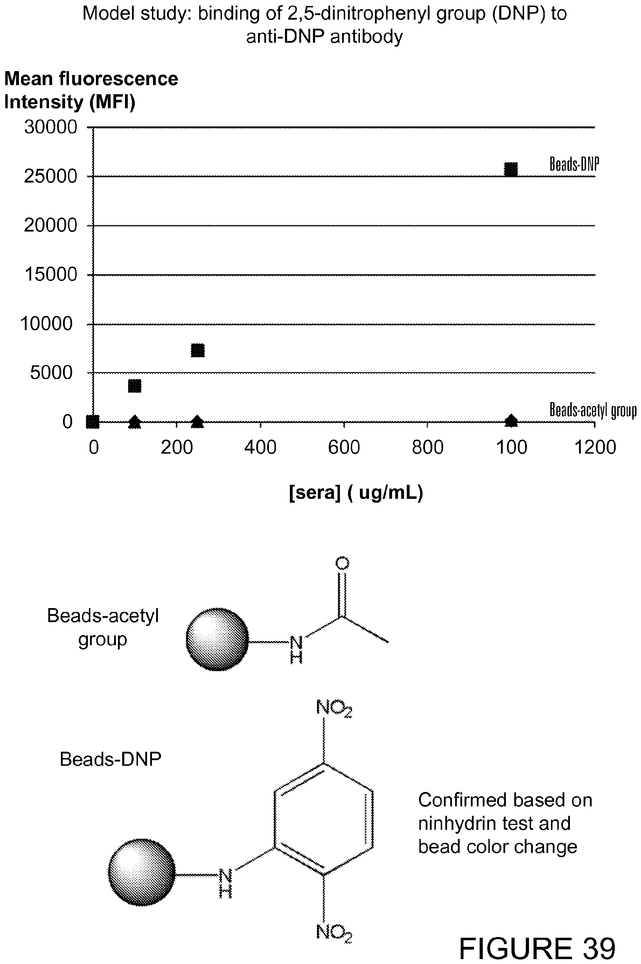

Figure 39 shows the degree of separation between beads having an acetyl group and beads having a 2,5-dintrophenyl group (DNP) at various concentrations of sera (100 ug/mL to 1,000 ug/mL) and in response to treatment with an anti-DNP labeled secondary antibody. The Mean fluorescence intensity (MFI) separation was greatest at the higher dilution of 1,000 ug/mL sera.

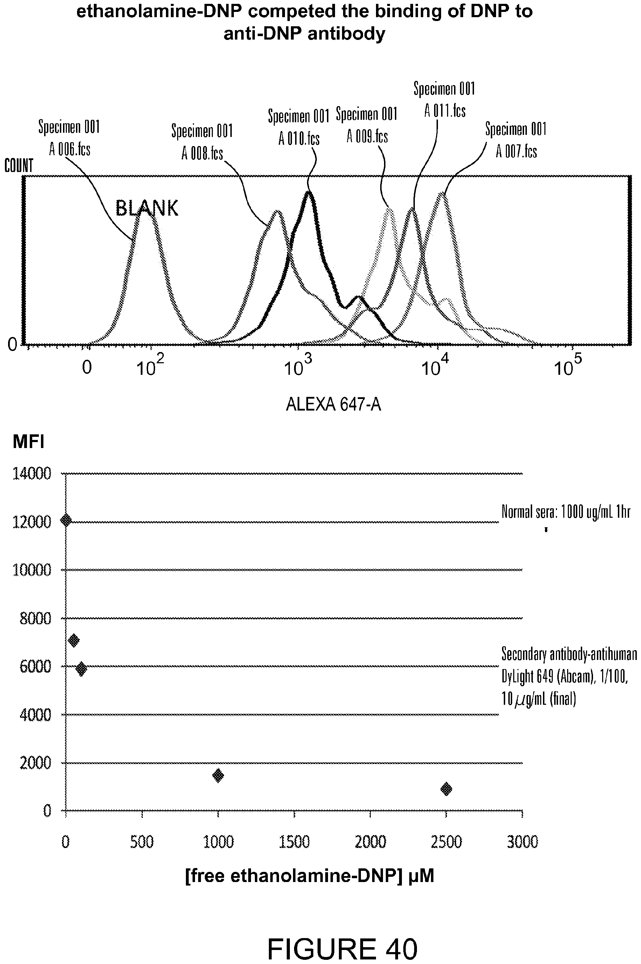

Figure 40 shows that there is a direct competition between free ethanolamine-DNP and the binding of DNP (on a plate) to anti-DNP antibody at 1,000 ug/mL sera concentration.

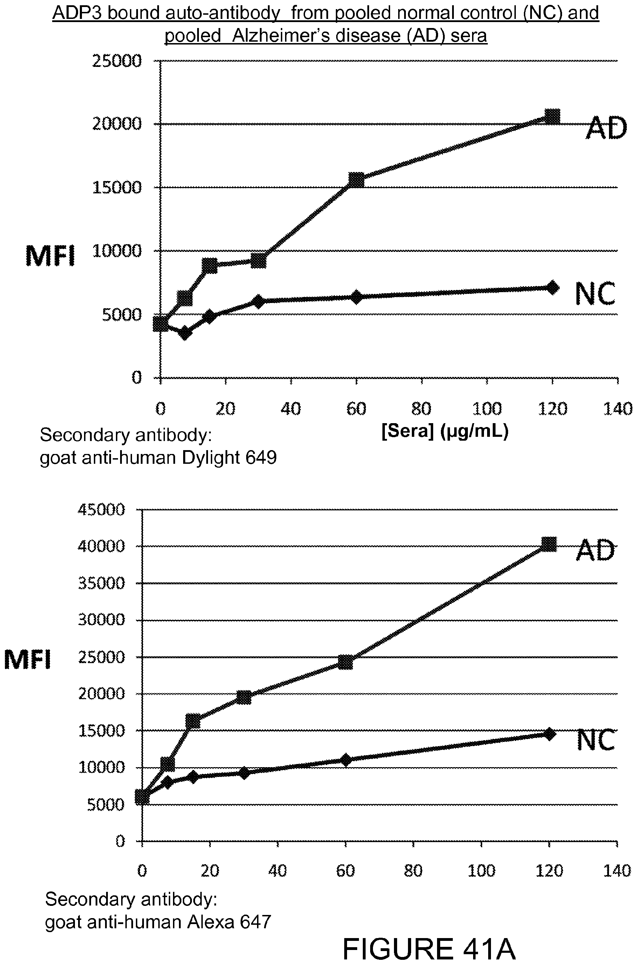

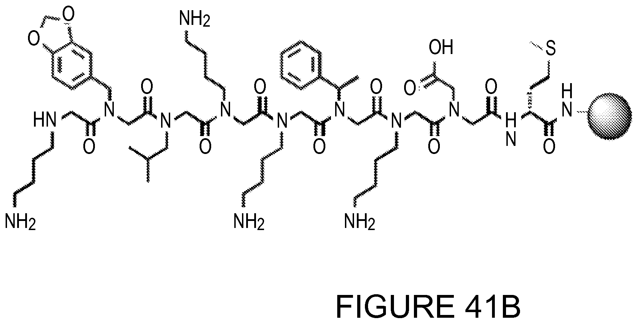

Figure 41 shows ADP3 bound anti-antibody from pooled normal control sera and pooled AD sera. The data shows good separation at sera concentration ranges of 20 and 140 ug/mL using two different secondary antibodies (goat anti-human Dylight 649 and goat anti-human Alexa 647).

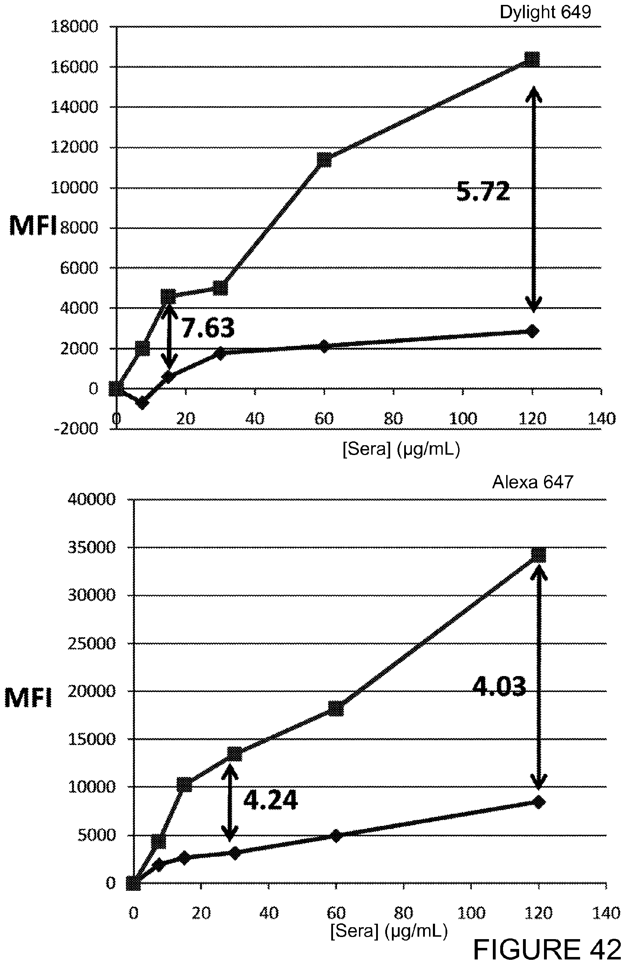

Figure 42 shows ADP3 bound auto-antibody from normal control and AD sera after background subtraction at various sera concentration ranges. There is a significant degree of separation at most sera concentration ranges from less than 20 ug/mL to 1.20 ug/mL or greater.

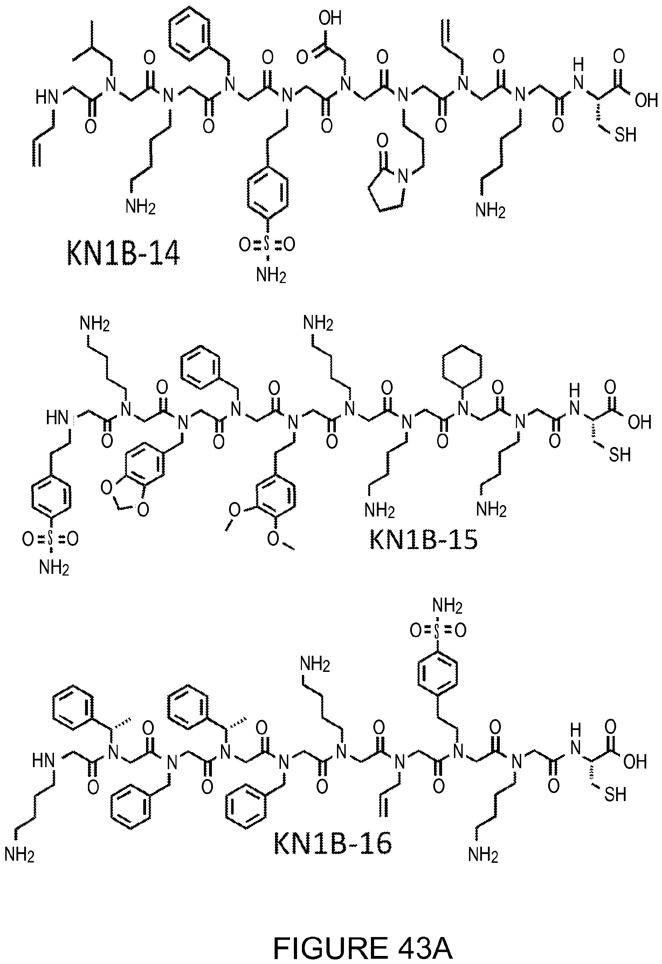

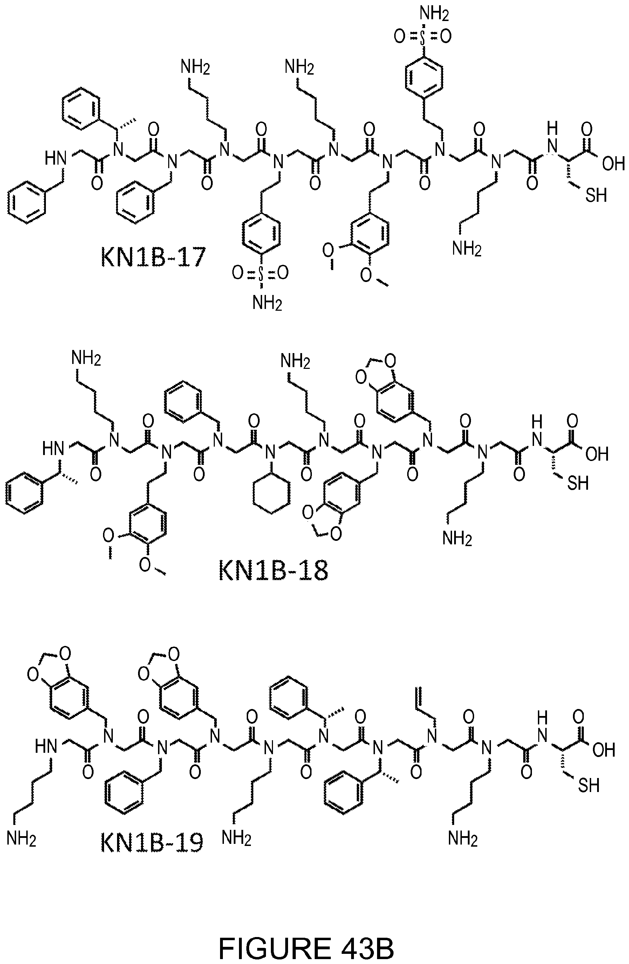

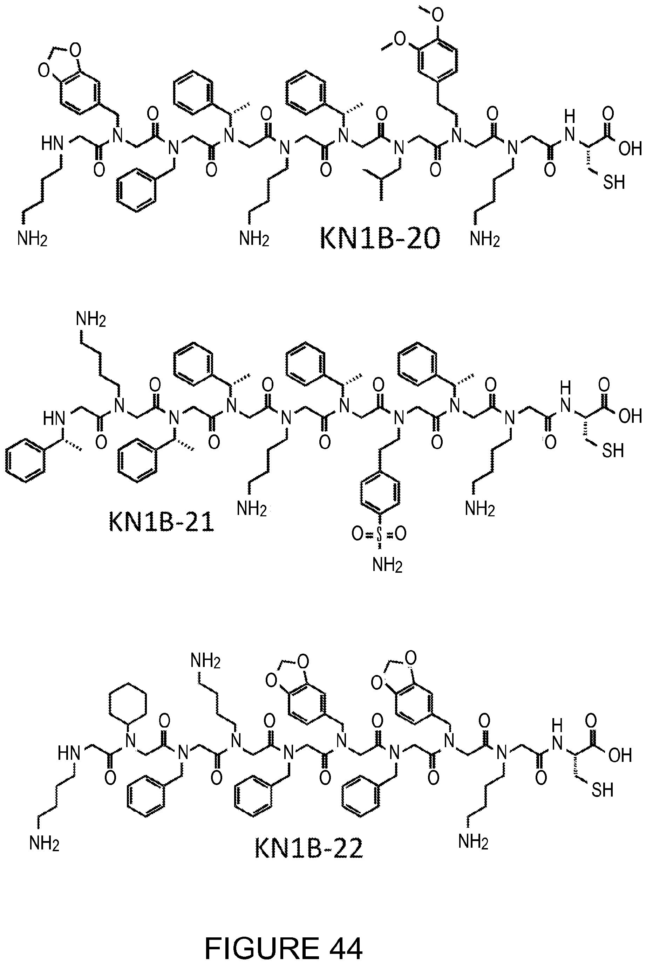

Figures 43 and 44 show the structures of the SLE (lupus) resynthesized peptoid ligand hits.

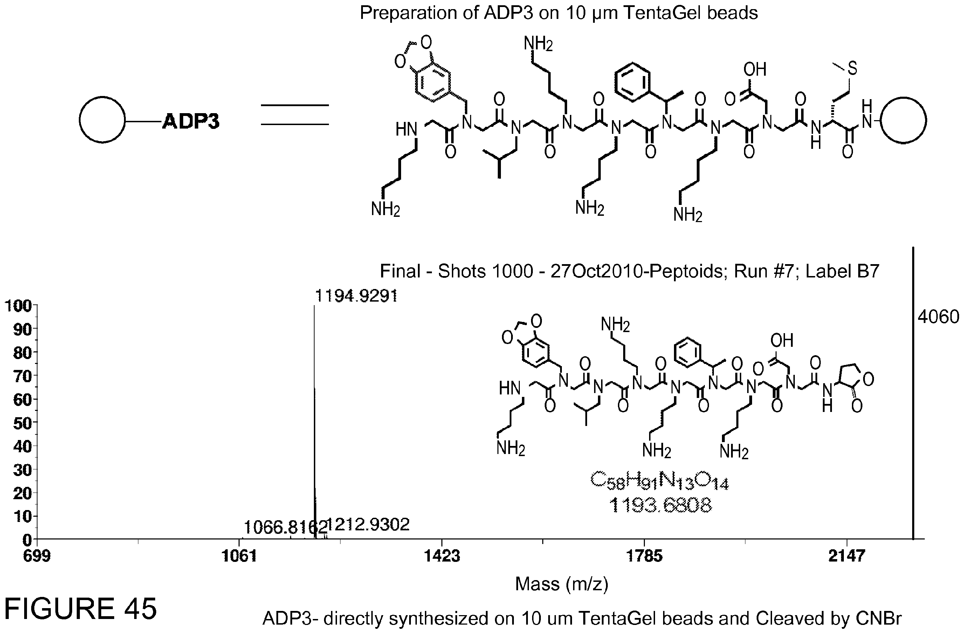

Figure 45 shows the preparation of ADP3 on 10 um Tentagel beads and the subsequent cleavage using CNBr along with a mass specttoraetry reading of the lactone shown.

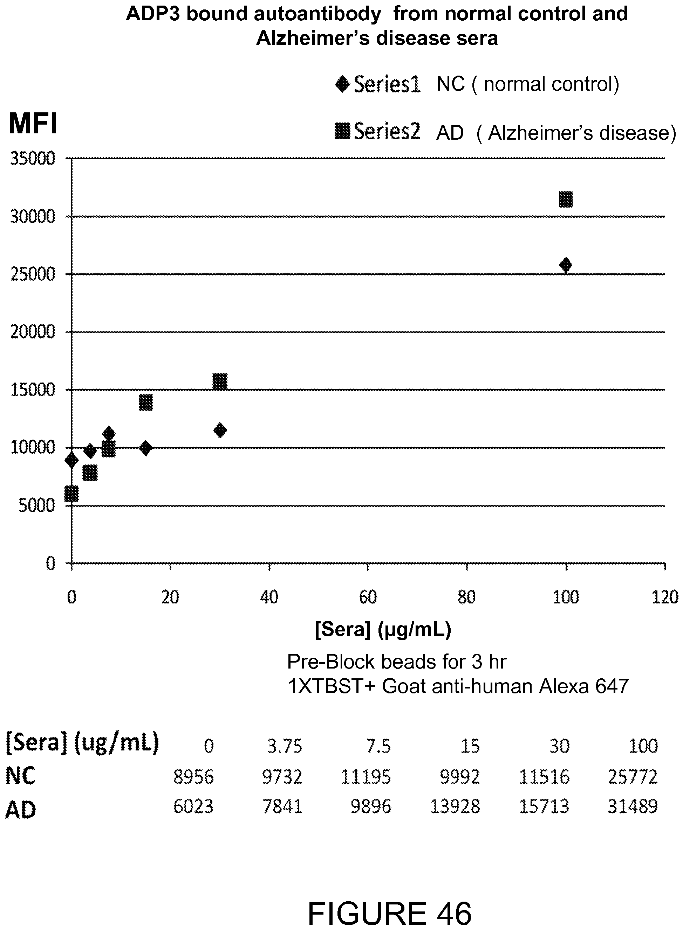

Figure 46 shows ADP3 bound autoantibody from normal control and Alzheimer's disease sera at different concentrations. The beads were preblocked for 3 hours with IX TBST and then detected using Goat anti-human Alexa 647 secondary antibody.

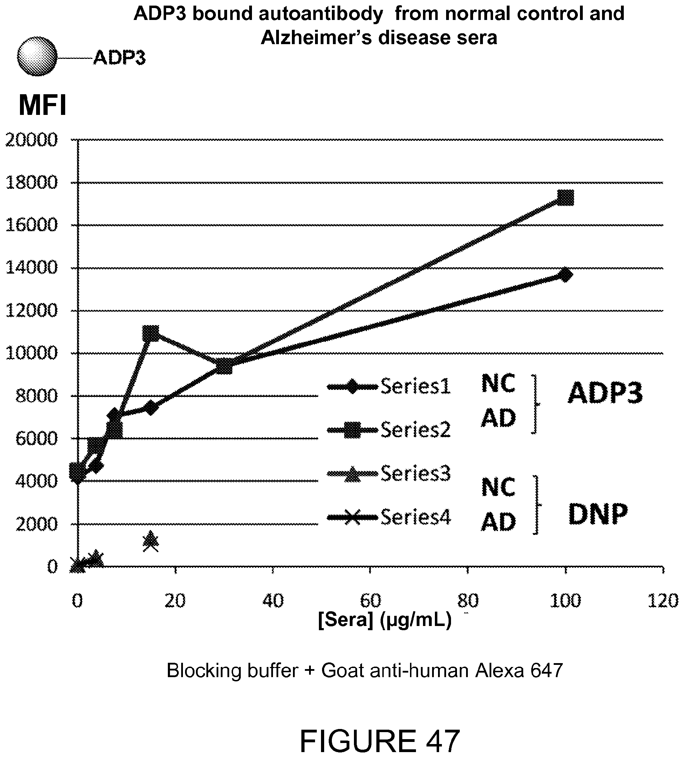

Figure 47 shows the ADP3 bound autoantibody from normal control and Alzheimer's disease sera at different sera concentrations and also shows DNP values.

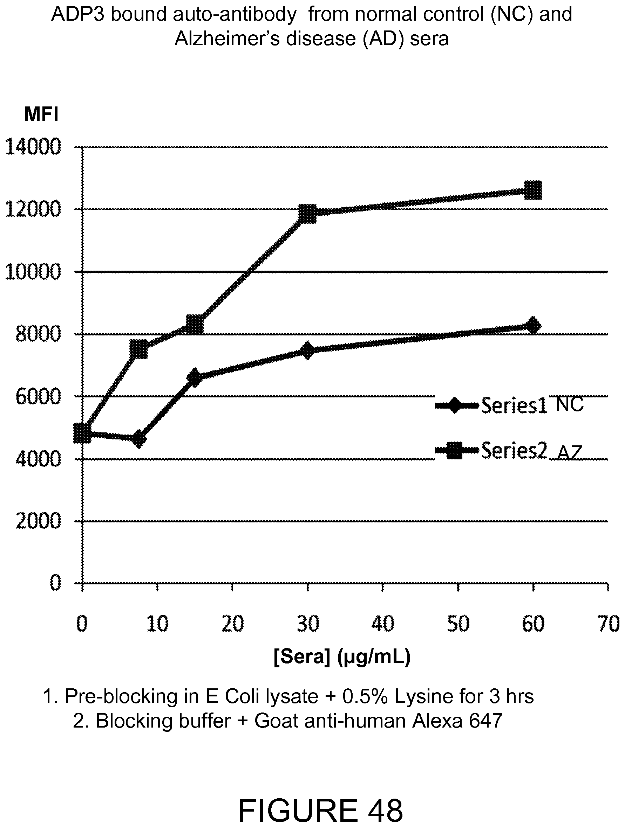

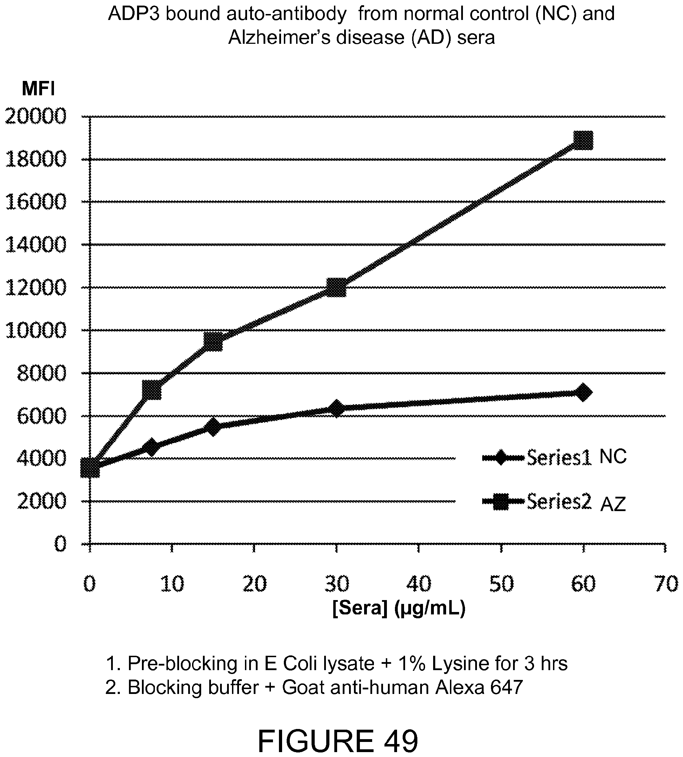

Figures 48 and 49 show ADP3 bound autoantibody from normal controls versus Alzheimer's disease sera using pre-blocking conditions such as E. coli lysate and lysine.

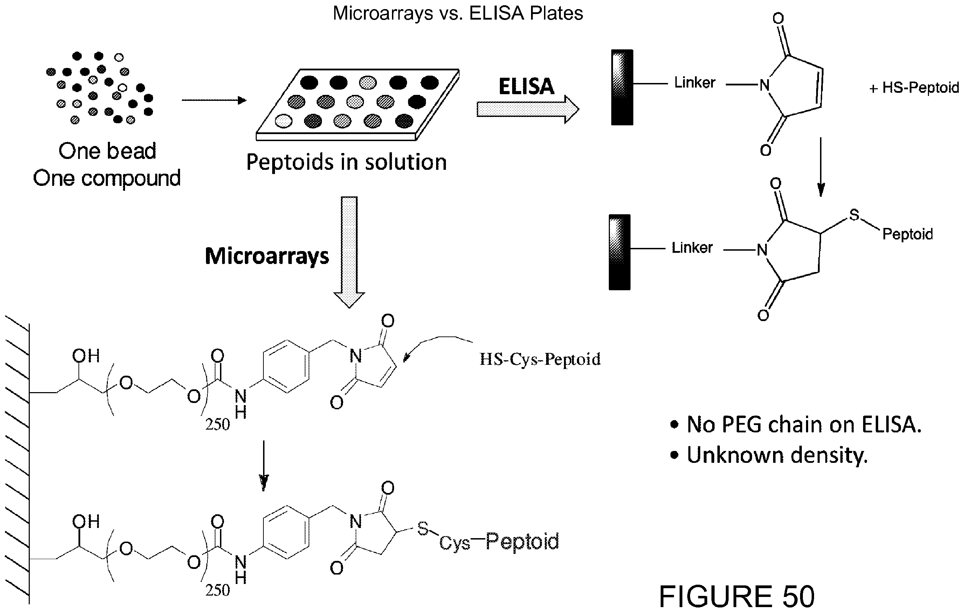

Figure 50 shows a simple schematic of the preparation of and distinction between peptoids that are used in microarrays versus those peptoids that are placed on ELISA plates. Schematic for how peptoid microarrays are made: individual beads are segregated into the wells of microtiter plates and the peptoids are cleaved from the beads to make a concentrated stock solution. Note that each well will now contain a single kind of peptoid. Several thousand peptoids are then spotted onto chemically-modified glass microscope slides in such a way that they bind covalently to the surface. Several thousand slides can be produced highly reproducibly from a single synthetic library. The ELISA production is similar except that there is no PEG chain on the surface but the density of peptoids on the ELISA plate may be different than it is on the microarrays.

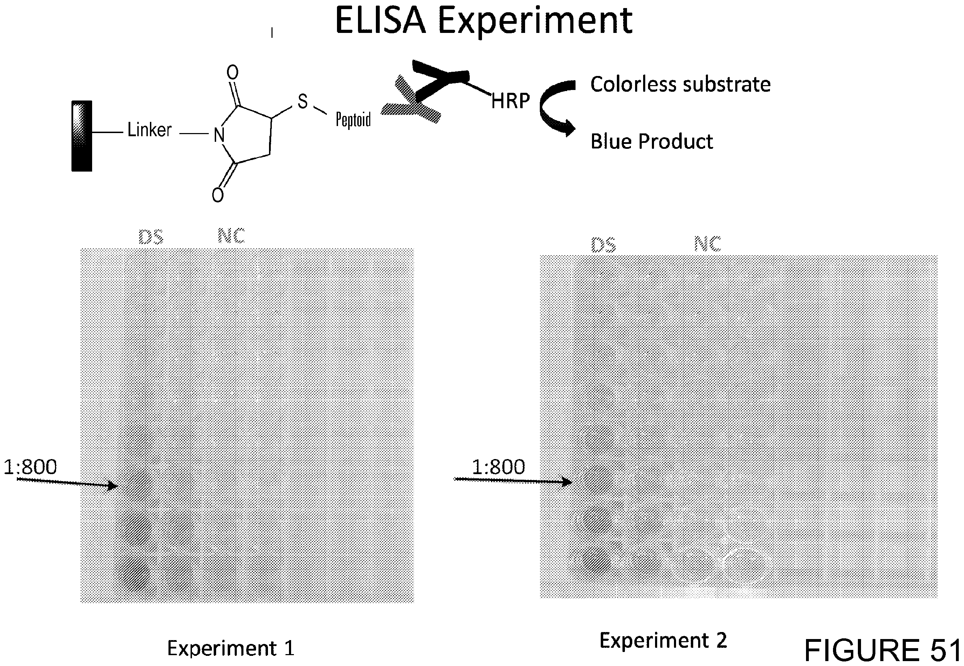

Figure 51 shows ELISA experiments with a clear distinction between normal control and diseased serum at a serum dilution of 1:800 using horseradish peroxidase linked to a secondary antibody that detects the disease associated antibody-peptoid complex. The colorless substrate is added and changes color (blue) upon reaction with the bound HRP enzyme.

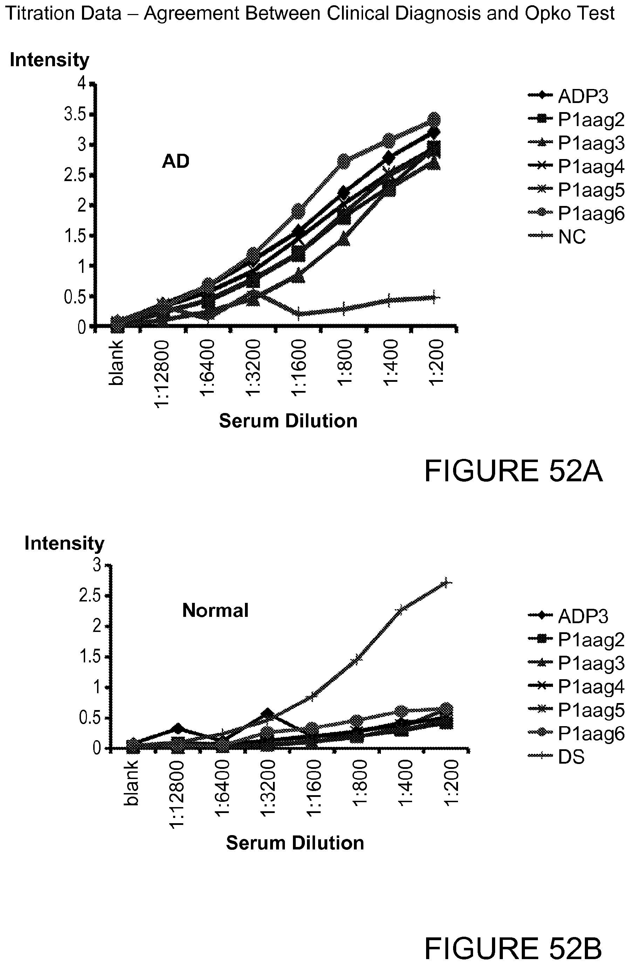

Figure 52 shows titration data that compares various AD peptoids in an ELISA test at various serum dilutions of diseased serum (A) versus normal serum (B). There is no intensity of the signals in the normal serum but clear distinction and intensity of all of the AD peptoids as the concentration increases from 1:12,800 to 1:200.

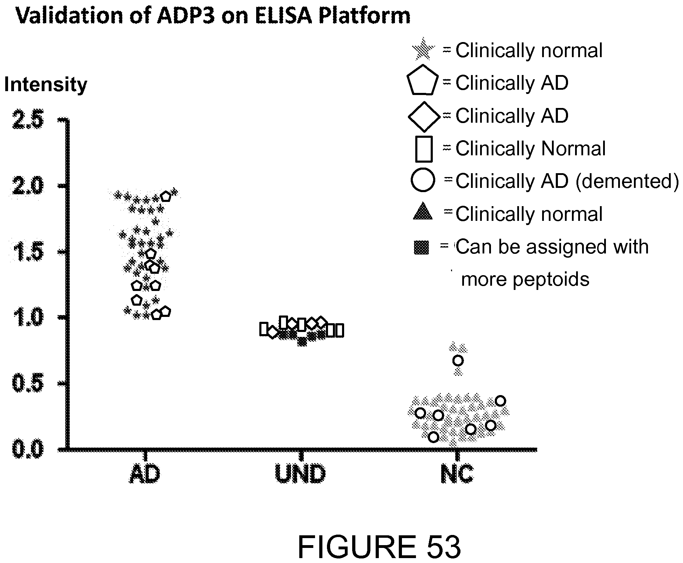

Figure 53 provides a diagram that validates the correlation between the clinical diagnosis of the unblinded sample set of AD patients at various stages of Alzheimer's disease (or not) versus the data obtained from the same patient serum samples (blinded) and which were screened against ADP3 peptoid to detect disease associated antibodies. The results shown are from a blinded study of plasma samples from Mayo Clinic Jacksonville. UND = Undecided. The plot was derived from taking a single serum concentration (1:800) dilution. A reading of >1. was considered positive, a reading between 1 and 0.7 was considered undecided and a reading below 0.7 was considered negative.

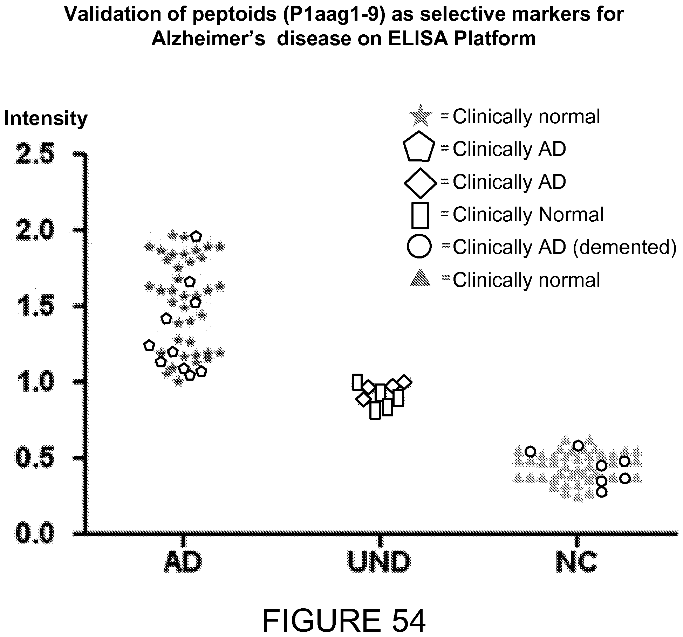

Figure 54 provides a diagram that validates the correlation between the clinical diagnosis of the unblinded sample set of AD patients at various stages of Alzheimer's disease (or not) versus the data obtained from the same patient serum samples (blinded) and which were screened against the various AD peptoids (plot is average value of results of 9 peptoids) to detect disease associated antibodies. The results shown are from a blinded study of plasma samples from Mayo Clinic Jacksonville. UND = Undecided. The plot was derived from taking a single serum concentration (1:800) dilution, A reading of > 3 was considered positive, a reading between 1 and 0.7 was considered undecided and a reading below 0.7 was considered negative.

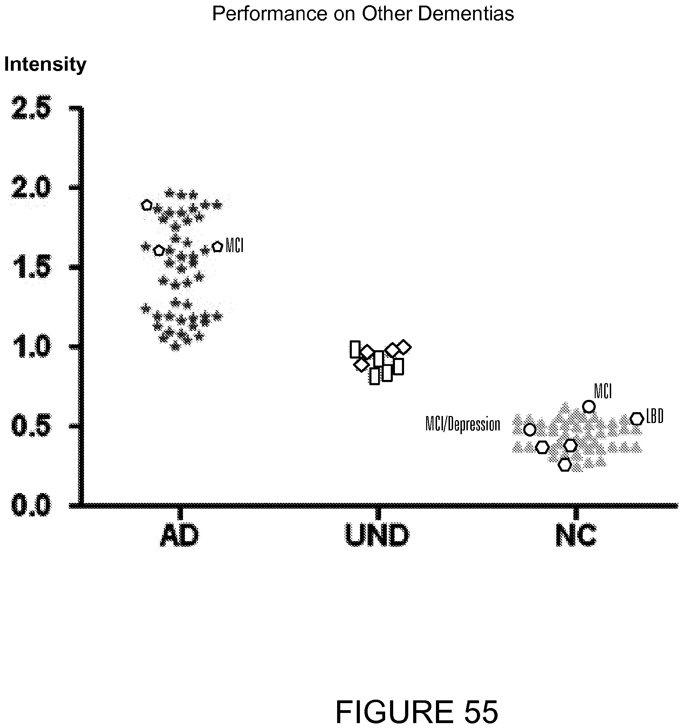

Figure 55 provides a diagram that validates the correlation between the clinical diagnosis of the unblinded sample set of AD patients at various stages of Alzheimer's disease (or not) versus the data obtained from the same patient serum samples (blinded) and which were screened against the various AD peptoids to detect disease associated antibodies. The results shown are from a blinded study of plasma samples from Mayo Clinic Jacksonville. UND = Undecided. The plot was derived from taking a single serum concentration (1:800) dilution. A reading of > 1 was considered positive, a reading between 1 and 0.7 was considered undecided and a reading below 0.7 was considered negative. The data also shows performance on other dementias where MCl/depression samples are labeled and Lewis Body Dementia samples are marked as well. The data shows that at least three MCI patients have serum samples with detectable amounts above 1 of the antibodies captured by the AD selective peptoids.

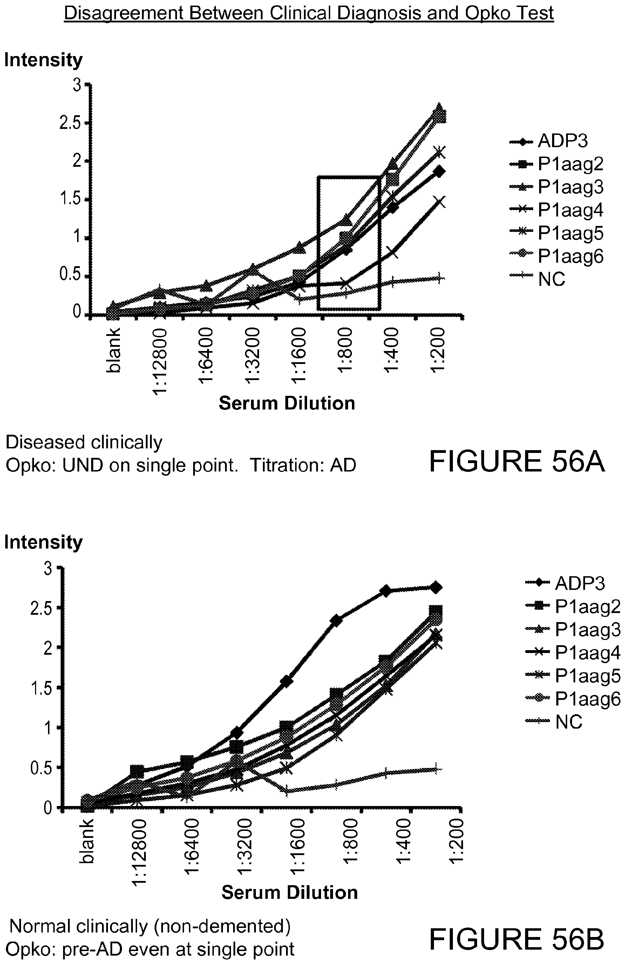

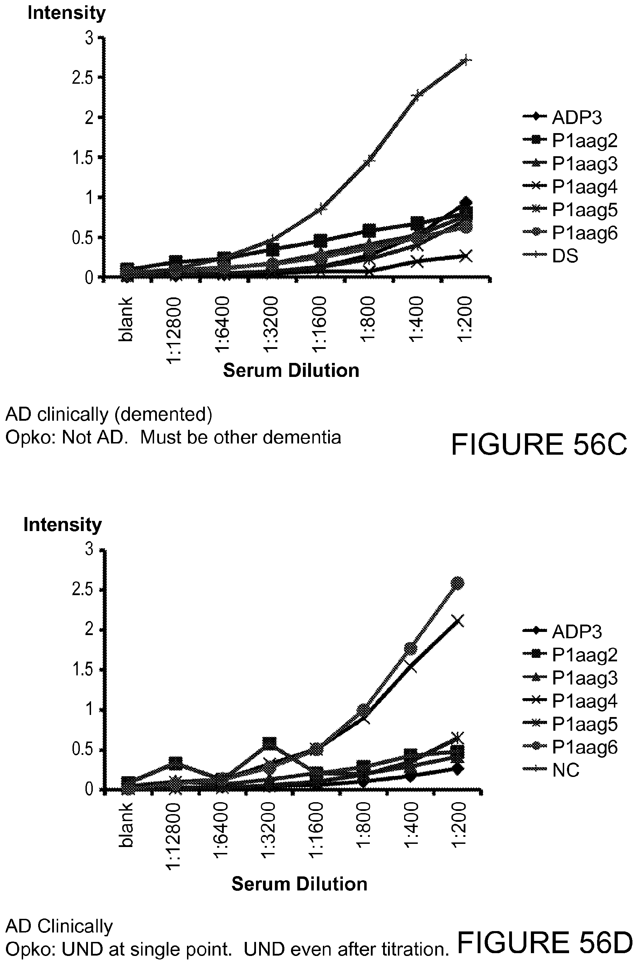

Figures 56A-D provide data on that subset of samples from patients that have disagreements between the Opko Health peptoid diagnostic assay using multiple AD peptoids versus the clinical diagnosis after this information was provided when unblinded. Figure 56A shows the data for peptoids ADP3 and others as shown for a patient that was diseased clinically but for which the Opko peptoid Plaag4 was below 1 .0 (UND at a single point; Titration AD positive). All other Opko peptoids were positive for AD (i.e., above 1.0). Figure 56B shows that all Opko peptoids were positive for disease associated antibodies in a patient that was currently diagnosed as normal (non-demented) suggesting pre-AD. Figure 56C shows that none of the Opko AD peptoids showed an intensity above 1 at any dilution point in a patient that was clinically diagnosed with AD suggesting that this patient had some other form of dementia. Figure 56D shows that in a clinically positive AD patient, multiple Opko AD peptoids were not positive for disease associated antibodies but two peptoids (Plaag6 and Plaag4) were positive, thus UND at a single point and UND even after titration.

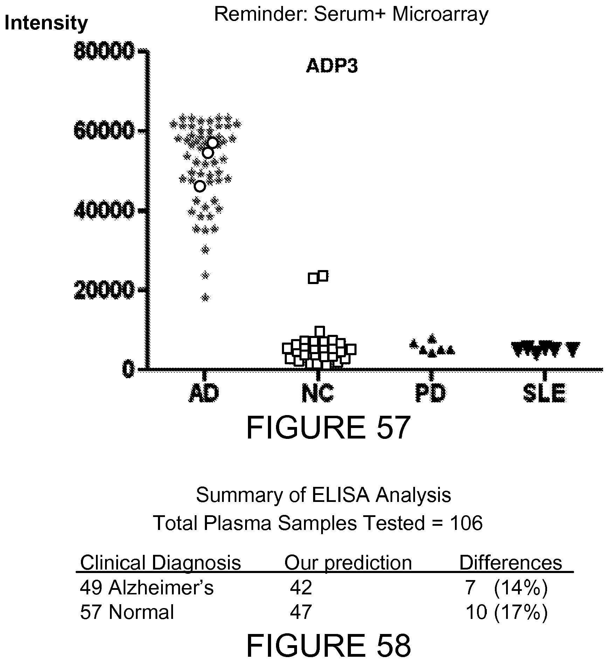

Figure 57 shows the cluster diagram generated from previous AD samples using a microarray spotted with ADP3. There is a clear correlation between diseased versus control in the microarray data and data generated using the ELISA platform. Figure 57 also shows that the ADP3 peptoid is selected for disease associated antibodies associated with Alzheimer's disease and not Parkinsons or Lupus (SLE).
Figure 58 provides a summary of ELISA analysis using a total of 106 serum samples tested.

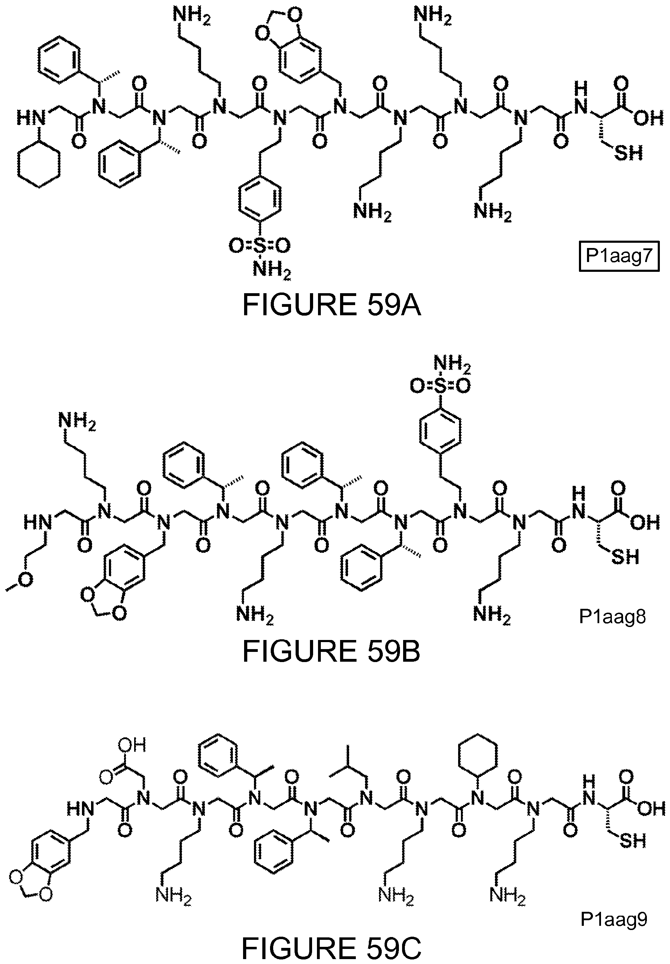

Figure 59 provides the chemical structures of Plaag7-9.

### DETAILED DESCRIPTION OF THE INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the invention.

The application discloses a ligand library and uses thereof. Specifically, it discloses the screening of molecules, pharmacoproteomics, diagnosis, treatment, and other uses of a random library.

Disclosed herein is a ligand library for a personalized medicine. The terms "personalized medicine," as used herein, may refer to the use of a test (or diagnostic) to target a drug (or therapy) at patients that are most likely to benefit therefrom, or to identify patients who may be at risk of harm from said therapy. Before a drug or diagnostic product can be marketed in the United States and most other countries, it is subjected to strict regulatory review of its safety and efficacy. In the case of a diagnostic for personalized medicine this will likely require the testing of tissue or bodily fluids from patients that received the drug to ascertain whether there is a link between their response to therapy and the presence of a particular marker.

Accordingly, provided herein is a method for diagnosing a response for a drug to treat a disease in a biological sample obtained from the subject, the method comprising: screening a ligand library against said sample; identifying binding characteristics of one or more markers in said sample with one or more ligands in the library; and determining whether said one or more markers are associated with said response for said drug, thereby diagnosing said response for said drug to treat said disease in said subject. Examples of a response include, but are not limited to, an adverse reaction, a side effect, a drug resistance, and a therapeutic efficacy including dosage efficacy.

Specifically, the invention provides a method for diagnosing a drug induced response in a subject, the method comprising screening a ligand library against a biological sample previously obtained from said subject; identifying binding characteristics of one or more markers in said sample with one or more ligands in the library; and determining whether said one or more markers are associated with said drug induced response, wherein the library comprises a compound of formula I, 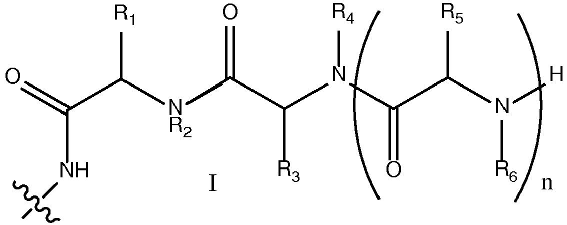 wherein
R₁ is -(C₁-C₆)SCH₃
R₂ is H; and
R₃-R₆ are independently selected from the groups consisting of H, -C₁-C₆alkyl, -C₁-C₆alkylSCH₃, -C₀-C₆alkylC₂-C₆alkenyl, -C₀-C₆alkyl C₂-C₆alkynyl, -C₁-C₆ COOH, -C₁-C6alkylOH, -C₁-C₆alkylN(R)₂, -C₃-C₈cycloalkyl, -C₁-C₆alkylaryl, -C₁-C₆alkylheteroaryl,-C₁-C₆alkylNC(O)C₁-C₆alkyl, -C₁-C₆alkylcycloamide wherein any of the aryl or heteroaryl groups may be independently substituted with -OH, Cl, F, Br, -OCH₃, -SO₂NH₂ or -O-CH₂-O-,
and wherein n is 3-11,
wherein said drug induced response is an adverse reaction to said drug, a side effect of said drug, or a resistance to said drug, thereby diagnosing said drug induced response in said subject.

Adverse drug reactions are a principal cause of the low success rate of drug development programs (less than one in four compounds that enters human clinical testing is ultimately approved for use by the U.S. Food and Drug Administration (FDA)). Drug-induced disease or toxicity presents a unique series of challenges to drug developers, as these reactions are often not predictable from preclinical studies and may not be detected in early clinical trials involving small numbers of subjects. When such effects are detected in later stages of clinical development they often result in termination of a drug development program. When a drug is approved despite some toxicity, its clinical use is frequently severely constrained by the possible occurrence of adverse reactions in even a small group of patients. The likelihood of such a compound becoming first line therapy is small (unless there are no competing products). Clinical trials that use the disclosed methods may allow for improved predictions of possible toxic reactions in studies involving a small number of subjects. The methods offer a quickly derived prediction of likely future toxic effects of an intervention. Accordingly, disclosed herein is a method for detecting a risk of adverse reaction to a drug in a subject, the method comprising: obtaining a biological sample from said subject; screening a ligand library against said subject; identifying binding characteristics of one or more markers in said sample with one or more ligands in the library; and determining whether said one or more markers are associated with said risk, thereby detecting said risk of adverse reaction to said drug in said subject.

The present invention is based, in part, on the surprising discovery that a combination of peptoid binding biomarkers can be used to individualize therapy in patients. Given the high inter-patient variability in response to a drug, the assay methods of the present invention are particularly advantageous because they utilize a combinatorial strategy that takes into account differences in binding characteristics of multiple molecular determinants (e.g., peptoid binding biomarkers) to determine whether a disease in a patient has a high likelihood of responding to treatment with a specific drug or combination of drugs. If the patient is classified as a responder, a dosing regimen tailored to that patient can then be created to achieve therapeutic efficacy without inducing toxic side-effects. Consequently, patients classified as responders can receive the full benefits of drug induced therapy without experiencing the side-effects associated with such therapy. Similarly, patients already undergoing treatment with a drug can experience a reduction in toxic side-effects without compromising therapeutic efficacy by adjusting the subsequent dose of the drug. Likewise, patients already undergoing treatment with a drug can be monitored to assess whether resistance to the drug has developed and an alternative therapy should be administered.

As a result, the methods disclosed herein enable treating a disease in a subject, the method comprising: obtaining a biological sample from said subject; screening a ligand library against said subject; identifying binding characteristics of one or more markers in said sample with one or more ligands in the library; determining whether said one or more markers are associated with a response to a drug for treating said disease; administering said drug to said subject, based on the determination of association between said one or more markers to said response, thereby treating said disease in said subject. Also disclosed herein is a method of monitoring a treatment by a drug in a subject, the method comprising: obtaining a biological sample from said subject; screening a ligand library against said subject; identifying binding characteristics of one or more markers in said sample with one or more ligands in the library; determining whether said one or more markers are associated with a response to a drug for treating said disease; administering said drug to said subject, based on the determination of association between said one or more markers to said response, thereby monitoring said treatment by said drug in said subject.

Also disclosed herein is a ligand library for pharmacoproteomics and identifying a marker associated with a response to drug. Accordingly, disclosed herein is a method for identifying a marker associated with a drug induced response for treating a disease, the method comprising: obtaining a biological sample from a subject; screening a ligand library against said biological sample; determining the binding characteristics of a marker in said sample to a ligand in the library; and determining whether said marker is associated with said drug induced response for treating said disease, thereby identifying said marker associated with said drug induced response for treating said disease. The response to a drug may be a patient's response to medication, its dosage, or adverse reaction.

As disclosed herein, one or more putative hits or leads of biomarkers may be identified using the disclosed ligand library. A biological sample may be obtained from a subject or a plurality of subjects (*e.g.,* patient population or sub-population). In some embodiments, a first set of biological samples may be obtained from a plurality of subjects exhibiting a response (*e.g*., drug induced response or response to a disease) and a second set of biological samples may be obtained from a plurality of subjects not exhibiting a response. The ligand library may be screened against the first and second set of biological samples. The differences in binding characteristics of one or more markers to one or more ligands in the library between the first and second set biological samples may be determined. Based on the differences in binding characteristics, the putative hits or leads of biomarkers can be identified. As disclosed herein, the putative hits or leads may be biomarkers that recognize antoantibodies to antigens associated with a response, for example, a patient's response to a drug, response to a disease, and response to a particular stage of a disease.

For identifying putative hits or leads of biomarkers, a bead-based large random peptoid ligand library having peptoid ligands ranging from about 350K to about 250MM may be used. The putative hits or leads of biomarkers identified in the initial random library screening may then be used to screen against the samples in a subsequent screening for diagnostics or companion diagnostics discussed herein.

The putative hits or leads may be validated in a subsequent screening. In one embodiment, the same ligand library that was used in the initial screening may be used in the validation subsequent screening. In another embodiment, a different ligand library (different from the one that was used in the initial screening) may be used in the validation subsequent screening.

In one embodiment, the putative hits or leads associated with a first trait (e.g., response to a disease) are identified in the initial screening and the identified putative hits or leads are used to screen against the samples to determine their association with a second trait (e.g., response to a drug). In one embodiment, the putative hits or leads associated with a disease are identified in the initial screening and the identified putative hits or leads are used to screen against the samples to both validate and determine an association with a specific stage of said disease.

In some embodiments, the initial screening for identifying putative hits may be performed on a bead-based device, using a large random ligand library, and the subsequent screening for diagnostics or companion diagnostics may be performed using any platform, for example, microarray, using a non-random or random ligand library.

In one embodiment, in a subsequent screening for diagnostics or companion diagnostics, a first screening may be performed against a first ligand library and a subsequent screening against a second ligand library, wherein the first ligand library comprises a first set of ligands and the second ligand library comprises a second set of ligands. In one example, the first ligand library is screened to identify markers associated with a disease, and a second ligand library is screened in a subsequent screening to identify markers associated with a drug induced response.

In one example, the putative hits or leads are used in a first screening in patient samples, collected prior to drug treatment, to determine their association with a disease. After drug treatment, the samples may be collected from patients, and in one embodiment, the same putative hits used in the pre-treatment group may be used to identify those patients having certain disease stages or responsive changes to drug treatment. In another embodiment, different putative hits may be used to monitor drug related side effects or treatment effects that are due to drug administration and are not necessarily correlated or related to the pretreatment profile. In some embodiments, another random library may be used to find additional biomarkers that might be associated with the drug treatment.

The term "drug," as used herein, may refer to any drug, including, but not limited to, a synthetic inorganic or organic compound, a protein, a peptide, a polysaccharides and other sugars, a lipid, DNA and RNA nucleic acid sequences, an antisense oligonucleotide, an antibodiy, a receptor ligand, an enzyme, an adhesion peptide, an antigen, a hormone, a growth factor, a ribozyme, and a retroviral vector.

The invention encompasses any suitable drug known one of skilled in the art. These drugs are listed in The Merck Index; Physicians' Desk Reference, PDR Network, 2011 Edition edition (December 1, 2010); U.S. Patent 7,932,294, and U.S. Patent Publications 20060046967, 20110274695, 20110269722, 20110269709, and 20060205674.

In one embodiment, the drug is selected from one or more of the following categories/groups: Antibiotics, Sedatives, Hypnotics, Antidepressants, Antipsychotics, Antimanics, Analgesics, Antipyretics, Antimigraine agents, Anticonvulsants, Drugs used in parkinsonism and movement disorders, Drugs for dementia, Antiemtics, drugs for Vertigo, CNS Stimulants activators, Antiinfective eye preparations, Antiinflammatory, antiallergic preparations, antiglucoma drugs, preparations to cure eye diseases, aural preparations, nasal preparations, oropharyngeal preparations, Antiarrhythemic drugs, Antihypertensives, alfa/beta-blockers, channel blockers, ACE inhibitors, Angiotensin II receptor antagonists, diuretics, Antianginals, nitrates, calcium channel blockers. Drugs for cardiac failure and shock, Vasodilators, Coagulants, Anticoagulants, Thrombolytics, antiplatelet drugs, Respiratory stimulants, Antitissives, Expectorants, Mucolytics, Decongestants, Antihistamine agents, antiasthmatics; Antiulcer, Antisecretory drugs, H.sub.2 receptor antagonists, Proton Pump Iinhibitors, Prostaglandin analogues, Antacids, Antispasmodics, drugs modifying intestinal motility, Antidiarrhoeals, antimotility drugs, antimicrobial drugs, drugs acting on gall bladder, Urinary antiinfectives, Diuretics, Urinary analgesics, Antispasmodics, Antiinfective drugs acting on urethra and vagina, drugs acting on uterus, Drugs for prostatic hypertrophy, alfa blockers, antiandrogens, Drugs for erectile dysfunction, Spermicidals, nonhormonal contraceptives, Emollients, keratolytics, topical antiinfectives, topical antifungals, topical parasiticidals, topical steroids, topical drugs for acne vulgaris, drugs for psoriasis, pigmentation disorders, and Antiseborrhoeics, Non Steroidal Anti Inflammatory Drugs (NSAIDs), COX-2 inhibitors, Antiarthritic agents, Immunosuppressants, Topical analgesics, Muscle relaxants, Neuromuscular Drugs, Penicillin antibiotics, Cephalosporin antibiotics, Quinolone, Fluoroquinolone antibiotics, Macrolide antibiotics, Chloramphenicol, Tetracyline antibiotics, Sulfonamides, Antianaerobics, Metronidazole, Antitubercular drugs, Antileprosy drugs, Antifungals, Antiprotozoals, Anthelminthics, Antiinfestive Drugs, Antimalarials, Antivirals, Anabolics, androgenic steroids, Corticosteroids, Oestrogens, Progestogens and Hormonal contraceptives, Fertility Agents, Trophic hormones and related drugs, Thyroid and antithyroid drugs, Antidiabetics and hyperglycaemics, Vitamins, Amino acids, Anti-obesity drugs, Hypolipidaemic drugs, fibric acid derivatives, statins, HMG CoA reductase inhibitors, nicotinic acid group, drugs used for Gout, drugs affecting bone metabolism, bisphosphonates, Anticancer drugs, alkylating agents, cytotoxic antibiotics, antimetabolites, cytarbine, Fludarbine, 5-Fluorouracil, Mercaptopurine, Thioguanine, Vinca alkaloids, Etoposide, Taxanes, Topoisomerase 1 inhibitors, Cytotoxic immunosuppressants, Immunostmulants, Cytoprotectives, Amifostine, Oestrogens, Progestogens, hormon antagonists, antineoplastic drugs, Antiallurgics, non-sedative antihistamins, Cetirizine, Desloratadine, Terfenadine, Fexofenadine, sedative histamines, histamine receptor blockers, Local anaesthetics, intravenous anaesthetics, inhalation anaesthetics, and muscle relaxants.

In another embodiment, the drug is selected from one or more of the drugs listed in Table 1 below.

**Table 1. Selected Examples of Branded Drugs in Market**

| **Generic Name** | **Brand Names** | **Indications** |
|---|---|---|
| Atorvastatin | Lipitor® | Cholesterol |
| Clopidrogel | Plavix® | Atherosclerosis |
| Etanercept | Enbrel® | RA, JRA, Ps, PsA, AS |
| Fluticasone Salmetrol | Advair® | Asthma |
| Infliximab | Remicade® | RA, UC, CD, Ps, PsA, AS |
| Valsartan | Diovan® | Hypertension |
| Rituximab | Rituxan® | NHL, RA |
| Esomaprazole | Nexium® | Ulcers |
| Bevacizumab | Avastin® | Colon cancer |
| Aripiprazole | Ability® | Schizophrenia |
| Trastuzumab | Herceptin® | Breast Cancer |
| Olanzapine | Zyprexa® | Schizophrenia |
| Quetiapine | Seroquel® | Schizophrenia |
| Adalimumab | Humira® | RA, Ps, JIA, PsA, AS, CD |
| Ontelukast | Singulair® | Asthma |
| Enoxaparin | Lovenox® | Anticoagulant DVT |
| Venlafaxine | Effexor® | Depression |
| Pioglitazone | Actos® | Diabetes |
| Candersartan | Atacand®, Blopress® | Hypertension |
| Escitalopram | Lexapro®, Cipralex® | Depression |
| Glatiramer | Copaxone® | Multiple Sclerosis |

| | | |
|---|---|---|
| NHL Non Hodgkin's Lymphoma, RA Rheumatoid Arthritis, JRA Juvenile Rheumatoid Arthritis, JIA Juvenile Idiopathic Arthritis, Ps Psoriasis, PsA Psoriatic arthritis, CD Crohn's Disease; UC Ulcerative Colitis, AS Ankylosing Spondylitis. | | |

In another embodiment, the drug is selected from one or more of the following: Abacavir, Aripiprazole, Arsenic Trioxide, Atomoxetine, Atorvastatin, Azathioprine, Boceprevir, Brentuximab Vedotin, Busulfan, Capecitabine, Carbamazepine, Carisoprodol, Carvedilol, Celecoxib, Cetuximab (1), Cetuximab (2), Cevimeline, Chlordiazepoxide and Amitriptyline, Chloroquine, Citalopram (1), Citalopram (2), Clobazam, Clomiphene, Clomipramine, Clopidogrel, Clozapine, Codeine, Crizotinib, Dapsone, Dasatinib, Desipramine, Desloratadine and Pseudoephedrine, Dexlansoprazole (1), Dexlansoprazole (2), Dextromethorphan and Quinidine, Diazepam, Doxepin, Drospirenone and Ethinyl, Estradiol, Erlotinib, Esomeprazole, Fluorouracil, Fluoxetine, Fluoxetine and Olanzapine, Flurbiprofen, Fluvoxamine (1), Fluvoxamine (2), Fluvoxamine (3), Fulvestrant, Galantamine, Gefitinib (1), Gefitinib (2), Iloperidone, Imatinib (1), Imatinib (2), Imatinib (3), Imatinib (4), Imipramine, Indacaterol, Irinotecan, Isosorbide and Hydralazine, Lapatinib, Lenalidomide, Maraviroc, Mercaptopurine, Metoprolol, Mivacurium, Modafinil (1), Modafinil (2), Nefazodone, Nelfinavir, Nilotinib (1), Nilotinib (2), Nortriptyline, Omeprazole, Panitumumab (1), Panitumumab (2), Pantoprazole, Paroxetine, Peginterferon alfa-2b, Perphenazine, Phenytoin, Pimozide, Prasugrel, Pravastatin, Propafenone, Propranolol, Protriptyline, Quinidine, Rabeprazole, Rasburicase, Rifampin, Isoniazid, Pyrazinamide, Risperidone, Sodium Phenylacetate and Sodium Benzoate, Sodium Phenylbutyrate, Tamoxifen, Telaprevir, Terbinafine, Tetrabenazine, Thioguanine, Thioridazine, Ticagrelor, Timolol, Tiotropium, Tolterodine, Tositumomab, Tramadol and Acetaminophen, Trastuzumab, Tretinoin, Trimipramine, Valproic Acid, Vemurafenib, Venlafaxine, Voriconazole, Warfarin (1), and Warfarin (2).

Also disclosed herein is a companion diagnostic to a drug that targets a known drug target, ora drug target that is undergoing a clinical trial. Examples of a drug target that is undergoing clinical trial include, but are not limited to, Bapineuzumab, Solanezumab, Intravenous immunoglobulin (IVIg), Latrepirdine (Dimebon), Scyllo-inositol/ELND 005, Methylthioninium chloride (Rember), CERE-110, PBT2, Davenutide/AL-108, BMS-708163, PF-04494700/ TTP488, Tideglusib/NP-12 (Nypta), Belimumab, Atacicept, Mapatumumab, Apomab, Dulanermin, Odanacatib, AMG-785, DG-041, OC-000459, PLX-4032, LX-1031, and LX-1032.

A binding profile of one or more sample components (e.g., biomarkers) can be used to predict, diagnose, assess, or treat, any disease, known to one of skilled in the art. The terms "disease" or "condition" are commonly recognized in the art and designate the presence of signs and/or symptoms in an individual or patient that are generally recognized as abnormal. Diseases or conditions may be diagnosed and categorized based on pathological changes. Signs may include any objective evidence of a disease such as changes that are evident by physical examination of a patient or the results of diagnostic tests. Symptoms are subjective evidence of disease or a patient's condition, i.e. the patient's perception of an abnormal condition that differs from normal function, sensation, or appearance, which may include, without limitations, physical disabilities, morbidity, pain, and other changes from the normal condition experienced by an individual. Various diseases or conditions include, but are not limited to; those categorized in standard textbooks of medicine including, without limitation, textbooks of nutrition, allopathic, homeopathic, and osteopathic medicine. In certain aspects of this invention, the disease or condition is selected from the group consisting of the types of diseases listed in standard texts such as Harrison's Principles of Internal Medicine, 14.sup.th Edition (Fauci et al, Eds., McGraw Hill, 1997), or Robbins Pathologic Basis of Disease, 6.sup.th Edition (Cotran et al, Ed. W B Saunders Co., 1998), or the Diagnostic and Statistical Manual of Mental Disorders: DSM-IV, 4.sup.th Edition, (American Psychiatric Press, 1994), or other text books. The diseases are also listed in U.S. Patent Publications 2007/0003954 and 2011/0092384.

Examples of a disease or condition include, but are not limited to cancer, autoimmune disease, inflammatory disease, infectious disease, neurodegenerative disease, cardiovascular disease, bacterial infection, viral infection, fungus infection, prion infection, physiologic state, contamination state, or health in general.

The random ligand library screening methods disclosed herein can use the binding characteristics to differentiate between different forms of a disease or its state, including pre-disease states or the severity of a disease state. For example, the methods may be used to determine the metastatic state of a cancer or the susceptibility to an agent or disease state. The disclosed methods and compositions may be for assessing ligand binding moieties present in or associated with a cancer, for example, but not limited to, breast cancer, lung cancer, prostate cancer, cervical cancer, head & neck cancer, testicular cancer, ovarian cancer, skin cancer, brain cancer, pancreatic cancer, liver cancer, stomach cancer, colon cancer, rectal cancer, esophageal cancer, lymphoma, and leukemia.

The disclosed methods and compositions may be for assessing ligand binding moieties present in autoimmune diseases, for example, but not limited to, myasthenia gravis, chronic active hepatitis, primary biliary cirrhosis, dilated cardiomyopathy, myocarditis, autoimmune polyendocrine syndrome type I (APS-I), autoimmune hepatitis, cystic fibrosis vasculitides, acquired hypoparathyroidism, goodpasture syndrome, Crohn disease, coronary artery disease, pemphigus foliaceus, pemphigus vulgaris, Guillain-Barre syndrome, Type 1 diabetes, stiff man syndrome, Rasmussen encephalitis, autoimmune gastritis, Addison disease, insulin hypoglycemic syndrome (Hirata disease), Type B insulin resistance, acanthosis, systemic lupus erythematosus (SLE), pernicious anemia, treatment-resistant Lyme arthritis, polyneuropathy, multiple sclerosis, demyelinating diseases, Rheumatic fever, atopic dermatitis, primary biliary cirrhosis, Graves disease, autoimmune hypothyroidism, vitiligo, thyroid associated ophthalmopathy, autoimmune thyroiditis, autoimmune Hashimoto thyroiditis, coeliac disease, ACTH deficiency, myositis, dermatomyositis, polymyositis, dermatomyositis, Sjogren syndrome, systemic sclerosis, progressive systemic sclerosis, systemic sclerosis, scleroderma, morphea, primary antiphospholipid syndrome, bullous pemphigoid, herpes gestationis, cicatricial pemphigoid, chronic idiopathic urticaria, connective tissue syndromes, necrotizing and crescentic glomerulonephritis (NCGN), systemic vasculitis, Wegener granulomatosis, Churg-Strauss syndrome, polymyositis, Raynaud syndrome, chronic liver disease, visceral leishmaniasis, autoimmune C1 deficiency, membrane proliferative glomerulonephritis (MPGN), prolonged coagulation time, autoimmune thrombocytopenia purpura, immunodeficiency, atherosclerosis, neuronopathy, paraneoplastic pemphigus, paraneoplastic stiff man syndrome, paraneoplastic encephalomyelitis, subacute autonomic neuropathy, cancer-associated retinopathy, paraneoplastic opsoclonus myoclonus ataxia, lower motor neuron syndrome, Lambert-Eaton myasthenic syndrome, and paraneoplastic cerebellar degeneration.

Disclosed herein are methods and compositions for assessing ligand binding moieties present in infectious diseases, for example, Acquired immunodeficiency syndrome (AIDS), Anthrax, Botulism, Brucellosis, Chancroid, Chlamydial infection, Cholera, Coccidioidomycosis, Cryptosporidiosis, Cyclosporiasis, Diphtheria, Ehrlichiosis, Arboviral Encephalitis, Enterohemorrhagic Escherichia coli (E. coli), Giardiasis, Gonorrhea, Haemophilus influenzae, Hansen's disease (leprosy), Hantavirus pulmonary syndrome, Hemolytic uremic syndrome, Hepatitis A, Hepatitis B, Hepatitis C, Human immunodeficiency virus (HIV), Legionellosis, Listeriosis, Lyme disease, Malaria, Measles, Meningococcal disease, Mumps, Pertussis (whooping cough), Plague, Paralytic Poliomyelitis (polio), Psittacosis (parrot fever), Q Fever, Rabies, Rocky Mountain spotted fever, Rubella, Congenital rubella syndrome, Salmonellosis, Severe acute respiratory syndrome (SARS), Shigellosis, Smallpox, Streptococcal disease (invasive Group A), Streptococcal toxic shock syndrome (STSS), Streptococcus pneumoniae, Syphilis, Tetanus, Toxic shock syndrome, Trichinosis, Tuberculosis, Tularemia, Typhoid fever, Vancomycin-Intermediate/Resistant Staphylococcus aureus, Varicella, Yellow fever, variant Creutzfeldt-Jakob disease (vCJD), Dengue fever, Ebola hemorrhagic fever, Echinococcosis (Alveolar Hydatid disease), Hendra virus infection, Human monkeypox, Influenza A H5N1 (avian influenza), Lassa fever, Marburg hemorrhagic fever, Nipah virus, O'nyong-nyong fever, Rift Valley fever, Venezuelan equine encephalitis, and West Nile virus.

The ligand library disclosed herein may be used to screen for any stage of a disease, for example, an early stage of a disease or an advanced late stage of a disease.

The disclosed methods and compositions for assessing ligand binding moieties present in neurodegenerative diseases, for example, stroke, hypovolemic shock, traumatic shock, reperfusion injury, multiple sclerosis, AIDS, associated dementia; neuron toxicity, Alzheimer's disease, head trauma, adult respiratory disease (ARDS), acute spinal cord injury, Huntington's disease, and Parkinson's Disease.

Disclosed are compositions which comprises particle based libraries of compounds selected from peptoids, peptides, oligomers, small molecules and any molecule naturally derived or synthetically made and which can be placed cm a support system such as a bead or small particle.

Also disclosed are compositions comprising peptoid(s) that bind antibodies indicative of a response to a drug and methods of detecting antibodies in an antibody-containing sample comprising contacting an antibody-containing sample with a support having affixed thereto a peptoid.

Ligand libraries for use in the invention include compounds of formula I, as defined above.

In one embodiment, the ligand libraries used in the methods of the invention may comprise a compound of formula I, as defined above, on a support.

Compounds of the random ligand library (e.g. the ligand libraries) for screening a complex biological fluid are fully described in U.S. Provisional Patent Application 61/467,256 and 61/491,717.

The large ligand libraries can be used directly in biological fluid, under the appropriate experimental conditions, to screen for biomarkers and without the need to use fewer support members (e.g. about 100,000 or less) or requirement to transfer such peptoids or ligands to a microarray before screening the biological fluid. In addition, the ligand libraries may also be used to screen for cell based receptors that specifically relate to a particular cell surface marker. The present disclosure, unlike prior methods, permits the inclusion of greater numbers of beads/resins and thus larger libraries in either the ligand binding agent screen or the cell receptor screen to directly screen the complex biological samples.

As previously described with respect to microarray systems, virtually any molecule or compound may be used to build a random bead or resin based library. These "molecules" or "compounds" may include natural products or man-made compounds or synthetically derived molecules. The source of such molecules can be from biological systems as well as non-biologically derived sources. The preferred ligands for purposes of the initial screen using large bead libraries under the conditions disclosed herein are made, in part, from submonomers, which are selected from any known monomeric amine and from any known acetic acid halide or substituted acetic acid halide. For example, Table 1 in U.S. Provisional Patent Application 61/467,256, provides a range of R groups on a monosubstituted amine.

In some embodiments, the monomers and/or submonotners are selected from, the group consisting of cysteine, glycine, methionine, allylamine, ethanol amine, isobutylamine, diaminobutane, methylbenzylamine (racemic or enantiomeric), piperonylamine, cyclohexylamine, 3,4 dimethoxyphenethylamine, benzylamine, N-(2-aminoethyl)acetamide, N-(3-aminopropyl)-2-pyrrolidinone, 4-(2-aminoethyl)benzenesulfonamide or furfurylamine.

Acetic acid halides and/or R substituted acetic acid, halides wherein R is selected from any amino acid side chain or from any other group including those groups or variables on the monosubstituted amines are also utilized as submonomers. Alternatively; the combination of any amine and any acetic acid halide may be reacted to form a monomer, which is then reacted, with another reactive monomer on a growing peptoid chain to form an oligomer.

Combinatorial libraries of peptoids may be prepared as follows: Peptoids having a cysteine or methionine monomeric amino acid attached to a support, or a linker on a support or resin or bead may be prepared by first adding the protected amino acid to a support or linker on a support. Following addition of said amino acid (or any amino acid desired which can serve a functional or other purpose in the oligomer or a diagnostic having said oligomer), remaining monomers can be added using standard peptide chemistry or using submonomers of bromoacetic acid (or α-substituted bromoacetic acid or similar reactant) and a monosubstituted amine wherein the amine is substituted with an R group. The R group may be selected from any known peptoid substituted including those described in, for example, U.S. Pat. Publication Nos. 2010/0303805 or 2010/0303835 and/or those described in Zuckermann and various Kodadek publications. The preferred amines are those selected from the libraries recited herein wherein particular monomeric amines are added to each library to build 1-250 MM bead or resin libraries. The preferred library size ranges from 1 MM to 50 MM beads or resins having said diversity of compounds thereon.

The process to make each peptoid generally involves (1) preparation of an amino acid reactant on a support (including an optional linker on a support); (2) reaction of the amino acid moiety on said support with an acyl halide such as bromoacetic acid or chloroacetic acid to form a halogenated derivative (3) reaction of the halogenated derivative with a monosubstitited amine to form an amide and (4) repeat of steps (2) and (3) to form a peptoid. Methionine containing peptoids are generally .made in the large libraries. Cysteine containing peptoids are typically made when larger scale quantities of high affinity peptoids are desired and following the initial screening of the large bead or resin libraries. In the large bead based libraries used to initially screen complex biological fluid such as serum, there is no need for or requirement for a long PEG linker which is typically necessary for microarray screens. A PEG linker may be on the bead or resin provided it is a short linker of less than about 10 monomeric units. In the diagnostic kits comprising heads or tentagel beads of less than about 50 microns (e.g. 10 microns), it is useful to use both short PEG linkers (e.g. between 2-10 PEG monomers) or longer PEG oligamers may be utilized.

The conditions used to perform each step in the oligomer building process utilize solvents such as DMF or acetonitrile or dichloromethane. Trifluoroacetic acid is utilized for cleavage purposes and piperidine or other suitable base is used as a base in the reaction between a bromo derivative and an amine. Various protecting groups are utilized in the preparation of the ammo acid reactant. In a preferred embodiment, diaminobutane is utilized as the first amine submonomer in the chain adjacent to the cysteine residue at the C-terminus of the peptoid. In the first step of the process, the selected beads or resins (in grain or milligram quantities) are swollen in a suitable solvent such as DMF, If the beads are "protected" with a protecting group on the reactive amine on said bead, a base solution such as piperidine is repeatedly added with subsequent washing with DMF to deprotect the head. Once the bead is deprotected or if a bead such as a tentagel bead is initially utilized, it may be reacted with a suitable ammo acid such as cysteine or rnethionine (protected with Fmoc or other suitable protecting group on the nitrogen and protected with Trt (triphenylmethyl) on the sulfur and in sufficient molar quantities to react with each bead) in a suitable solvent such as DMF. HBTU (tetramethyluronium hexafluorophosphate (coupling reagent) and 4-rnethylmorpholine (base) along with the protected amino acid are added to the bead solution in a beaker (or tube or flask) and shaken at room temperature to form the Fmoc/Trt protected amino acid on the resin (or on a linker on the resin). The beads are then washed multiple times in a solvent such as DMF. The Fmoc group is then deprotected using a suitable reagent which permits reaction of the amine on the amino acid with another reactant such as another protected amino acid or a submonomer such as bromoacetic acid and an activating agent e.g. DIG (3-isopropylcarbodiimide) in a suitable solvent under heat (microwave with stirring). The resultant beads are then washed multiple times and then treated with a desired monomeric amine (in slight mole excess) in a suitable solvent under heat. The resultant beads are washed multiple times and then treated repeatedly with bromoacetic acid and the amine of choice to build the oligomer and oligomeric library. The peptoids may be cleaved from the beads using triflouroacetic acid. Art alternative or other suitable process for peptoids comprising a preferred embodiment-e.g., those peptoids having cysteine adjacent to a monomer having a 1-yl-n-butylamine includes building a peptoid having two amino acids on the C-terminus followed by a process that further includes adding any of the monomers built in a submonomer process wherein the second arnino acid is lysine. This further includes the selection of any monomer or submonomer to make α-substituted bromoacetic acid submonomers wherein the carbon substituents may be selected from typical amino acid side chains to form, after reaction of the reactants, α-substituted peptoids wherein an R group is found on either or both of the carbon on the peptoid chain or the nitrogen on the peptoid chain. The substituents on either the α-carbon or nitrogen may be virtually any substituent as recited herein.

Combinatorial libraries of small molecules may be obtained commercially or prepared using methods known in the art. See for example, Bichler et al. 1995; Cho et al, 1999; LePiae et al., 2002; Ostergaard and Holm, 1997; Yang et al, 1999). In addition, U.S. Pat. No. 6,344,334 and publications Gallop et al, (1994), Gordon et al, (1994); Thompson and Eilman (1996) are also sources of such molecules and libraries.

Combinatorial libraries of peptides may be obtained commercially or prepared using methods known in the art. See, for example, Stewart and Young (1984); Tam et al. (1983); MerrifteM (1986); and Barany and Merritleld (1979).

Combinatorial libraries of nucleic acids including RNA or DNA may be obtained, commercially or prepared using methods known in die art. Combinatorial libraries of oligosaccharides may be obtained commercially or prepared using methods known in the art.

In each instance, the "ligands" or random ligands may be added to support resins or beads to form screening libraries can be used, under the conditions described, herein, to screen for biomarkers in complex biological fluid. The preferred ligands are peptoid ligands.

In addition to building and/or using such libraries, it may be necessary or desired to characterize, purify and/or synthesize or re-synthesize any such ligand. Such methods are known in the art and include the entire gamut of purification methods such as HPLC via chromatographic means or purification methods via chemical means; characterization methods such as mass spec or NMR or combinations of any of these methods. Such methods axe -farther described in, for example, US Pat. Publication 2007/0003954. In such cases, any such purified ligand may be referred to as a compound or substantially purified compound.

In the initial screening methodology of the invention, beads and/or resins are utilized as the support means having an oligomer operably coupled to said, support. In diagnostic kits or other kits having "hits" or "putative hits" from such initial screen, the support systems can be broadened to virtually any support system including microarrays or any other known diagnostic platforms. In these cases, it is necessary to ensure that such kits or other support, systems with the putative hits also have or are adapted to have a detector or detection methods to permit detection of ligands having ligand binding moieties attached to such ligands. The preferred detection methods include, for example, ELISA or other methods which involve the use of labeled secondary antibodies.

Supports can be made of any suitable material. Materials utilized to make such supports can include, for example, glass, plastic, ceramic or polymeric resins or beads. Supports may also include materials such as nickel, brass, steel or other metals or mixtures of metals. The supports may also be conditioned to have linkers and/or other means to bind to or connect to or react with a ligand or active group on a ligand. Such groups are also described in U.S. Pat. Pub. No. 2007/0003954. In the present invention, the number of resins or beads having individual ligands bonded thereto or to a linker and then to said support ranges from greater than 100K to about 150 million (MM). The preferred number utilized in the initial screening methods of the invention ranges between 1MM and 2MM ligands/resins.

TentaGel® resins are most preferred for the large ligand screening methodology of the invention. These resins are grafted copolymers consisting of a low crosslinked polystyrene matrix on which polyethylene glycol (PEG or FOB) is grafted. TentaGel resins are commercially available (Rapp Polymere GmbH). As PECS is a "cameleon type" polymer with hydrophobic and hydrophilic properties, the graft copolymer shows modified chemical properties. According to the manufacturer, there are in principle two ways to introduce PEG onto the modified polystyrene matrix. The simplest immobilization procedure is to couple PEG via one of its terminal hydroxyl groups to chloromethylated polystyrene according to the classical ether synthesis or to use other bifunctional PEG's for coupling onto the solid support. The manufacturer found that by means of anionic graft copolymerization setting up the PEG step by step directly on the matrix, PEG chains of molecular masses up to 20 kilo dalton have been immobilized on functionalized crosslinked polystyrenes. Graft copolymers with PEG chains of about 2000-3000 dalton proved to be optimal in respect of kinetic rates, mobility, swelling and resin capacity. As there is no procedure to get monodisperse PEG with more than 10 ethylene oxide units by any polymerization techniques, there is theoretically no way to introduce monodisperse PEG chains with more than 10 ethylene oxide units to the resin or to get monodisperse PEG by direct polymerization onto the polystyrene backbone (monodisperse is defined as: PEG without any molecular weight distribution). These graft copolymers are pressure stable and can be used in batch processes as well as under continuous flow conditions. The copolymer contains about 50 - 70 % PEG (w/w). The properties of these polymers are highly dominated by the properties of PEG and versus by the polystyrene matrix.

Setting up a chemical library or peptide library by the "one bead one compound" approach it is essential to know the number of beads which are available within a certain amount of resin as well as the capacity of single beads. Table 1 summarizes some particle sizes and correlates them to the corresponding capacity of a single bead. The calculations are based on a typical loading of TentaGel beads which are in the range of 0.25 - 0,3 mmol/g. For analytical characterization at least 5 pmol of resin-bound peptide are needed for sequencing on a bead. In order to estimate the optimum resin quantity for the library, which can be handled economically one, has to take into account the bead sizes and bead capacities. In respect to homogeneity of diffusion process and kinetic rates as well as for single bead analysis and single bead quantification, all our beads show a very narrow size distribution.

| **resin** | **size [µm]** | **beads/g** | **capacity/bead** |
|---|---|---|---|
| TentaGel NH₂ | 750 µm | 4.62 x 10³ | 65 nmol |
| TentaGel NH₂ | 500 µm | 1.5 x 10⁴ | 19 nmol |
| TentaGel NH₂ | 300 µm | 6.4 x 10⁴ | 4 nmol |
| TentaGel NH₂ | 200 µm | 2.15 x 10⁵ | 1.3 nmol |
| TentaGel NH₂ | 130 µm | 8.87 x 10⁵ | 280 - 330 pmol |
| TentaGel NH₂ | 90 µm | 2.86 x 10⁶ | 80 - 100 pmol |
| TentaGel M NH₂ | 35 µm | 4.55 x 10⁷ | 5.5 pmol |
| TentaGel M NH₂ | 20 µm | 2.4 x 10⁸ | 1.0 pmol |
| TentaGel M NH₂ | 10 µm | 1.95 x 10⁹ | 0.13 pmol |

| | | | |
|---|---|---|---|
| Correlation of particle size, number of beads per gram resin and capacity per single bead. Calculation of single bead capacity is based on a capacity of 0.25 - 0.3 nmol/g resin | | | |

There are several types of TentaGel resins available showing tailored properties dependent on their application:

### TentaGel S resins:

The PEG spacer is attached to the polystyrene backbone via an alkyl linkage. This linkage is not sensitive to acids or bases. This type of resin is a standard type of resin used for peptide synthesis, solid phase organic synthesis or combinatorial chemistry.

### TentaGel PAP resins:

The PEG is attached to the polystyrene backbone via a benzyl ether linkage. This benzyl ether linkage is sensitive to harsh acid conditions like 100 % TFA or mixtures of TFA/FMSBr.

These specially tailored resins are used for immunization procedures or for synthesizing PEG modified derivatives (PEG Attached Products), Using harsh acid conditions, the PEG spacer is cleaved together with the synthesized compound from the solid support to get soluble PEG modified compounds by applying solid phase conditions (e.g. PEG modified peptides).

### TentaGel N resins:

The PEG spacer is attached to the polystyrene backbone via a benzyl ether linkage. These tailored resins are used in oligonucleotide chemistry for small and large scale oligormcleolide synthesis. In comparison to CPG glass the capacity is increased by a factor of K).

As TentaGel resins are copolymers composed from polystyrene and polyethylene glycol, chemical and physico chemical properties of both base polymers have to be taken in account.

PEG itself is a hygroscopic polymer. It is known from literature that PEG esters are not very stable and easily hydrolyzed. Dependent on the storage conditions and. storage time, PEG itself can be oxidized along the polyether chain to form peroxides or esters. Consequently, acid treatment or treatment with bases hydrolyzes the formed PEG - esters which result in a small amount of "PEG - leakage". This leakage can be noticed by MS or NMR as PEG signals and impurities in the final product. This chemical behavior is true to all PEG's - and PEG based polymers.

| | |
|---|---|
| **T**enta**G**el S: | "S" means Standard resin, applicable to a large number of applications, useful in batch and flow through systems. |
| **T**enta**G**el R: | a special suited resin for research purpose synthesis. The resin shows an increased swelling volume but is less pressure resistant. Well suited for large peptides and difficult sequences. |
| **T**enta**G**el HL: | this highloaded version of **T**enta**G**el combines a significant higher capacity with the advantages of **T**enta**G**el resins. |
| **T**enta**G**el MB: | **T**enta**G**el Macrobeads are highlighted by extraordinary large particle diameters and high capacities based on the **T**enta**G**el technology and designed for single bead synthesis and single bead analysts. |
| **T**enta**G**el N: | this resin type is designed for automated large scale oligonucleotide synthesis. |

| | |
|---|---|
| Tenta**G**el J: | this resin type has been developed for polymeric immunoconjugates. |
| **T**enta**G**el M: | the microspherical shape of 10,20,30 µm of this **T**enta**G**el and it's monodispersity allows applications in automated sorters, for creating huge libraries, high speed synthesis etc. |
| **T**enta**G**el B: | describes bifunctional **T**enta**G**el resins, where the reactive sites on the outer surface of the bead is orthogonally protected to the reactive sites located in the internal volume of the bead and hybrid resins for sequentional cleavage. |

In addition to TentaGel beads, other resins and/or particles may be utilized build a one ligand per bead library. For example, lightly cross-linked polystyrene resins or polyamide resins may be utilized. The group that joins the substrate to the resin bead can be an essential part of solid phase synthesis. The linker is a specialized protecting group, in that much of the time, the linker will tie up a functional group, only for it to reappear at the end of the synthesis. The linker must not be affected by the chemistry used to modify or extend the attached compound. And finally the cleavage step should proceed readily and in a good yield. The best linker must allow attachment, and cleavage in quantitative yield.

In certain aspects, the support may be a bead, a plate, a dipstick, a filter, a membrane a pin, or a well. Detecting may comprise RIA, FIA, ELISA, Western blot, flow cytometry, FRET, or surface plasmon resonance.

### Carboxylic acid linkers

The first linking group used for peptide synthesis bears the name of the father of solid phase synthesis. Merrifield resin is cross-linked polystyrene functionalised with a chloromethyl group. The carbonyl group is attached by the nucleophilic displacement of the chloride with a cesium carboxylate salt in DMF. Cleavage to regenerate the carboxylic acid is usually achieved by hydrogen fluoride.

The second class of linker used for carboxylic acid is the Wang linker. Hits linker is generally attached to cross-linked polystyrene, TentaGel and polyacrylamide to form Wang resin, it was designed for the synthesis of peptide carboxylic acids using the Fmoc-protection strategy, and due to the activated benzyl alcohol design, the carboxylic acid product can be cleaved with TFA.A more acid-labile form of the Wang resin has been developed. The SA.SRIN resin has the same structure as the Wang linker but with the addition of a methoxy group to stabilise the carbomum ion formed during acid catalysed, cleavage.

### Carboxamide Linkers

The rink linker is generally preferred for generating primary carboxamide on solid phase, which may be utilized when manufacturing or re-synthesizing the hits or putative hits from the primary screen as disclosed herein. In such cases, cysteine is the first monomer reacted with the rink linker and then the process involves either subsequent monomer addition to build the oligomer or subsequent submonomer chemistry to build the oligomer. The greater acid sensitivity in the rink linker is a consequence of the two additional electron donating methoxy groups. In the generation of primary carboxamide, the starting material is attached, to the linker as a carboxylic acid and after synthetic modification is cleaved from the resin with TEA.

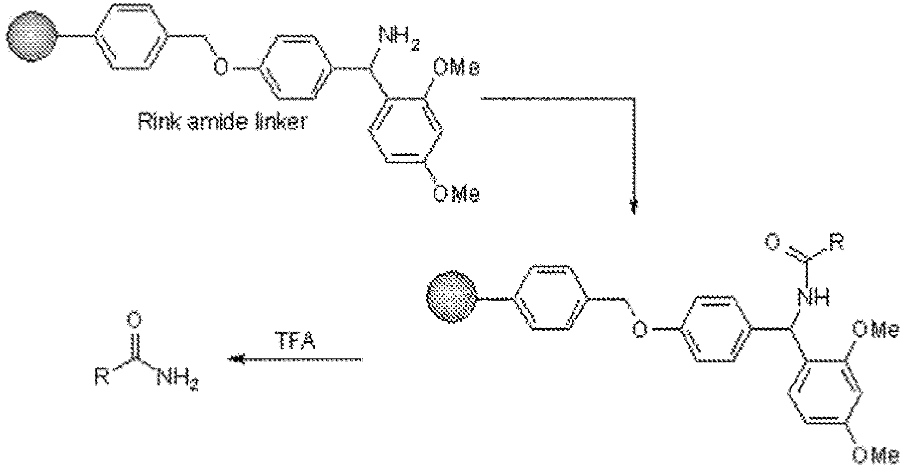

The use of Rink resin to produce carboxamide following TFA-catalysed cleavage. Alcohol Linkers

A hydroxyl linker based on the tetrahydropyranyl (THP) protecting group has been developed by Thompson and Ellmann. All type of alcohols readily add to dihydropyraa and the resulting THP protecting group is stable to strong base, but easily cleaved with acid. This linker is attached to a Merrifield resin. The trityl group is a good acid-labile protecting group for a lot of heteroatoms. The trityl group has been used to anchor alcohols in the synthesis of a library of β-mercaptoketones.

### Carbamates and Amines Linker

Carbamates linker has been used for the synthesis of a combinatorial library of 576 polyamines prepared in the search of inhibitors of trypanosomal parasitic infections. Two linkers were investigated. One based on hydroxymethylbenzoic acid 1, and the other one, an electron-donating group has been added 2. The last one allowed cleavage by TFA while the first one could be cleaved with strong acidic conditions.

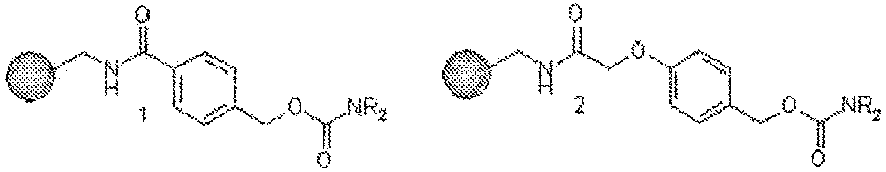

A very useful linker has been recently developed for the generation of tertiary amine. (Tertiary amines are commonly used in drag molecules.) Primary and secondary amines are introduced to the linker by Michael addition. The amine may be alkylated to gives a resin-bound quaternary ammonium ion. In mildly basic condition, Hoffmann elimination occurs to give a tertiary amines of high purity.

### Traceless Linkers

In some case, the starting materials are loaded onto the resin in one form, such as carboxylic acid, and cleaved in another form; a carboxamide for example. This is perfectly acceptable if the target, compound requires the released function. (Peptides invariably contain a carboxylic acid or carboxamide.) However, the growth in interest in combinatorial libraries of low molecular weight non-peptides has elicited a need in new types of linker. These linkers show non-specific function after cleavage, Traceless linkers are so called because an examination of the final compound reveals no trace of the point of linkage to the solid phase.

### Samples

As discussed previously, the complex biological fluids prepared for analysis in the process of the invention include or can include a host of potential biomarkers including markers expressed on cells (non-adherent cells, including T-cells or other immune effector cells), microorganisms, proteins, peptides, lipids, polysaccharides, small molecules, organic molecules, inorganic molecules, biological molecules and including any detectable or readable moiety in such complex milieu. In a preferred embodiment, such markers are antibodies and, in particular, are antibodies generated as a result of a disease or condition. In a preferred embodiment, body fluids such as serum, plasma, saliva or other fluids or samples derived from a patient or animal or organism are the source of such markers. Each sample or tissue or biologically derived, or environmentally derived or obtained sample is conditioned, or treated or diluted, or otherwise handled in order to expose said sample to either the initial screening or any subsequent screening using putative hits or ligands which have affinity for such biomarkers. The samples are diluted pursuant to the methods recited herein to provide or permit sufficient distinction between background levels or noise and signals associated with the binding of a ligand to a ligand binding moiety.

The time and/or conditions necessary to expose the ligands/supports to such samples depend upon the particular sample and other factors. The preferred conditions for the process of the claimed invention are further described herein. In almost all cases, washing or eluting steps and other conditioning means are utilized following exposure of the biological fluid to the large ligand library and/or ligands or kits derived from such library. Aqueous solutions are utilized including buffered solutions such as HEPES buffer, 'Iris buffer or phosphate buffered saline. Support systems may also be treated with energy absorbing materials to facilitate desorption or ionization of a "complex" from a support surface. Chemical means are also utilized to decouple or remove ligand-ligand binding moiety complexes from supports.

Detection methods for detecting ligand-ligand binding moiety complexes on a support include photometric and non-photometric means. Such methods include ensuring that the process includes a method to detect and measure absorbance, flurorescence, refractive index, polarization or light, scattering. These include direct and/or indirect means to measure such parameters. Methods involving fluorescence include fluorescent tagging in immunological methods such as ELISA or sandwich assay. Methods involving refractive index include surface plasmon resonance (SPR), grating coupled methods (e.g. sensors uniform grating couplers, wavelength-interrogated optical sensors (WIOS) and chirped grating couplers), resonant mirror and iiiterferomeiric techniques. Methods involving polarization include ellipsometry. Light scattering methods may also be used. Other means for tagging and/or separating and/or detecting can also include magnetic means. Magnetic resonance imaging, gas phase ion spectrometry, MRI may all be used.

Analysis of the data generated typically involves quantification of a signal due to the detected biomarker versus a control or reference. The data can be analyzed by any suitable means. Computers and computer programs may be utilized to generate and analyze the data. Beads and/or other supports may be computer coded or coded for identification purposes. Data analysis includes analysis of signal strength under the particular conditions of the assay or detection method, Ligands, ligand binding moieties or reference moieties and/or secondary detection moieties may be labeled or radio-labeled or tagged with a detectable moiety. One of ordinary skill in the art can assess the difference and/or distinction between biological fluid samples that have disease associated biomarkers versus those control or healthy patient, samples that do not contain such markers. One of ordinary skill in the art can also determine, pursuant to the methods described herein, the presence of false positives or other hits that are or may be found in control samples to account for and/or remove such "hits" and one of ordinary skill in the art, pursuant to the methods described herein, can continue the process of determining or finding disease associated biomarkers in patient samples having tray disease or condition. The "detection" of such hits, in all cases, is accomplished by means for detecting the binding of a ligand-binding moiety such as a disease associated biomarker or other marker to ligands in a Iigand library such as those described herein.

Biomarkers associated with the diseases and/or conditions recited herein will vary depending upon the particular stage of the disease and/or condition of the particular patient or animal or other organism assessed. The ligands, which are the putative hits and the compounds recited herein, are expected to, *in* most cases, mimic the natural antigen that initiates the immune response and/or formation of antibodies or immune cells in me first instance. The present invention and screening process claimed and recited herein does not require knowledge of either the particular antigen or the antibody generated in response to the antigen. The ligands, however, may be useful in their own right as vaccines or drug candidates in addition to being useful in the screens and diagnostic methods recited herein.

### Peptoid Screens:

To screen one-bead-one-compound (OBOC) combinatorial peptoid libraries, tens of thousands to millions of peptoid bearing beads are prepared *and* then mixed with a complex biological sample. The initial complex biological sample is preferably a control sample and a subsequent complex biological sample treated with a ligand library that has "removed" the control hits is then treated and/or screened against a diseased complex biological sample. The ligands/beads that interact, with at least one disease associated biomarker are then detected, identified and isolated and/or characterized. In a preferred embodiment, a Tentagel screening protocol is used which comprises (1) bead preparation, (2) screening of complex biological fluid and (3) detection of hits.

### Peptide Screens:

To screen one-bead-one-compound (OBOC) combinatorial peptide libraries, tens of thousands to millions of peptide bearing beads are prepared and then mixed with a complex biological sample following the processes described herein. The beads that interact with disease associated biomarkers are then identified and isolated for compound structure determination. For example, OBOC peptide library screening using streptavidin (SA) as probe protein, labeled with a red fluorescent dye and using the COPAS BIO-BEAD flow sorting equipment to separate fluorescent from nonfluorescent beads may be performed. See Manmi et a!., J. Comb. Chein,, 2009, 11 (1), pp 146-150. The red dyes which may be used are ATTO 590 and Texas Red. After incubating the library with the SA-red fluorescent dye conjugate, positive beads caused by peptide-SA interaction are obtained. The beads are analyzed by matrix-assisted laser desorption ionization time-of-flight mass spectrometry (MALDI-TOF MS). Thus, peptide libraries may be used in a manner that is analogous to the process described herein with peptoids wherein initial control biological fluid samples are used to remove any ligand/bead hits from the starting compound library and wherein the remaining members of the library are used to then screen for any hits in a diseased complex biological fluid sample. These hits are the putative hits which are then carried forward in any diagnostic kits.

In a similar manner, any ligand may be screened on the beads or supports using the processes described herein. These ligands include, in addition to peptoids or peptides, nucleic acid oligomers, polysaccharides, small molecules and/or any combination thereof which can be build into libraries and, under the conditions recited herein, used to screen complex biological fluid.

### Kits and Diagnostic Tools

Any of the compounds or compositions described herein may be further utilized in diagnostic kits either in a clinical or laboratory setting. These kits can range from simple point of care diagnostic assays to complex and multiplex instruments or probes. The support systems and "packaging" surrounding the core support/ligand system can be selected from current commercial kits that are designed to include the putative hits and or hits that are resynthesized and installed on such suitable platforms or they can be used in newly designed diagnostic kits. The kits will typically be accompanied by all suitable reagents and instructions to use the kits to screen for and/or diagnosis the particular disease or condition the kit is designed for. Any such kit or method will comprise at least one putative hit or ligand that has been identified pursuant to the screening method recited herein. This ligand or plurality of ligands may be selected from the same ligand or a mixture of ligands which comprise the compounds disclosed herein. The ligands may be selected based upon their affinity for a disease associated biomarker for one particular disease state or a group or battery of diseases or conditions. The preferred ligands are peptoid ligands. The kits will also contain instructions for the physicians diagnosing a particular disease or condition and specific labeling for the particular kit and disease state or condition. The present application discloses a kit including all of its essential components such as the putative peptoids or ligands found from an initial screening using any one of the libraries disclosed herein and/or known pursuant to the specific methods recited herein and labeling instructions. It is also envisioned that the particular processes and methods and materials disclosed herein may be utilized in a clinical and laboratory setting under the supervision of a skilled operator. The kits and/or instruments or equipment comprise ligands such as peptoids that are specific for disease associated antibodies and/or ceils. The "kit" may comprise a complete diagnostic kit and or screening kit or the "kit" may comprise components or sub-components containing or comprising the diagnostic peptoids, antibodies discovered and characterized through such peptoids or native antigens that are discovered and purified and/or characterized as a result of interaction with and discovery from the autoantibody.

In one embodiment, disclosed herein is a kit for diagnosis of a disease. In another embodiment, disclosed herein is a kit for treating a disease. The kit may comprise a ligand library, detection reagents for screening the ligand library against a biological sample, adjuvants for the screening, and a package insert. The package insert may include instructions for performing the diagnostic steps, instructions for determining a drug administration, and instructions for administering the drug based on the determination. In some embodiments, the kit may include a package insert that is a label approved by FDA or a drug approval authority in other countries.

The ligand libraries disclosed herein may be utilized to find and determine ligands that bind to disease associated biomarkers. Such ligands are then utilized in the kits and/or methods described generally above to assess, screen or diagnose disease states or conditions. These diagnostic methods typically involve screening for and finding disease associated biomarkers which comprise antibodies and/or other biological markers. As stated above, these antibodies can be further identified and characterized using the ligands on suitable columns to pull out or remove such antibodies from blood samples. The antibodies can in turn be used to probe for and discover the native antigen associated with such antibody.

Kits and/or other means to screen for and/or diagnose disease states or conditions must, in the first instance, be assessed against patient samples. These patient samples may be derived from normal control samples or from patient samples wherein said patient, has been identified as a patient that has or is suspected of having that disease or condition. The patient may have other symptoms associated with the disease beyond the "presence" of a disease associated biomarker. The patient may be in an early stage of the disease, may not have the disease or condition at all or may be in a late stage of a particular disease. In any clinical context and under appropriate guidelines and controls, patient and clinical samples may be provided in a blinded fashion and then assessed using the compounds as disclosed herein. The data generated as a result of the screening may then be analyzed after un-blinding to find or not find statistically significant results or correlations with known or underlying data about any particular patient or group of patients. The present application discloses a method of screening for the presence of a disease or condition comprising (1) screening a biological sample from a patient with at least one compound as disclosed herein: (2) screening a control biological sample under the same conditions using said at least one compound and (3) comparing the healthy control data versus the patient data to determine the presence or absence of a disease associated, biomarker. A group of patients or patient samples having or suspected of having disease X may be screened against a kit or diagnostic probe having at least one compound as disclosed herein and the data generated with respect to each patient may be utilized on a case by case basis to confirm or validate a disease state or condition or lack thereof. Such data generated herein may be used in combination with the total information known about that particular patient, to assess the patient's condition and to provide guidance to the medical practitioner providing treatment options. The "information" generated as a result of any such screen may be used in the clinical trial setting to assess individual patients that are taking drag therapy. The present application thus discloses a method of assessing clinical trial progression comprising use of a screen performed, according to the methods described herein. The present disclosure relates to a method of screening for or diagnosing an early disease state comprising use of a screen or compound claimed herein to detect a disease associated biomarker. The disclosure is particularly useful, in the context of early disease intervention wherein detection of such biomarkers is expected to occur well before aggressive progression of the disease. In another context; early intervention in cardiovascular disease and/or metabolic disease as well as neurological disease is expected to save lives and prevent or be useful for preventing further development of such diseases without early medical intervention or treatment.

The present application also discloses methods to increase the resolution or efficiency of the difference between a control or standard solution and the complex biological fluid containing the disease associated biomarker. For example, methods include preconditioning or pre-treating or pre-blocking the system/serum with buffers and/or conditioning agents such as E coli lysate and/or lysine.

Also discloses herein is a method of treating a subject suspected of having a disease comprising (a) contacting an antibody-containing sample from said subject with one or more supports having affixed thereto a peptoid comprising a peptoid of the formulas recited herein (b) detecting antibodies bound to said peptoids; and (c) making a treatment decision based on the result of step (b). The method may further comprise obtaining said sample from a subject. The method may also further comprise making a diagnosis of a disease for a subject from which said sample was obtained if antibody binding to the peptoid is greater than, that observed for control non-diseased patients. The method may also further comprise making a treatment decision for said subject. The sample may be contacted with more than one peptoid of formulas recited herein. The sample may be contacted with a multiplex platform for the purposes of diagnosing multiple disease states or conditions. The support may be a bead, a plate, a dipstick, a filter, a membrane a pin, or a well. The sample may be blood, serum, saliva or CSF. Detecting may comprise RIA, FLA, ELISA, Western blot, flow cytometry, FRET, or surface plasmon resonance.

Also disclosed is an antibody composition isolated from a biological fluid that is indicative of a disease, in certain embodiments the antibodies are isolated by contacting a sample having such antibodies with a peptoid composition that specifically binds antibodies indicative or associated with a disease. The antibodies can be removed, isolated, or purified from other non-antibody and non-D specific components. The antibodies can then be washed and/or disassociated from the peptoid capture agent(s).

In certain embodiments, a peptoid array made from the peptoids discovered in the process described herein is hybridized with a biological sample that has been supplemented with, a bacterial lysate, e.g., an E, coli lysate. The biological sample includes a control sample and a sample having a marker for a central nervous system disorder. For example, microarray slides are covered with a hybridization chamber and equilibrated with 1XTBST (50 mM Tris, pH 8.0, 150 mM NaCl, 0.1% Tween20) for about 15 minutes. The slides are then blocked with a bacterial lysate at a concentration at least, at most, or about 0.5, 1, 1.5, 2 mg/ml of lysate. The lysate is removed and the slides are incubated with about a milliliter of biological sample (having an approximate protein concentration of 5, 10, 15, 20 or 25 Dg/ml including ail ranges and values there between) in bacterial lysate with gentle shaking. Microarrays are then washed with 1XTBST and hybridized with labeled Anti-IgG antibodies (e.g., at 1:400 dilution). The slides are then washed with an appropriate buffer. The slides are dried using a centrifuge (e.g., 5 min spin at 1500 rpm) and scanned on a microarray scanner, for example, using a 635-nm laser at 100% power and a 600 or 650 photomultiplier tube gain. The present application also discloses a method of reducing background antisera noise in a diagnostic assay comprising treating the control plasma sample and the diseased sample with, an E. coli lysate and contacting said samples with a peptoid or ligand array. It is believed that this process can be used to support treatment of any array used to detect and distinguish antibodies in sera in the context of comparing a control sample to a diseased sample.

It is contemplated that any method or composition described herein can be implemented with respect to any other method or composition described herein.

The use of the word "a" or "an" when used in conjunction with the term "comprising" in the claims and/or the specification may mean "one," but it is also consistent with the meaning of "one or snore," "at least one," and "one or more than one."

It is contemplated that any embodiment discussed in this specification can be implemented with respect to any method of the invention, and *vice versa.* Furthermore, compositions and kits disclosed herein can be used to achieve methods of the invention.

Throughout this application, the term "about" is used to indicate that a value includes the inherent, variation of error for the device, the method being employed to determine the value, or the variation that exists among the study subjects.

It is understood that any one of the putative hits or peptoids discovered through the process recited herein may also be a therapeutic drug or vaccine candidate. The present disclosure thus relates to a process for discovering drug candidates or vaccines comprising use of the screen pursuant to the methods described herein.

The following examples are presented in order to more fully illustrate the preferred embodiments of the invention.

### EXAMPLES

### EXAMPLE 1: Library Preparation

### Protocol for Peptoid Synthesis (Cyst-Peptoid or Methionine-Peptoid)

The following example demonstrates how peptoid libraries were generated. The materials utilized in the example include reaction flasks or beakers, plastic tubing, 10-15 3 ml syringes with needles. Latex gloves, 10-15 15 ml polypropylene test tubes and micropipettes with solvent safe tips (1000 µl), glass pipettes and Resin beads. The chemicals and/or reagents utilized included N,N Dimethylformamide, Bromoacetic acid (BMA), Anhydrous Dimethylformamide, Piperidine,, Acetonitrile, 3-diisopropylcarbodimide (D1C), Trifluoroacetie acid, 5(6)-Carboxyfluorescein, Dichloromethane (DCM), and 4-Methylmorpholine (NMM). The various amines utilized in each library preparation were also used as well as HBTU (Tetramethyluronium Hexafluorophosphate) and triethylsilane.

### Peptoid Preparation

The concentration of each amine used in the process is calculated using following the formula: V-FW/d/1000x2Mx5ml

### Procedure:

### Step 1

Swelling of the resin beads.
(a) 250 milligrams of resin beads were placed into a clean dried reaction flask and 5 mls of hydrous DMF was added to the beads which were allowed to swell over a period of an hour or less. The beads were then washed with DMF multiple times (2 or 3X) under vaccum.

### Step 2

Steps (b), (c) and (d) were omitted when "unprotected beads" (e.g.,TeMa∼Gel) were used.

20% solution of Piperidine (base) using anhydrous DMF as the solvent was used in the following process:
The following process comprising steps (b), (c) and (d) was done 2 times when using "protected beads"
(b) 2.5 ml of 20% piperidine solution was added to the protected beads;
(c) After adding piperidine, the reaction flask was placed on a shaker/incubator for 20 minutes, set at 200 rpm @ 25 °C .
(d) the reaction flask was then washed 8-10 times with hydrous DMF using 5 mls of DMF.

The following solutions were also prepared:
468 mg Fmoc-Cys(Trt)-OH in anhydrous DMF (2 ml volume)(solution A).
161.6 mg of NMM, in anhydrous DMF 2 ml
303.2mg HBTU was added to NMM vial (solution B).

### Addition of HBTU/NMM and Fmoc-Cys

1 ml of each of solution A and solution 8 was added to the beads - (HBTU/NMM) and Fmoc-Cys(Trt)-OH) and shake for 1 hour.

The beads were washed in DMF 5-10 times.

The remaining 1 ml solution of solution A and B were added to the beads which were shaken for a period of 1 hour and then washed again in DMF 5-10 times.

The following solutions were also prepared;
20% Piperidine (in anhydrous DMF)
2M Bromoacetic acid
50% D1C/A. DMF
2M solution of each amine

The following steps (a), (b) and (c) were performed 2 times, 2.5 ml of 20% piperidine solution was added; (b) the reaction flask was shaken at 200 rpm at 25 degrees C and then (c) the beads were washed with DMF 8X to 10X.

A 10 ml solution of 2M Bromoacetic acid was prepared.

A 10 ml solution of 50% 3.2M DlC/anhydrous DMF (v/v) was also prepared.

2M amine solutions were prepared of each amine in and for each library.

For peptoid synthesis, 1 ml of 2M stock solution was used each time an amine was added on the peptoid chain.

### Step 3

1 ml of Bromoacetic acid was added to the reaction vessel;
(b) 1 ml of 50% DIC/DMF solution was then added and the resultant solution, was (c) rnicrowaved for 15 seconds @ 10% power.

Step (c) was performed 2 times swirling the flask side to side between sets of micro waving.

A white precipitate was formed after each micro waving step. The beads were then washed 8-10 times with DMF.

### Step 4

One ml of the first amine in the sequence was added to the reaction flask containing the bromo intermediate from the preceding step and the vessel was shaken to evenly distribute the amine on the beads. The reaction was then initiated using the microwave for 15 seconds @ 10% power 2 times. The reacted beads were then washed with hydrous DMF 8-10 times.

Steps 3 and 4 were repeated until all amines were added to make the target peptoids.

### Step 5

The heads were then washed with dichloromethane (DCM) 3 times and allowed to dry.

### Step 6

The peptoids were then cleaved from the beads using a 95% TFA solution (5 mls). The peptoids were then collected off the beads which were washed with a solvent (CH3CN and water) to remove residual peptoids. Argon gas was used to remove any residual TFA. The peptoids were then lyophilized and characterized and purified as necessary.

The reaction conditions specified above may be modified on an as-needed basis depending upon the quantities needed for any particular bead composition.

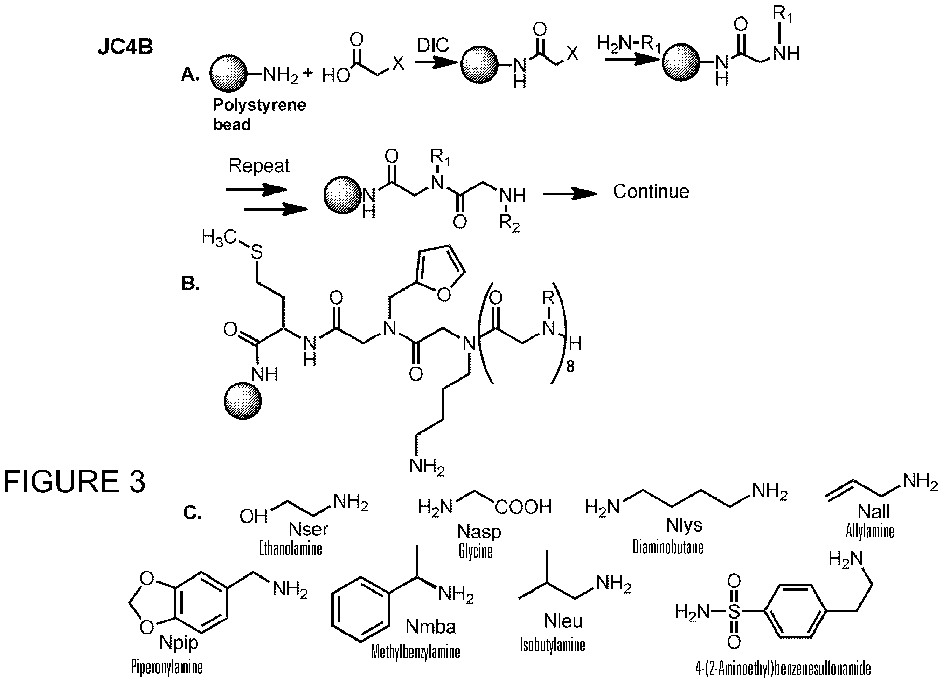

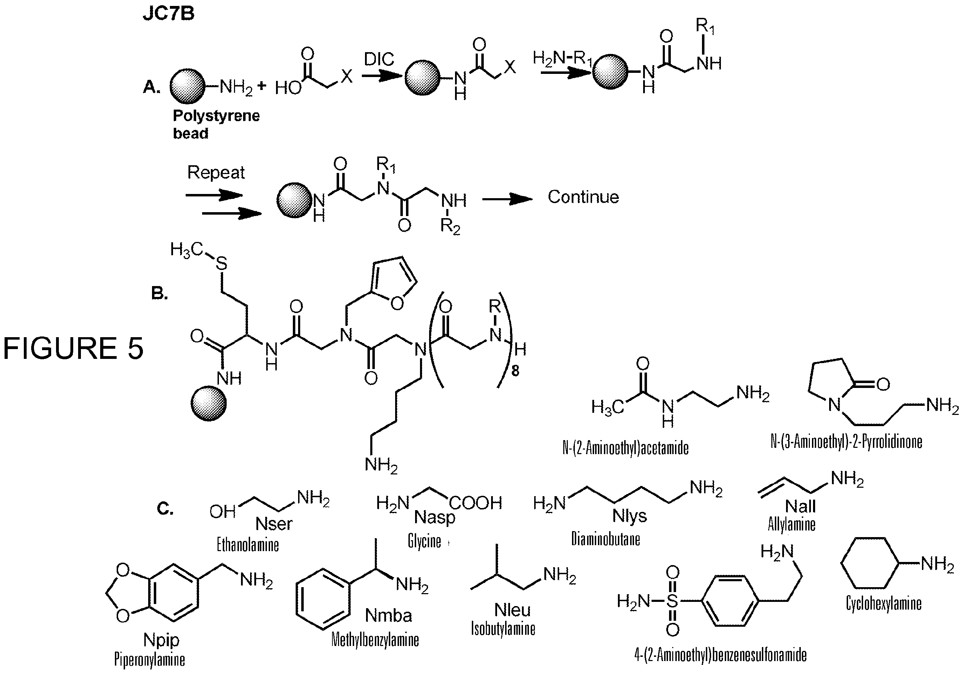

Figures 1-5 generally demonstrate how the library was prepared for a disease, for example, AD diagnostics, pancreatic cancer diagnostics and lupus. In general the beads having an. amine moiety were linked to an amino acid residue through a series of steps using standard peptide chemistry which was then reacted with an activated carbonyl moiety having a halide group which was then reacted with a monomeric amine having an R group. Steps 2 and 3 of the cycle were repeated as shown in the Figures to create large peptoid libraries having 1 MM to 2MM distinct ligands. The initial screening library prepared on Tentagel resin or beads typically had a methionine amino acid as the first monomer in the chain. The present inventor uses such an amino acid to facilitate cleavage from a bead or resin that does not have a cleavable linker. The Rink resin used to build the cysteine containing peptoids have linkers which do not need or require the use of methionine as the first amino acid. The cysteine containing peptoids were typically resynthesized after the initial screen found the putative hits. The cysteine sulfur group permits reaction of the peptoid chain with, for example, another reactive moiety on a diagnostic platform substrate. The peptoids which were resynthesized also contained a 1-yl-n-butylamino moiety as the first side chain in the chain after the amino acid amine. It is believed this group is necessary to display the peptoid and to solubilize the peptoid in aqueous containing solutions.

### EXAMPLE 2: General Screening Methodology

160 micron Tentagei beads attached to a peptoid of choice were swelled overnight in DMF. Beads were then washed ten times in a reaction vessel with Millipore water and vigorous shaking. Fresh Millipore water was added each time, and on the 10^{lh} wash, beads were allowed to shake overnight at 150-200 rpm. The next day, beads were washed in the same fashion with IX TBST and allowed to shake at 150-200 rpm for at least 3 hours.

Beads were then split evenly into 15 ml conical tubes, about 0.5 grams per tube in IX TBST. TBST was removed, and 4ml of diluted normal human serum was added to each tube. Serum stock made in IX TBST was Nano-dropped to get desired concentration of 20ug/ml. Tubes containing serum and heads were then tumbled overnight at 4 degrees Celsius in the dark. Serum was then pipetted out of the tubes, and replaced with 4ml IX TBST. Tubes were then slowly inverted to re-suspend then beads, and then allowed to settle. TBST was removed and added twice more, for a total of three TBST washes.

Secondary antibody solution was then prepared, by preparing 5ul of goat anti-human igG Qdot 655 per 1ml IX TBST. Once the last TBST addition was removed from the beads, 4ml of the Qdot solution was added, and beads were tumbled for 2 hours at four degrees Celsius in the dark. The beads were then allowed to settle, and the Qdot solution was removed. The beads were then washed three times with 4ml of IX TBST. Beads were then poured into a clear Petri dish of viewed under a UV microscope containing a DAP I filter. All beads stained red were removed.

After the first screen was completed, beads were poured back into 15 ml conical tubes, and tumbled at four degrees Celsius for at least four hours before the next serum sample addition. Disease serum was then added to the beads in the same fashion as normal serum addition, with the exception that the serum was diluted in PBS starting block as opposed to IX TBST. However, the original stock was prepared in IX TBST in order to obtain the proper concentration with the nanodrop. The serum addition and secondary antibody addition is the same as with the normal serum.

Once diseased "hits" were removed, they were pooled into a 1.5ml eppendorf tube, and heated at 95 degrees Celsius for 25-30 minutes in 1% SDS. The SDS was then removed from the tube, and replaced with Millipore water. Beads were then tumbled for 15 minutes at four degrees Celsius. The water was then replaced with fresh water, and beads were tumbled for another 15 minutes. The water was then removed and replaced with 50/50 Acetonitrile/water solution and allowed to tumble another 15 minutes. Beads were then separated into individual wells in a 96 well plate and allowed to dry.

A solution of 20-30mg Cyanogen Bromide, 500ul Acetonitrile, 400ul Glacial Acetic Acid, and 100ul Millipore water was made, and 20ul of solution was added to each well containing a hit bead. The plate was covered and allowed to shake for 16 hours at 100 rpm. The cover was then removed, and the cleavage solution was evaporated from the wells. The hits were then spotted onto a MSMS plate and sequenced using a 4800 MALD1/TOF/FOF analyzer.

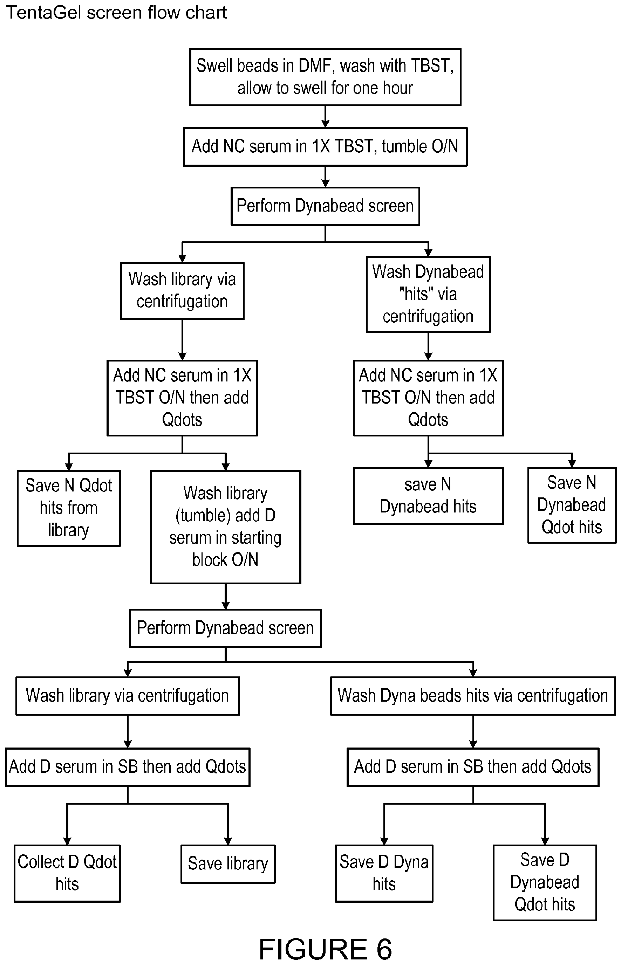

Figure 6 provides a general schematic of the screening methodology disclosed and claimed herein.

### EXAMPLE 3: Diagnosing a Response to Drug for Treating a Disease

Five hundred milligrams of 160 micron Tentagel beads (JC3B library) is added to a fifteen milliliter conical tube. Five milliliters of DMF is added to the tube, and the beads are allowed to sit overnight to swell. The next day, DMT' is pipetted out of the tube and replaced with five milliliters of IX TBST. The tube is inverted to mix, and beads are then allowed to settle to the bottom and 1X TBST is removed. Five milliliters of 1X TBST is added and removed five more times.

Normal serum samples are prepared by adding 4 milliliters of PBS starting block to a tube, and adding 7ul of each of four separate drug treated samples to the same tube. The serum is added to the washed beads and beads and serum are allowed to tumble overnight at four degrees Celsius in the dark. The next morning, beads are removed from the tumbler and allowed to settle before the serum is pippeted out of the tube. Four milliliters of IX TBST is added to the tube, and the tube is inverted to mix. The TBST is then pipetted out of the tube and replaced with four milliliters of fresh IX TBST and removed again.

DYNA-bead solution is then prepared by adding 50 ul of well-mixed goat anti-human lgG DYNA beads to four milliliters of IX TBST. The mixture is then added to the washed beads. The beads are then allowed to rumble for two hours at four degrees Celsius in the dark.

Without washing the beads, the DYNA beads screen is performed. The tube is placed in a magnet holder and filled to the brim with IX TBST. The magnet and tube are slowly agitated for two minutes, and the beads are allowed to settle in the magnet holder. The TBST and free beads that settled to the bottom are removed carefully, to not touch the hit beads attached to the sides by the magnet and replaced with fresh IX TBST, The process is repeated two to three times, until no beads could be seen attached to the sides of the tube. The hit beads are then combined into one tube.

Remaining non-hit beads are divided into 15 milliliter tubes, inverted and quickly pulsed centrifuged. The supernatant is removed and replaced with fresh IX TBST. This process repeated 6-8 times until no more DYNA beads are visible in the bead/TBST solution. The hit beads are washed in the same fashion.

Beads are combined back into a 15 ml tube, and normal serum is added to the beads in the same way as previously stated, and allowed to tumble overnight at 4 degrees Celsius in the dark. In addition, three Milliliters of each four normal samples is added to 1 milliliter of PBS starting block, and this solution is added to the DYNA bead "hit" bead tube. The next day, beads are washed in the same fashion as with the normal serum addition.

20 ul of goat-anti human IgG Quantum Dot 655 is diluted in 4 milliliter IX TBST (20ul Qdot in I Ml IX TBST for the "hit" tube), and added to the beads. The solution is tumbled for two hours at four degrees Celsius in the dark. Both hit and non-hit tube is washed four times with IX TBST and screened for bright red beads under a UV microscope. Remaining beads are tumbled in tour milliliters of IX TBST for one hour, and disease or drug treated serum sample is added in the same fashion as the normal serum sample. The magnetic screen and Qdot additions are performed in the same manner as previously stated. The hits are then sequenced on a MALD1 TOF/TOF mass spectrometer.

### EXAMPLE 4: Microarray Data with A Single Measurement

Microarrays were prepared as described in U.S. Pat. Publication No. 2010/0303805, Microarray slides are covered with hybridization chamber and equilibrated with IXTBST (50 roM Iris, pH 8.0, 150 mM NaCL 0.1% Tween20) for 15 minutes. The slides are then blocked with 1 ml of blocking buffer for 1 hour at 4°C. The blocking buffer is removed and the slides are incubated with 1 nil of serum (20mg/ml) for 16 hours at 4°C with gentle shaking. In an alternative method, the slides are blocked with 1 nil of *E. coli* lysate (1.5 mg/ml) for 1 hour at 4°C. The *E. coli* lysate is removed and the slides are incubated with 1 ml of serum (I5mg/ml) in *E. coli* lysate (1.5 mg/ml) for 18 hours at 4°C with gentle shaking. Microarrays are then washed three times with IXTBST and hybridized with Alexa-647 labeled Anti *IgG* antibody (5mg/ml) for 2 hours on orbital shaker at 4°C. The chamber cassettes were removed from microarray slides and washed with IXTBST (3x15 min) followed by 0.1XTBST (1x10). The slides are then dried on centrifuge (5 min at 1500RPM) and scanned on microarray scanner (Gene Pix Autoloader 4200) by using 635-nm laser at 100% power and 600 or 650 photomultiplier tube gain. All the scanned images were analyzed by the Gene Fix Pro 6.0 software and Genespring software.

### EXAMPLE 5: ELISA Protocol

96 well Maleimide-activated plates were obtained from Thermo Scientific, and washed three times with 400uL/well wash buffer (0.1M sodium phosphate, 0.15M sodium chloride, 0.05% Tween 20, pH 7.2), using a plate washer from Beckman Coulter. The peptoid of interest was diluted to lOmM in PBS binding buffer (0.1M sodium phosphate, 0.15M sodium chloride, lOmM EDTA, p.H 7.2), and 200ul of the peptoid solution was added to the appropriate wells. The plate was then allowed to incubate in the dark for two hours at room temperature with shaking at 500 rpm. The peptoid solution was then aspirated from the wells using die plate washer, and again washed three times with 400ul/well of wash buffer. L-Cysteine HCL: H20 (Thermo Scientific) was diluted to 10ug/mL in binding buffer, and 200ul per well was added. The plate was then incubated for one hour in the dark at room temperature with shaking at 500 rpm, and washed three times. 200ul StartingBloek™ (PBS) Blocking Buffer (Thermo Scientific) was added to the wells and the plate was incubated for one hour at 4° Celsius in the dark with shaking at 500 rpm. The plate was washed three times with the plate washer, and serum samples were prepared by serially diluting in binding buffer from 1:200 downward. Concentrations of the 1:200 sample stocks were taken using a nanodrop (Thermo Scientific), to make sure that they were similar. Each diluted sample was voilexed before preparing the next dilution. 200 ul of the appropriate dilution for serum (both disease and normal) was added to die plate, as well as binding buffer without serum as a control. The serum was allowed to incubate for two hours at room temperature in the dark with 500 rpm shaking. The plate was again washed, and 200ul of a 1;30,000 dilution of goat anti-human IgG HRP (MilHpore) in binding buffer was added to the appropriate wells and incubated at; room temperature for 30 minutes with 500 rpm shaking in the dark. The plate was washed three times, and 100 ul of TMB (3,3',5,5'∼tetramethylbenzidine) solution was added to each well, and color was allowed to develop for 30 minutes on the bench in the dark. LOOul of 2M Sulfuric acid stop solution was added to stop the reaction, and the wells were read at an absorbance of 450 using a plate reader.

Thus, in each case and with respect to each drug treatment, the process of the invention may be utilized to rapidly discover biomarkers associated with a drug response (e.g., an adverse reaction, a drug resistance, and a therapeutic dosage efficancy) and ligands which bind to such markers. These ligands-this larger pool of ligands- can then be used for multiple diagnostic and/or therapeutic purposes. The diagnostic platforms include microarrays, bead based methods and ELISA systems. The conditions utilized above comprise an important aspect of the invention. These conditions include dilution ranges for sera as well as the concentration of a particular peptoid on a bead or in a well and detection methods. The number of beads having a peptoid on a bead may vary depending upon the particular test kit or screening kit. These numbers may also vary depending upon whether beads/ligands are used in the initial screening protocol and method recited herein and/or are used in a test kit based upon the discovery of a high affinity ligand.

## Claims

1. A method for diagnosing a drug induced response in a subject, the method comprising:
screening a ligand library against a biological sample previously obtained from said subject;
identifying binding characteristics of one or more markers in said sample with one or more ligands in the library; and determining whether said one or more markers are associated with said drug induced response,
wherein the library comprises a compound of formula I, 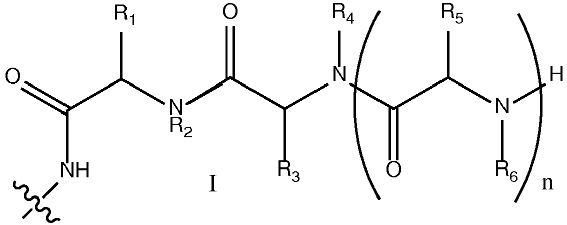 wherein
R₁ is -(C₁-C₆)SCH₃;
R₂ is H; and
R₃-R₆ are independently selected from the groups consisting of H, -C₁-C₆alkyl, -C₁-C₆alkylSCH₃, -C₀-C₆alkylC₂-C₆alkenyl, -C₀-C₆alkyl C₂-C₆alkynyl, -C₁-C₆ COOH, -C₁-C₆alkylOH, -C₁-C₆alkylN(R)₂, -C₃-C₈cycloalkyl, -C₁-C₆alkylaryl, -C₁-C₆alkylheteroaryl, -C₁-C₆alkylNC(O)C₁-C₆alkyl, -C₁-C₆alkylcycloamide wherein any of the aryl or heteroaryl groups may be independently substituted with -OH, Cl, F, Br, -OCH₃, -SO₂NH₂ or -O-CH₂-O-,
wherein said drug induced response is an adverse reaction to said drug, a side effect of said drug, or a resistance to said drug, thereby diagnosing said drug induced response in said subject, and
wherein n is 3-11.

2. The method of claim 1, wherein the library comprise a compound of formula I, 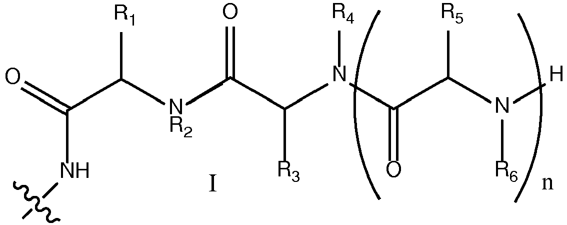 wherein the compounds are produced by a process which comprises use of a reactant selected from the group consisting of
(A) furfurylamine; 3,4-dimethoxyethanolamine; benzylamine; N-(2-aminoethyl)acetamide; N-(3-aminopropyl)-2-pyrrolidinone; ethanolamine; glycine; diaminobutane; allylamine; piperonylamine; methylbenzylamine; isobutylamine;4-(2-aminoethyl)benzenesulfonamide or cyclohexylamine; or
(B) methoxyethylamine; piperonylamine; cyclohexylamine; diaminobutane; methylbenzylamine; isobutylamine; furfurylamine or 4-(2-aminoethyl)benzenesulfonamide; or
(C) furfurylamine, ethanolamine; glycine; diaminobutane; allylamine; piperonylamine; methylbenzylamine; isobutylamine or 4-(2-aminoethyl)benzenesulfonamide; or
(D) furfurylamine, N-(2-aminoethyl)acetamide; N-(3-aminoethyl)-2-pyrrolidinone; ethanolamine; glycine; diaminobutane; allylamine; piperonylamine; methylbenzylamine; isobutylamine; 4-(2-aminoethyl)benzenesulfonamide; or
(E) cysteine, glycine, allylamine, ethanolamine, isobutylamine, methylbenzylamine, piperonylamine, methionine, cyclohexylamine, 3,4-dimethoxyphenethylamine, benzylamine, N-(2-aminoethyl)acetamide, N-(3-aminopropyl)-2-pyrrolidone, 4-(2-aminoethyl)benzenesulfonamide and furfurylamine; and
wherein,
R₁ is -(C₁-C₆)SCH₃;
R₂ is H;
R₃ and R₅ are independently selected from the groups consisting of H, -C₁-C₆alkyl, -C₁-C₆alkylSCH₃, -C₀-C₆alkylC₂-C₆alkenyl, -C₀-C₆alkyl C₂-C₆alkynyl, -C₁-C₆ COOH, -C₁-C₆alkylOH, -C₁-C₆alkylN(R)₂, -C₃-C₈cycloalkyl, -C₁-C₈alkylaryl, -C₁-C₆alkylheteroaryl, -C₁-C₆alkylNC(O)C₁-C₆alkyl, -C₁-C₆alkylcycloamide wherein any of the aryl or heteroaryl groups may be independently substituted with -OH, Cl, F, Br, -OCH₃, -SO₂NH₂ or -O-CH₂-O-;
R₄ is selected from the group consisting of furfuryl or-(C₁-C₆alkyl)NR₇R₈, and
R₆ is selected from the group consisting of H, 1-yl-allyl, 1-yl-2-hydroxyethyl, isobutyl, 1-yl-n-butylamine, methylbenzyl, piperonyl, cyclohexyl, 1-yl-2-(3,4-dimethoxyphenyl)ethyl, benzyl, 1-yl-2-(acetamide)ethyl, 1-yl-3-2-pyrrolidinone, 1-yl-2-(4-benzenesulfonamide)ethyl or furfuryl.

3. The method of claim 1, wherein the library comprises a compound having a formula Ia 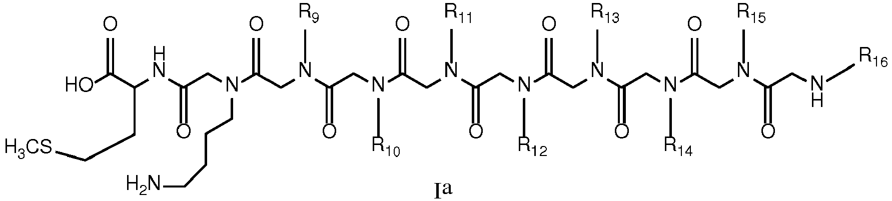 wherein the compound is selected from the group consisting of a compound of formula Ia wherein,
(a) R₉ is n-butylamine; R₁₀ is 1-yl-n-butylamine; R₁₁ is piperonyl; R₁₂ is methylbenzyl; R₁₃ is piperonyl; R₁₄ is methylbenzyl; R₁₅ is 1-yl-2-(4(benzenesulfonamide)ethyl and R₁₆ is 1-yl-2-methoxyethyl;
(b) R₉ is 1-yl-n-butylamine; R₁₀ is 1-yl-2-(4(benzenesulfonamide)ethyl; R₁₁ is 1-yl-n-butylamine; R₁₂ is cyclohexyl; R₁₃ is 1-yl-2-methoxyethyl; R₁₄ is 1-yl-2,2-dimethylethyl (isobutyl); R₁₅ is 1-yl-2-(4(benzenesulfonamide)ethyl and R₁₆ is methylbenzyl;
(c) R₉ is 1-yl-n-butylamine; R₁₀ is 1-yl-n-butylamine; R₁₁ is piperonyl; R₁₂ is 1-yl-2-(4(benzenesulfonamide)ethyl; R₁₃ is 1-yl-n-butylamine; R₁₄ is methylbenzyl; R₁₅ is methylbenzyl and R₁₆ is cyclohexyl;
(d) R₉ is piperonyl; R₁₀ is 1-yl-n-butylamine; R₁₁ is isobutyl; R₁₂ is 1-yl-2-(4(benzenesulfonamide)ethyl; R₁₃ is methylbenzyl; R₁₄ is cyclohexyl; R₁₅ is isobutyl and R₁₆ is 1-yl-n-butylamine;
(e) R₉ is 1-yl-n-butylamine; R₁₀ is 1-yl-n-butylamine; R₁₁ is methylbenzyl; R₁₂ is 1-yl-2-methoxyethyl; R₁₃ is cyclohexyl; R₁₄ is cyclohexyl; R₁₅ is methylbenzyl and R₁₆ is piperonyl;
(f) R₉ is piperonyl; R₁₀ is 1-yl-n-butylamine; R₁₁ is isopropyl; R₁₂ is isopropyl; R₁₃ is 1-yl-2-methoxyethyl; R₁₄ is cyclohexyl; R₁₅ is 1-yl-2-(4(benzenesulfonamide)ethyl and R₁₆ is 1-yl-2-(4(benzenesulfonamide)ethyl;
(g) R₉ is 1-yl-n-butylamine; R₁₀ is 1-yl-n-butylamine; R₁₁ is piperonyl; R₁₂ is methylbenzyl; R₁₃ is piperonyl; R₁₄ is methylbenzyl; R₁₅ is 1-yl-2-(4(benzenesulfonamide)ethyl and R₁₆ is cyclohexyl;
(h) R₉ is 1-yl-n-butylamine; R₁₀ is 1-yl-n-butylamine; R₁₁ is methylbenzyl; R₁₂ is 1-yl-2-methoxyethyl; R₁₃ is cyclohexyl; R₁₄ is cyclohexyl; R₁₅ is methylbenzyl and R₁₆ is piperonyl;
(i) R₉ is 1-yl-n-butylamine; R₁₀ is methylbenzyl; R₁₁ is methylbenzyl; R₁₂ is 1-yl--n-butylamine; R₁₃ is 1-yl-n-butylamine; R₁₄ is cyclohexyl; R¹⁵ is 1-yl-2-(4(benzenesulfonamide)ethyl and R¹⁶ is cyclohexyl;
(j) R₉ is 1-yl-2-(4(benzenesulfonamide)ethyl; R₁₀ is methylbenzyl; R₁₁ is methylbenzyl; R₁₂ is cyclohexyl; R₁₃ is 1-yl-n-butylamine; R₁₄ is methylbenzyl; R₁₅ is isobutyl and R₁₆ is 1-yl-n-butylamine;
(k) R₉ is 1-yl-2-methoxyethyl; R₁₀ is isobutyl; R₁₁ is 1-yl-2-(4(benzenesulfonamide)ethyl; R₁₂ is piperonyl; R₁₃ is 1-yl-n-butylamine; R₁₄ is cyclohexyl; R₁₅ is methylbenzyl and R₁₆ is 1-yl-2-methoxyethyl;
(I) R₉ is 1-yl-n-butylamine; R₁₀ is 1-yl-2-(4(benzenesulfonamide)ethyl; R₁₁ is 1-yl-n-butylamine; R₁₂ is cyclohexyl; R₁₃ is 1-yl-2-methoxyethyl; R₁₄ is isobutyl; R₁₅ is 1-yl-2-(4(benzenesulfonamide)ethyl and R₁₆ is methylbenzyl;
(m) R9 is 1-yl-2-methoxyethyl; R10 is 1-yl-n-butylamine; R11 is 1-yl-n-butylamine; R12 is 1-yl-2-methoxyethyl; R13 is methylbenzyl; R14 is 1-yl-n-butylamine; R15 is furfuryl and R16 is furfuryl;
(n) R9 is cyclohexyl; R10 is cyclohexyl; R11 is 1-yl-n-butylamine; R12 is furfuryl; R13 is 1-yl-2-methoxyethyl; R14 is 1-yl-2-methoxyethyl; R15 is 1-yl-2-(4(benzenesulfonamide)ethyl and R16 is furfuryl;
(o) R9 is 1-yl-n-butylamine; R10 is piperonyl; R11 is 1-yl-2-(4(benzenesulfonamide)ethyl; R12 is 1-yl-2-methoxyethyl; R13 is methylbenzyl; R14 is 1-yl-n-butylamine; R15 is 1-yl-2-methoxyethyl and R16 is methylbenzyl;
(p) R9 is cyclohexyl; R10 is cyclohexyl; R11 is piperonyl; R12 is 1-yl-n-butylamine; R13 is 1-yl-n-butylamine; R14 is 1-yl-n-butylamine; R15 is 1-yl-n-butylamine and R16 is isobutyl;
(q) R9 is piperonyl; R10 is 1-yl-n-butylamine; R11 is 1-yl-2-methoxyethyl; R12 is 1-yl-2-(4(benzenesulfonamide)ethyl; R13 is piperonyl; R14 is 1-yl-n-butylamine; R15 is methylbenzyl and R16 is methylbenzyl;
(r) R9 is methylbenzyl; R10 is methylbenzyl; R11 is methylbenzyl; R12 is 1-yl-n-butylamine; R13 is piperonyl; R14 is 1-yl-n-butylamine; R15 is piperonyl and R₁₆ is 1-yl-n-butylamine;
(s) R₉ is 1-yl-n-butylamine; R₁₀ is 1-yl-n-butylamine; R₁₁ is methylbenzyl; R₁₂ is 1-yl-n-butylamine; R₁₃ is methylbenzyl; R₁₄ is methylbenzyl; R₁₅ is 1-yl-2-methoxyethyl and R₁₆ is piperonyl;
(t) R₉ is methylbenzyl; R₁₀ is 1-yl-2-(4(benzenesulfonamide)ethyl; R₁₁ is 1-yl-n-butylamine; R₁₂ is 1-yl-2-(4(benzenesulfonamide)ethyl; R₁₃ is isobutyl; R₁₄ is 1-yl-n-butylamine; R₁₅ is methylbenzyl and R₁₆ is 1-yl-2-methoxyethyl;
(u) R₉ is 1-yl-2-methoxyethyl; R₁₀ is 1-yl-n-butylamine; R₁₁ is isobutyl; R₁₂ is isobutyl; R₁₃ is cyclohexyl; R₁₄ is 1-yl-n-butylamine; R₁₅ is 1-yl-2-(4(benzenesulfonamide)ethyl and R₁₆ is cyclohexyl;
(v) R₉ is isobutyl; R₁₀ is 1-yl-n-butylamine; R₁₁ is 1-yl-n-butylamine; R₁₂ is methylbenzyl; R₁₃ is 1-yl-n-butylamine; R₁₄ is 1-yl-n-butylamine; R₁₅ is piperonyl and R₁₆ is piperonyl;
(w) R₉ is 1-yl-2-(4(benzenesulfonamide)ethyl; R₁₀ is isobutyl; R₁₁ is methylbenzyl; R₁₂ is 1-yl-2-methoxyethyl; R₁₃ is 1-yl-2-(4(benzenesulfonamide)ethyl; R₁₄ is isobutyl; R₁₅ is 1-yl-2-methoxyethyl and R₁₆ is cyclohexyl;
(x) R₉ is furfuryl; R₁₀ is furfuryl; R₁₁ is piperonyl; R₁₂ is cyclohexyl; R₁₃ is piperonyl; R₁₄ is 1-yl-n-butylamine; R₁₅ is 1-yl-2-(4(benzenesulfonamide)ethyl and R₁₆ is cyclohexyl;
(y) R₉ is piperonyl; R₁₀ is piperonyl; R₁₁ is 1-yl-2-methoxyethyl; R₁₂ is 1-yl-2-methoxyethyl; R₁₃ is 1-yl-n-butylamine; R₁₄ is 1-yl-n-butylamine; R₁₅ is 1-yl-n-butylamine and R₁₆ is 1-yl-2-methoxyethyl;
(z) R₉ is piperonyl; R₁₀ is 1-yl-n-butylamine; R₁₁ is isobutyl; R₁₂ is 1-yl-2-(4(benzenesulfonamide)ethyl; R₁₃ is methylbenzyl; R₁₄ is cyclohexyl; R₁₅ is isobutyl and R₁₆ is 1-yl-n-butylamine;
(aa) R₉ is 1-yl-2-(4(benzenesulfonamide)ethyl; R₁₀ is 1-yl-2-(4(benzenesulfonamide)ethyl; R₁₁ is methylbenzyl; R₁₂ is methylbenzyl; R₁₃ is 1-yl-n-butylamine; R₁₄ is 1-yl-n-butylamine; R₁₅ is 1-yl-2-(4(benzenesulfonamide)ethyl and R₁₆ is 1-yl-2-(4(benzenesulfonamide)ethyl;
(bb) R₉ is 1-yl-n-butylamine; R₁₀ is 1-yl-2-methoxyethyl; R₁₁ is 1-yl-n-butylamine; R₁₂ is isobutyl; R₁₃ is cyclohexyl; R₁₄ is 1-yl-n-butylamine; R₁₅ is 1-yl-n-butylamine and R₁₆ is piperonyl;
(cc) R₉ is cyclohexyl; R₁₀ is methylbenzyl; R₁₁ is cyclohexyl; R₁₂ is piperonyl; R₁₃ is 1-yl-n-butylamine; R₁₄ is 1-yl-2-(4(benzenesulfonamide)ethyl; R₁₅ is 1-yl-n-butylamine and R₁₆ is 1-yl-2-methoxyethyl;
(dd) R₉ is 1-yl-2-methoxyethyl; R₁₀ is 1-yl-2-(4(benzenesulfonamide)ethyl; R₁₁ is 1-yl-n-butylamine; R₁₂ is 1-yl-2-methoxyethyl; R₁₃ is 1-yl-2-(4(benzenesulfonamide)ethyl; R₁₄ is 1-yl-2-methoxyethyl; R₁₅ is isobutyl and R₁₆ is cyclohexyl;
(ee) R₉ is 1-yl-2-methoxyethyl; R₁₀ is methylbenzyl; R₁₁ is 1-yl-n-butylamine; R₁₂ is 1-yl-n-butylamine; R₁₃ is piperonyl; R₁₄ is isobutyl; R₁₅ is 1-yl-2-(4(benzenesulfonamide)ethyl and R₁₆ is 1-yl-n-butylamine;
(ff) R₉ is 1-yl-n-butylamine; R₁₀ is methylbenzyl; R₁₁ is 1-yl-2-(4(benzenesulfonamide)ethyl; R₁₂ is methylbenzyl; R₁₃ is 1-yl-n-butylamine; R₁₄ is 1-yl-2-(4(benzenesulfonamide)ethyl; R₁₅ is 1-yl-2-(4(benzenesulfonamide)ethyl and R₁₆ is cyclohexyl;
(gg) R₉ is 1-yl-n-butylamine; R₁₀ is 1-yl-2-methoxyethyl; R₁₁ is 1-yl-n-butylamine; R₁₂ is 1-yl-2-(4(benzenesulfonamide)ethyl; R₁₃ is 1-yl-2-methoxyethyl; R₁₄ is 1-yl-2-methoxyethyl; R₁₅ is 1-yl-n-butylamine and R₁₆ is methylbenzyl;
(hh) R₉ is cyclohexyl; R₁₀ is cyclohexyl; R₁₁ is methylbenzyl; R₁₂ is 1-yl-n-butylamine; R₁₃ is methylbenzyl; R₁₄ is cyclohexyl; R₁₅ is methylbenzyl and R₁₆ is 1-yl-n-butylamine;
(ii) R₉ is 1-yl-n-butylamine; R₁₀ is furfuryl; R₁₁ is methylbenzyl; R₁₂ is 1-yl-2-(4(benzenesulfonamide)ethyl; R₁₃ is furfuryl; R₁₄ is cyclohexyl; R₁₅ is methylbenzyl and R₁₆ is cyclohexyl;
(jj) R₉ is 1-yl-n-butylamine; R₁₀ is methylbenzyl; R₁₁ is 1-yl-n-butylamine; R₁₂ is 1-yl-2-(4(benzenesulfonamide)ethyl; R₁₃ is 1-yl-2-methoxyethyl; R₁₄ is methylbenzyl; R₁₅ is 1-yl-2-methoxyethyl and R₁₆ is isobutyl;
(kk) R₉ is 1-yl-2-(4(benzenesulfonamide)ethyl; R₁₀ is 1-yl-n-butylamine; R₁₁ is 1-yl-n-butylamine; R₁₂ is methylbenzyl; R₁₃ is methylbenzyl; R₁₄ is cyclohexyl; R₁₅ is 1-yl-2-(4(benzenesulfonamide)ethyl and R₁₆ is methylbenzyl;
(II) R₉ is 1-yl-2-(4(benzenesulfonamide)ethyl; R₁₀ is 1-yl-2-(4(benzenesulfonamide)ethyl; R₁₁ is 1-yl-n-butylamine; R₁₂ is 1-yl-n-butylamine; R₁₃ is methylbenzyl; R₁₄ is 1-yl-n-butylamine; R₁₅ is methylbenzyl and R₁₆ is 1-yl-n-butylamine;
(mm) R₉ is 1-yl-n-butylamine; R₁₀ is 1-yl-2-(4(benzenesulfonamide)ethyl; R₁₁ is 1-yl-2-(4(benzenesulfonamide)ethyl; R₁₂ is 1-yl-2-methoxyethyl; R₁₃ is 1-yl-n-butylamine; R₁₄ is cyclohexyl; R₁₅ is 1-yl-2-(4(benzenesulfonamide)ethyl and R₁₆ is methylbenzyl;
(nn) R₉ is 1-yl-n-butylamine; R₁₀ is 1-yl-n-butylamine; R₁₁ is piperonyl; R₁₂ is 1-yl-2-(4(benzenesulfonamide)ethyl; R₁₃ is 1-yl-n-butylamine; R₁₄ is methylbenzyl; R₁₅ is methylbenzyl and R₁₆ is cyclohexyl;
(oo) R₉ is 1-yl-2-(4(benzenesulfonamide)ethyl; R₁₀ is methylbenzyl; R₁₁ is methylbenzyl; R₁₂ is 1-yl-n-butylamine; R₁₃ is methylbenzyl; R₁₄ is piperonyl; R₁₅ is 1-yl-n-butylamine and R₁₆ is 1-yl-2-methoxyethyl;
(pp) R₉ is piperonyl; R₁₀ is 1-yl-n-butylamine; R₁₁ is 1-yl-n-butylamine; R₁₂ is methylamine; R₁₃ is piperonyl; R₁₄ is 1-yl-n-butylamine; R₁₅ is piperonyl and R₁₆ is 1-yl-2-methoxyethyl;
(qq) R₉ is cyclohexyl; R₁₀ is cyclohexyl; R₁₁ is furfuryl; R₁₂ is 1-yl-2-methoxyethyl; R₁₃ is isobutyl; R₁₄ is cyclohexyl; R₁₅ is methylbenzyl and R₁₆ is methylbenzyl;
(rr) Rg is piperonyl; R₁₀ is 1-yl-n-butylamine; R₁₁ is isobutyl; R₁₂ is isobutyl; R₁₃ is 1-yl-2-methoxyethyl; R₁₄ is cyclohexyl; R₁₅ is 1-yl-2-(4(benzenesulfonamide)ethyl and R₁₆ is 1-yl-2-(4(benzenesulfonamide)ethyl;
(ss) R₉ is cyclohexyl; R₁₀ is cyclohexyl; R₁₁ is 1-yl-n-butylamine; R₁₂ is methylbenzyl; R₁₃ is 1-yl-n-butylamine; R₁₄ is methylbenzyl; R₁₅ is cyclohexyl and R₁₆ is piperonyl;
and pharmaceutically acceptable salts thereof.

4. The method of claim 1, wherein the library comprises a compound of the formula: 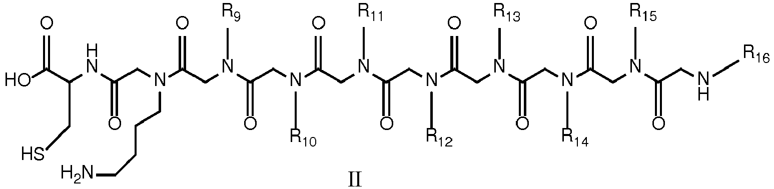 wherein the compound is selected from the group consisting of a compound of formula II wherein,
(a) R₉ is n-butylamine; R₁₀ is 1-yl-n-butylamine; R₁₁ is piperonyl; R₁₂ is methylbenzyl; R₁₃ is piperonyl; R₁₄ is methylbenzyl; R₁₅ is 1-yl-2-(4(benzenesulfonamide)ethyl and R₁₆ is 1-yl-2-methoxyethyl;
(b) Rg is 1-yl-n-butylamine; R₁₀ is 1-yl-2-(4(benzenesulfonamide)ethyl; R₁₁ is 1-yl-n-butylamine; R₁₂ is cyclohexyl; R₁₃ is 1-yl-2-methoxyethyl; R₁₄ is 1-yl-2,2-dimethylethyl (isobutyl); R₁₅ is 1-yl-2-(4(benzenesulfonamide)ethyl and R₁₆ is methylbenzyl;
(c) Rg is 1-yl-n-butylamine; R₁₀ is 1-yl-n-butylamine; R₁₁ is piperonyl; R₁₂ is 1-yl-2-(4(benzenesulfonamide)ethyl; R₁₃ is 1-yl-n-butylamine; R₁₄ is methylbenzyl; R₁₅ is methylbenzyl and R₁₆ is cyclohexyl;
(d) R₉ is piperonyl; R₁₀ is 1-yl-n-butylamine; R₁₁ is isobutyl; R₁₂ is 1-yl-2-(4(benzenesulfonamide)ethyl; R₁₃ is methylbenzyl; R₁₄ is cyclohexyl; R₁₅ is isobutyl and R₁₆ is 1-yl-n-butylamine;
(e) Rg is 1-yl-n-butylamine; R₁₀ is 1-yl-n-butylamine; R₁₁ is methylbenzyl; R₁₂ is 1-yl-2-methoxyethyl; R₁₃ is cyclohexyl; R₁₄ is cyclohexyl; R₁₅ is methylbenzyl and R₁₆ is piperonyl;
(f) Rg is piperonyl; R₁₀ is 1-yl-n-butylamine; R₁₁ is isopropyl; R₁₂ is isopropyl; R₁₃ is 1-yl-2-methoxyethyl; R₁₄ is cyclohexyl; R₁₅ is 1-yl-2-(4(benzenesulfonamide)ethyl and R₁₆ is 1-yl-2-(4(benzenesulfonamide)ethyl;
(g) Rg is 1-yl-n-butylamine; R₁₀ is 1-yl-n-butylamine; R₁₁ is piperonyl; R₁₂ is methylbenzyl; R₁₃ is piperonyl; R₁₄ is methylbenzyl; R₁₅ is 1-yl-2-(4(benzenesulfonamide)ethyl and R₁₆ is cyclohexyl;
(h) Rg is 1-yl-n-butylamine; R₁₀ is 1-yl-n-butylamine; R₁₁ is methylbenzyl; R₁₂ is 1-yl-2-methoxyethyl; R₁₃ is cyclohexyl; R₁₄ is cyclohexyl; R₁₅ is methylbenzyl and R₁₆ is piperonyl;
(i) Rg is 1-yl-n-butylamine; R₁₀ is methylbenzyl; R₁₁ is methylbenzyl; R₁₂ is 1-yl--n-butylamine; R₁₃ is 1-yl-n-butylamine; R₁₄ is cyclohexyl; R₁₅ is 1-yl-2-(4(benzenesulfonamide)ethyl and R₁₆ is cyclohexyl;
(j) R₉ is 1-yl-2-(4(benzenesulfonamide)ethyl; R₁₀ is methylbenzyl; R₁₁ is methylbenzyl; R₁₂ is cyclohexyl; R₁₃ is 1-yl-n-butylamine; R₁₄ is methylbenzyl; R₁₅ is isobutyl and R₁₆ is 1-yl-n-butylamine;
(k) R₉ is 1-yl-2-methoxyethyl; R₁₀ is isobutyl; R₁₁ is 1-yl-2-(4(benzenesulfonamide)ethyl; R₁₂ is piperonyl; R₁₃ is 1-yl-n-butylamine; R₁₄ is cyclohexyl; R₁₅ is methylbenzyl and R₁₆ is 1-yl-2-methoxyethyl;
(I) Rg is 1-yl-n-butylamine; R₁₀ is 1-yl-2-(4(benzenesulfonamide)ethyl; R₁₁ is 1-yl-n-butylamine; R₁₂ is cyclohexyl; R₁₃ is 1-yl-2-methoxyethyl; R₁₄ is isobutyl; R₁₅ is 1-yl-2-(4(benzenesulfonamide)ethyl and R₁₆ is methylbenzyl;
(m) R₉ is 1-yl-2-methoxyethyl; R₁₀ is 1-yl-n-butylamine; R₁₁ is 1-yl-n-butylamine; R₁₂ is 1-yl-2-methoxyethyl; R₁₃ is methylbenzyl; R₁₄ is 1-yl-n-butylamine; R₁₅ is furfuryl and R₁₆ is furfuryl;
(n) Rg is cyclohexyl; R₁₀ is cyclohexyl; R₁₁ is 1-yl-n-butylamine; R₁₂ is furfuryl; R₁₃ is 1-yl-2-methoxyethyl; R₁₄ is 1-yl-2-methoxyethyl; R₁₅ is 1-yl-2-(4(benzenesulfonamide)ethyl and R₁₆ is furfuryl;
(o) Rg is 1-yl-n-butylamine; R₁₀ is piperonyl; R₁₁ is 1-yl-2-(4(benzenesulfonamide)ethyl; R₁₂ is 1-yl-2-methoxyethyl; R₁₃ is methylbenzyl; R₁₄ is 1-yl-n-butylamine; R₁₅ is 1-yl-2-methoxyethyl and R₁₆ is methylbenzyl;
(p) R₉ is cyclohexyl; R₁₀ is cyclohexyl; R₁₁ is piperonyl; R₁₂ is 1-yl-n-butylamine; R₁₃ is 1-yl-n-butylamine; R₁₄ is 1-yl-n-butylamine; R₁₅ is 1-yl-n-butylamine and R₁₆ is isobutyl;
(q) R₉ is piperonyl; R₁₀ is 1-yl-n-butylamine; R₁₁ is 1-yl-2-methoxyethyl; R₁₂ is 1-yl-2-(4(benzenesulfonamide)ethyl; R₁₃ is piperonyl; R₁₄ is 1-yl-n-butylamine; R₁₅ is methylbenzyl and R₁₆ is methylbenzyl;
(r) R₉ is methylbenzyl; R₁₀ is methylbenzyl; R₁₁ is methylbenzyl; R₁₂ is 1-yl-n-butylamine; R₁₃ is piperonyl; R₁₄ is 1-yl-n-butylamine; R₁₅ is piperonyl and R₁₆ is 1-yl-n-butylamine;
(s) R₉ is 1-yl-n-butylamine; R₁₀ is 1-yl-n-butylamine; R₁₁ is methylbenzyl; R₁₂ is 1-yl-n-butylamine; R₁₃ is methylbenzyl; R₁₄ is methylbenzyl; R₁₅ is 1-yl-2-methoxyethyl and R₁₆ is piperonyl;
(t) R₉ is methylbenzyl; R₁₀ is 1-yl-2-(4(benzenesulfonamide)ethyl; R₁₁ is 1-yl-n-butylamine; R₁₂ is 1-yl-2-(4(benzenesulfonamide)ethyl; R₁₃ is isobutyl; R₁₄ is 1-yl-n-butylamine; R₁₅ is methylbenzyl and R₁₆ is 1-yl-2-methoxyethyl;
(u) R₉ is 1-yl-2-methoxyethyl; R₁₀ is 1-yl-n-butylamine; R₁₁ is isobutyl; R₁₂ is isobutyl; R₁₃ is cyclohexyl; R₁₄ is 1-yl-n-butylamine; R₁₅ is 1-yl-2-(4(benzenesulfonamide)ethyl and R₁₆ is cyclohexyl;
(v) Rg is isobutyl; R₁₀ is 1-yl-n-butylamine; R₁₁ is 1-yl-n-butylamine; R₁₂ is methylbenzyl; R₁₃ is 1-yl-n-butylamine; R₁₄ is 1-yl-n-butylamine; R₁₅ is piperonyl and R₁₆ is piperonyl;
(w) Rg is 1-yl-2-(4(benzenesulfonamide)ethyl; R₁₀ is isobutyl; R₁₁ is methylbenzyl; R₁₂ is 1-yl-2-methoxyethyl; R₁₃ is 1-yl-2-(4(benzenesulfonamide)ethyl; R₁₄ is isobutyl; R₁₅ is 1-yl-2-methoxyethyl and R₁₆ is cyclohexyl;
(x) R₉ is furfuryl; R₁₀ is furfuryl; R₁₁ is piperonyl; R₁₂ is cyclohexyl; R₁₃ is piperonyl; R₁₄ is 1-yl-n-butylamine; R₁₅ is 1-yl-2-(4(benzenesulfonamide)ethyl and R₁₆ is cyclohexyl;
(y) R₉ is piperonyl; R₁₀ is piperonyl; R₁₁ is 1-yl-2-methoxyethyl; R₁₂ is 1-yl-2-methoxyethyl; R₁₃ is 1-yl-n-butylamine; R₁₄ is 1-yl-n-butylamine; R₁₅ is 1-yl-n-butylamine and R₁₆ is 1-yl-2-methoxyethyl;
(z) Rg is piperonyl; R₁₀ is 1-yl-n-butylamine; R₁₁ is isobutyl; R₁₂ is 1-yl-2-(4(benzenesulfonamide)ethyl; R₁₃ is methylbenzyl; R₁₄ is cyclohexyl; R₁₅ is isobutyl and R₁₆ is 1-yl-n-butylamine;
(aa) Rg is 1-yl-2-(4(benzenesulfonamide)ethyl; R₁₀ is 1-yl-2-(4(benzenesulfonamide)ethyl; R₁₁ is methylbenzyl; R₁₂ is methylbenzyl; R₁₃ is 1-yl-n-butylamine; R₁₄ is 1-yl-n-butylamine; R₁₅ is 1-yl-2-(4(benzenesulfonamide)ethyl and R₁₆ is 1-yl-2-(4(benzenesulfonamide)ethyl;
(bb) Rg is 1-yl-n-butylamine; R₁₀ is 1-yl-2-methoxyethyl; R₁₁ is 1-yl-n-butylamine; R₁₂ is isobutyl; R₁₃ is cyclohexyl; R₁₄ is 1-yl-n-butylamine; R₁₅ is 1-yl-n-butylamine and R₁₆ is piperonyl;
(cc) Rg is cyclohexyl; R₁₀ is methylbenzyl; R₁₁ is cyclohexyl; R₁₂ is piperonyl; R₁₃ is 1-yl-n-butylamine; R₁₄ is 1-yl-2-(4(benzenesulfonamide)ethyl; R₁₅ is 1-yl-n-butylamine and R₁₆ is 1-yl-2-methoxyethyl;
(dd) R₉ is 1-yl-2-methoxyethyl; R₁₀ is 1-yl-2-(4(benzenesulfonamide)ethyl; R₁₁ is 1-yl-n-butylamine; R₁₂ is 1-yl-2-methoxyethyl; R₁₃ is 1-yl-2-(4(benzenesulfonamide)ethyl; R₁₄ is 1-yl-2-methoxyethyl; R₁₅ is isobutyl and R₁₆ is cyclohexyl;
(ee) Rg is 1-yl-2-methoxyethyl; R₁₀ is methylbenzyl; R₁₁ is 1-yl-n-butylamine; R₁₂ is 1-yl-n-butylamine; R₁₃ is piperonyl; R₁₄ is isobutyl; R₁₅ is 1-yl-2-(4(benzenesulfonamide)ethyl and R₁₆ is 1-yl-n-butylamine;
(ff) R₉ is 1-yl-n-butylamine; R₁₀ is methylbenzyl; R₁₁ is 1-yl-2-(4(benzenesulfonamide)ethyl; R₁₂ is methylbenzyl; R₁₃ is 1-yl-n-butylamine; R₁₄ is 1-yl-2-(4(benzenesulfonamide)ethyl; R₁₅ is 1-yl-2-(4(benzenesulfonamide)ethyl and R₁₆ is cyclohexyl;
(gg) R₉ is 1-yl-n-butylamine; R₁₀ is 1-yl-2-methoxyethyl; R₁₁ is 1-yl-n-butylamine; R₁₂ is 1-yl-2-(4(benzenesulfonamide)ethyl; R₁₃ is 1-yl-2-methoxyethyl; R₁₄ is 1-yl-2-methoxyethyl; R₁₅ is 1-yl-n-butylamine and R₁₆ is methylbenzyl;
(hh) Rg is cyclohexyl; R₁₀ is cyclohexyl; R₁₁ is methylbenzyl; R₁₂ is 1-yl-n-butylamine; R₁₃ is methylbenzyl; R₁₄ is cyclohexyl; R₁₅ is methylbenzyl and R₁₆ is 1-yl-n-butylamine;
(ii) Rg is 1-yl-n-butylamine; R₁₀ is furfuryl; R₁₁ is methylbenzyl; R₁₂ is 1-yl-2-(4(benzenesulfonamide)ethyl; R₁₃ is furfuryl; R₁₄ is cyclohexyl; R₁₅ is methylbenzyl and R₁₆ is cyclohexyl;
(jj) R₉ is 1-yl-n-butylamine; R₁₀ is methylbenzyl; R₁₁ is 1-yl-n-butylamine; R₁₂ is 1-yl-2-(4(benzenesulfonamide)ethyl; R₁₃ is 1-yl-2-methoxyethyl; R₁₄ is methylbenzyl; R₁₅ is 1-yl-2-methoxyethyl and R₁₆ is isobutyl;
(kk) Rg is 1-yl-2-(4(benzenesulfonamide)ethyl; R₁₀ is 1-yl-n-butylamine; R₁₁ is 1-yl-n-butylamine; R₁₂ is methylbenzyl; R₁₃ is methylbenzyl; R₁₄ is cyclohexyl; R₁₅ is 1-yl-2-(4(benzenesulfonamide)ethyl and R₁₆ is methylbenzyl;
(II) Rg is 1-yl-2-(4(benzenesulfonamide)ethyl; R₁₀ is 1-yl-2-(4(benzenesulfonamide)ethyl; R₁₁ is 1-yl-n-butylamine; R₁₂ is 1-yl-n-butylamine; R₁₃ is methylbenzyl; R₁₄ is 1-yl-n-butylamine; R₁₅ is methylbenzyl and R₁₆ is 1-yl-n-butylamine;
(mm) R₉ is 1-yl-n-butylamine; R₁₀ is 1-yl-2-(4(benzenesulfonamide)ethyl; R₁₁ is 1-yl-2-(4(benzenesulfonamide)ethyl; R₁₂ is 1-yl-2-methoxyethyl; R₁₃ is 1-yl-n-butylamine; R₁₄ is cyclohexyl; R₁₅ is 1-yl-2-(4(benzenesulfonamide)ethyl and R₁₆ is methylbenzyl;
(nn) Rg is 1-yl-n-butylamine; R₁₀ is 1-yl-n-butylamine; R₁₁ is piperonyl; R₁₂ is 1-yl-2-(4(benzenesulfonamide)ethyl; R₁₃ is 1-yl-n-butylamine; R₁₄ is methylbenzyl; R₁₅ is methylbenzyl and R₁₆ is cyclohexyl;
(oo) R₉ is 1-yl-2-(4(benzenesulfonamide)ethyl; R₁₀ is methylbenzyl; R₁₁ is methylbenzyl; R₁₂ is 1-yl-n-butylamine; R₁₃ is methylbenzyl; R₁₄ is piperonyl; R₁₅ is 1-yl-n-butylamine and R₁₆ is 1-yl-2-methoxyethyl;
(pp) Rg is piperonyl; R₁₀ is 1-yl-n-butylamine; R₁₁ is 1-yl-n-butylamine; R₁₂ is methylamine; R₁₃ is piperonyl; R₁₄ is 1-yl-n-butylamine; R₁₅ is piperonyl and R₁₆ is 1-yl-2-methoxyethyl;
(qq) Rg is cyclohexyl; R₁₀ is cyclohexyl; R₁₁ is furfuryl; R₁₂ is 1-yl-2-methoxyethyl; R₁₃ is isobutyl; R₁₄ is cyclohexyl; R₁₅ is methylbenzyl and R₁₆ is methylbenzyl;
(rr) Rg is piperonyl; R₁₀ is 1-yl-n-butylamine; R₁₁ is isobutyl; R₁₂ is isobutyl; R₁₃ is 1-yl-2-methoxyethyl; R₁₄ is cyclohexyl; R₁₅ is 1-yl-2-(4(benzenesulfonamide)ethyl and R₁₆ is 1-yl-2-(4(benzenesulfonamide)ethyl;
(ss) R₉ is cyclohexyl; R₁₀ is cyclohexyl; R₁₁ is 1-yl-n-butylamine; R₁₂ is methylbenzyl; R₁₃ is 1-yl-n-butylamine; R₁₄ is methylbenzyl; R₁₅ is cyclohexyl and R₁₆ is piperonyl;
and pharmaceutically acceptable salts thereof.

5. The method of claim 1, wherein said drug is at least one of the drugs listed in Table 1.

6. The method of claim 1, further comprising the step of determining whether said subject is responsive or non-responsive to a treatment or therapy by said drug.

7. The method of claim 1, wherein said drug induced response is associated with a disease, or a stage of said disease, wherein said disease is optionally a cancer, an autoimmune disease, an inflammatory disease, an infectious disease, a neurodegenerative disease, or a cardiovascular disease.

8. The method of claim 7, wherein the cancer disease is breast cancer, lung cancer, prostate cancer, cervical cancer, head and neck cancer, testicular cancer, ovarian cancer, skin cancer, brain cancer, pancreatic cancer, liver cancer, stomach cancer, colon cancer, rectal cancer, esophageal cancer, lymphoma, or leukemia,
or wherein the autoimmune disease is lupus, myestenia gravis, multiple sclerosis, narcolepsy, rheumatoid arthritis, nephritis, Chagas disease, scleroderma, or Sjogren's disease,
or wherein the infectious disease is a result of infection with viruses, bacteria or fungi,
or wherein the neurodegenerative disease is Alzheimer's disease, dementia, or Creutzfeld-Jacob disease.

9. The method of claim 1, wherein said ligand library comprises a plurality of ligands identified in an earlier initial screening or preselected based on known reactivity to said ligand binding markers.

10. The method of claim 1, wherein said ligand library is a bead-based ligand library comprising a plurality of beads having ligands bound to the beads.

11. The method of claim 1, wherein said screening is performed by an array, and wherein said array is optionally a microarray, a microscope slide, a plate, a chip, or a population of beads.

12. The method of claim 11, wherein said array comprises a solid substrate having a surface; a plurality of ligands bound to said substrate, each of said ligand comprising an anchor segment stably bound to the substrate surface, a peptoid segment, and a linker segment connecting and separating the anchor and peptoid segments, or wherein said array comprises between 1000 and 100,000 distinct ligands.

## Patentansprüche

1. Verfahren zum Diagnostizieren einer arzneistoffinduzierten Reaktion bei einem Subjekt, wobei das Verfahren Folgendes umfasst:
Screenen einer Ligandenbibliothek gegen eine biologische Probe,
die zuvor von dem Subjekt erhalten wurde; Identifizieren der Bindungseigenschaften eines oder mehrerer Marker in der Probe mit einem oder mehreren Liganden in der Bibliothek; und
Bestimmen, ob der eine oder die mehreren Marker mit der arzneistoffinduzierten Reaktion assoziiert sind,
wobei die Bibliothek eine Verbindung der Formel I umfasst, 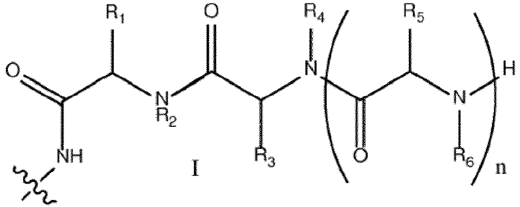 wobei
R₁-(C₁-C₆)SCH₃ ist
R₂ H ist; und
R₃-R₆ jeweils unabhängig aus den Gruppen bestehend aus H, - C₁-C₆Alkyl, -C₁-C₆AlkylSCH₃, -C₀-C₆Alkyl-C₂-C₆alkenyl, -C₀-C₆-Alkyl, C₂-C₆Alkinyl, -C₁-C₆COOH, -C₁-C₆AlkylOH, -C₁-C₆AlkylN(R)₂, -C₃-C₈Cycloalkyl, -C₁-C₆Alkylaryl, -C₁-C₆Alkylheteroaryl, -C₁-C₆AlkylNC(O)C₁-C₆Alkyl, -C₁-C₆Alkylcycloamid ausgewählt sind, wobei jegliche der Aryl- oder Heteroarylgruppen jeweils unabhängig mit -OH, Cl, F, Br, -OCH₃, SO₂NH₂ oder -O-CH₂-O-substituiert sein können,
wobei die arzneistoffinduzierte Reaktion eine unerwünschte Reaktion auf den Arzneistoff, eine Nebenwirkung des Arzneistoffs oder eine Resistenz gegen den Arzneistoff ist, wodurch die arzneistoffinduzierte Reaktion bei dem Subjekt diagnostiziert wird und
wobei n 3-11 ist.

2. Verfahren nach Anspruch 1, wobei die Bibliothek eine Verbindung der Formel I umfasst, 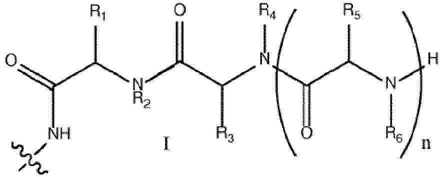 wobei die Verbindungen durch ein Verfahren hergestellt werden, welches die Verwendung eines Reaktanten umfasst, der aus der Gruppe bestehend aus Folgendem ausgewählt ist:
(A) Furfurylamin; 3,4-Dimethoxyethanolamin; Benzylamin; N-(2-Aminoethyl)acetamid; N-(3-Aminopropyl)-2-pyrrolidinon; Ethanolamin; Glycin; Diaminobutan; Allylamin; Piperonylamin; Methylbenzylamin; Isobutylamin; 4-(2-Aminoethyl)benzolsulfonamid oder Cyclohexylamin; oder
(B) Methoxyethylamin; Piperonylamin; Cyclohexylamin; Diaminobutan; Methylbenzylamin; Isobutylamin; Furfurylamin oder 4-(2-Aminoethyl)benzolsulfonamid; oder
(C) Furfurylamin, Ethanolamin; Glycin; Diaminobutan; Allylamin; Piperonylamin; Methylbenzylamin; Isobutylamin oder 4-(2-Aminoethyl)benzolsulfonamid; oder
(D) Furfurylamin, N-(2-Aminoethyl)acetamid; N-(3-Aminoethyl)-2-pyrrolidinon; Ethanolamin; Glycin; Diaminobutan; Allylamin; Piperonylamin; Methylbenzylamin; Isobutylamin; 4-(2-Aminoethyl)benzolsulfonamid; oder
(E) Cystein, Glycin, Allylamin, Ethanolamin, Isobutylamin, Methylbenzylamin, Piperonylamin, Methionin, Cyclohexylamin, 3,4-Dimethoxyphenethylamin, Benzylamin, N-(2-Aminoethyl)acetamid, N-(3-Aminopropyl)-2-pyrrolidon, 4-(2-Aminoethyl)benzolsulfonamid und Furfurylamin; und
wobei,
R₁ ist -(C₁-C₆)SCH₃ ist;
R₂ H ist;
R₃ und R₅ jeweils unabhängig aus den Gruppen bestehend aus H, -C₁-C₆Alkyl, -C₁-C₆AlkylSCH₃, -C₀-C₆Alkyl-C₂-C₆Alkenyl, -C₀-C₆-Alkyl, C₂-C₆Alkinyl, -C₁-C₆COOH, -C₁-C₆AlkylOH, -C₁-C₆AlkylN(R)₂, - C₃-C₈Cycloalkyl, -C₁-C₆Alkylaryl, -C₁-C₆Alkylheteroaryl, -C₁-C₆AlkylNC(O)C₁-C₆Alkyl, -C₁-C₆Alkylcycloamid ausgewählt sind, wobei jegliche der Aryl- oder Heteroarylgruppen jeweils unabhängig mit -OH, Cl, F, Br, OCH₃, SO₂NH₂ oder -O-CH₂-O-substituiert sein können;
R₄ aus der Gruppe bestehend aus Furfuryl oder -(C₁-C₆Alkyl)NR₇R₈ ausgewählt ist und
R₆ aus der Gruppe bestehend aus H, 1-Ylallyl, 1-Yl-2-hydroxyethyl, Isobutyl, 1-Yl-n-butylamin, Methylbenzyl, Piperonyl, Cyclohexyl, 1-Yl-2-(3,4-dimethoxyphenyl)ethyl, Benzyl, 1-Yl-2-(acetamid)ethyl, 1-Yl-3-2-pyrrolidinon, 1-Yl-2-(4-benzolsulfonamid)ethyl oder Furfuryl ausgewählt ist.

3. Verfahren nach Anspruch 1, wobei die Bibliothek eine Verbindung der Formel Ia umfasst 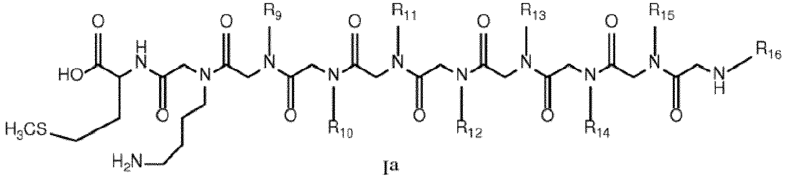 wobei die Verbindung aus der Gruppe bestehend aus einer Verbindung der Formel Ia ausgewählt ist, wobei,
(a) R₉ n-Butylamin; R₁₀ 1-Yl-n-butylamin; R₁₁ Piperonyl ist; R₁₂ Methylbenzyl ist; R₁₃ Piperonyl ist; R₁₄ Methylbenzyl ist; R₁₅ 1-Yl-2-(4(benzolsulfonamid)ethyl ist und R₁₆ 1-Yl-2-methoxyethyl ist;
(b) R₉ 1-Yl-n-butylamin ist; R₁₀ 1-Yl-2-(4(benzolsulfonamid)ethyl ist; R₁₁ 1-Yl-n-butylamin ist; R₁₂ Cyclohexyl ist; R₁₃ 1-Yl-2-methoxyethyl ist; R₁₄ 1-Yl-2,2-dimethylethyl(isobutyl) ist; R₁₅ 1-Yl-2-(4(benzolsulfonamid)ethyl ist und R₁₆ Methylbenzyl ist;
(c) R₉ 1-Yl-n-butylamin ist; R₁₀ 1-Yl-n-butylamin ist; R₁₁ Piperonyl ist; R₁₂ 1-Yl-2-(4(benzolsulfonamid)ethyl ist; R₁₃ 1-Yln-Butylamin ist; R₁₄ Methylbenzyl ist; R₁₅ Methylbenzyl ist und R₁₆ Cyclohexyl ist;
(d) R₉ Piperonyl ist; R₁₀ 1-Yl-n-butylamin ist; R₁₁ Isobutyl ist; R₁₂ 1-Yl-2-(4(benzolsulfonamid)ethyl ist; R₁₃ Methylbenzyl ist; R₁₄ Cyclohexyl ist; R₁₅ Isobutyl ist und R₁₆ 1-Yl-n-butylamin ist;
(e) R₉ 1-Yl-n-butylamin ist; R₁₀ 1-Yl-n-butylamin ist; R₁₁ Methylbenzyl ist; R₁₂ 1-Yl-2-methoxyethyl ist; R₁₃ Cyclohexyl ist; R₁₄ Cyclohexyl ist; R₁₅ Methylbenzyl ist und R₁₆ Piperonyl ist;
(f) R₉ Piperonyl ist; R₁₀ 1-Yl-n-butylamin ist; R₁₁ Isopropyl ist; R₁₂ Isopropyl ist; R₁₃ 1-Yl-2-methoxyethyl ist; R₁₄ Cyclohexyl ist; R₁₅ 1-Yl-2-(4(benzolsulfonamid)ethyl ist und R₁₆ 1-Yl-2-(4(benzolsulfonamid)ethyl ist;
(g) R₉ 1-Yl-n-butylamin ist; R₁₀ 1-Yl-n-butylamin ist; R₁₁ Piperonyl ist; R₁₂ Methylbenzyl ist; R₁₃ Piperonyl ist; R₁₄ Methylbenzyl ist; R₁₅ 1-Yl-2-(4(benzolsulfonamid)ethyl ist und R₁₆ Cyclohexyl ist;
(h) R₉ 1-Yl-n-butylamin ist; R₁₀ 1-Yl-n-butylamin ist; R₁₁ Methylbenzyl ist; R₁₂ 1-Yl-2-methoxyethyl ist; R₁₃ Cyclohexyl ist; R₁₄ Cyclohexyl ist; R₁₅ Methylbenzyl ist und R₁₆ Piperonyl ist;
(i) R₉ 1-Yl-n-butylamin ist; R₁₀ Methylbenzyl ist; R₁₁ Methylbenzyl ist; R₁₂ 1-Yl-n-butylamin ist; R₁₃ 1-Yl-n-butylamin ist; R₁₄ Cyclohexyl ist; R₁₅ 1-Yl-2-(4(benzolsulfonamid)ethyl ist und R₁₆ Cyclohexyl ist;
(j) R₉ 1-Yl-2-(4(benzolsulfonamid)ethyl ist; R₁₀ Methylbenzyl ist; R₁₁ Methylbenzyl ist; R₁₂ Cyclohexyl ist; R₁₃ 1-Yl-n-butylamin ist; R₁₄ Methylbenzyl ist; R₁₅ Isobutyl ist und R₁₆ 1-Yl-n-butylamin ist;
(k) R₉ 1-Yl-2-methoxyethyl ist; R₁₀ Isobutyl ist; R₁₁ 1-Yl-2-(4(benzolsulfonamid)ethyl ist; R₁₂ Piperonyl ist; R₁₃ 1-Yl-n-butylamin ist; R₁₄ Cyclohexyl ist; R₁₅ Methylbenzyl ist und R₁₆ 1-Yl-2-methoxyethyl ist;
(1) R₉ 1-Yl-n-butylamin ist; R₁₀ 1-Yl-2-(4(benzolsulfonamid)ethyl ist; R₁₁ 1-Yl-n-butylamin ist; R₁₂ Cyclohexyl ist; R₁₃ 1-Yl-2-methoxyethyl ist; R₁₄ Isobutyl ist; R₁₅ 1-Yl-2-(4(benzolsulfonamid)ethyl ist und R₁₆ Methylbenzyl ist;
(m) R₉ 1-Yl-2-methoxyethyl ist; R₁₀ 1-Yl-n-butylamin ist; R₁₁ 1-Yl-n-butylamin ist; R₁₂ 1-Yl-2-methoxyethyl ist; R₁₃ Methylbenzyl ist; R₁₄ 1-Yl-n-butylamin ist; R₁₅ Furfuryl ist und R₁₆ Furfuryl ist;
(n) R₉ Cyclohexyl ist; R₁₀ Cyclohexyl ist; R₁₁ 1-Yl-n-butylamin ist; R₁₂ Furfuryl ist; R₁₃ 1-Yl-2-methoxyethyl ist; R₁₄ 1-Yl-2-methoxyethyl ist; R₁₅ 1-Yl-2-(4(benzolsulfonamid)ethyl ist und R₁₆ Furfuryl ist;
(o) R₉ 1-Yl-n-butylamin ist; R₁₀ Piperonyl ist; R₁₁ 1-Yl-2-(4(benzolsulfonamid)ethyl ist; R₁₂ 1-Yl-2-methoxyethyl ist; R₁₃ Methylbenzyl ist; R₁₄ 1-Yl-n-butylamin ist; R₁₅ 1-Y1-2-methoxyethyl ist und R₁₆ Methylbenzyl ist;
(p) R₉ Cyclohexyl ist; R₁₀ Cyclohexyl ist; R₁₁ Piperonyl ist; R₁₂ 1-Yl-n-butylamin ist; R₁₃ 1-Yl-n-butylamin ist; R₁₄ 1-Yl-n-butylamin ist; R₁₅ 1-Yl-n-butylamin ist und R₁₆ Isobutyl ist;
(q) R₉ Piperonyl ist; R₁₀ 1-Yl-n-butylamin ist; R₁₁ 1-Yl-2-methoxyethyl ist; R₁₂ 1-Yl-2-(4(benzolsulfonamid)ethyl ist; R₁₃ Piperonyl ist; R₁₄ 1-Yl-n-butylamin ist; R₁₅ Methylbenzyl ist und R₁₆ Methylbenzyl ist;
(r) R₉ Methylbenzyl ist; R₁₀ Methylbenzyl ist; R₁₁ Methylbenzyl ist; R₁₂ 1-Yl-n-butylamin ist; R₁₃ Piperonyl ist; R₁₄ 1-Yl-n-butylamin ist; R₁₅ Piperonyl ist und R₁₆ 1-Yl-N-butylamin ist;
(s) R₉ 1-Yl-n-butylamin ist; R₁₀ 1-Yl-n-butylamin ist; R₁₁ Methylbenzyl ist; R₁₂ 1-Yl-butylamin ist; R₁₃ Methylbenzyl ist; R₁₄ Methylbenzyl ist; R₁₅ 1-Yl-2-methoxyethyl ist und R₁₆ Piperonyl ist;
(t) R₉ Methylbenzyl ist; R₁₀ 1-Yl-2-(4(benzolsulfonamid)ethyl ist; R₁₁ 1-Yl-n-butylamin ist; R₁₂ 1-Yl-2-(4(benzolsulfonamid)ethyl ist; R₁₃ Isobutyl ist; R₁₄ 1-Yl-n-butylamin ist; R₁₅ Methylbenzyl ist und R₁₆ 1-Yl-2-methoxyethyl ist;
(u) R₉ 1-Yl-2-methoxyethyl ist; R₁₀ 1-Yl-n-butylamin ist; R₁₁ Isobutyl ist; R₁₂ Isobutyl ist; R₁₃ Cyclohexyl ist; R₁₄ 1-Yl-n-butylamin ist; R₁₅ 1-Yl-2-(4(benzolsulfonamid)ethyl ist und R₁₆ Cyclohexyl ist;
(v) R₉ Isobutyl ist; R₁₀ 1-Yl-n-butylamin ist; R₁₁ 1-Yl-n-butylamin ist; R₁₂ Methylbenzyl ist; R₁₃ 1-Yl-n-butylamin ist; R₁₄ 1-Yl-n-butylamin ist; R₁₅ Piperonyl ist und R₁₆ Piperonyl ist;
(w) R₉ 1-Yl-2-(4(benzolsulfonamid)ethyl ist; R₁₀ Isobutyl ist; R₁₁ Methylbenzyl ist; R₁₂ 1-Yl-2-methoxyethyl ist; R₁₃ 1-Yl-2-(4(benzolsulfonamid)ethyl ist; R₁₄ Isobutyl ist; R₁₅ 1-Yl-methoxyethyl ist und R₁₆ Cyclohexyl ist;
(x) R₉ Furfuryl ist; R₁₀ Furfuryl ist; R₁₁ Piperonyl ist; R₁₂ Cyclohexyl ist; R₁₃ Piperonyl ist; R₁₄ 1-Yl-n-butylamin ist; R₁₅ 1-Yl-2-(4(benzolsulfonamid)ethyl ist und R₁₆ Cyclohexyl ist;
(y) R₉ Piperonyl ist; R₁₀ Piperonyl ist; R₁₁ 1-Yl-2-methoxyethyl ist; R₁₂ 1-Yl-2-methoxyethyl ist; R₁₃ 1-Yl-n-butylamin ist; R₁₄ 1-Yl-n-butylamin ist; R₁₅ 1-Yl-n-butylamin ist und R₁₆ 1-Yl-2-methoxyethyl ist;
(z) R₉ Piperonyl ist; R₁₀ 1-Yl-n-butylamin ist; R₁₁ Isobutyl ist; R₁₂ 1-Yl-2-(4(benzolsulfonamid)ethyl ist; R₁₃ Methylbenzyl ist; R₁₄ Cyclohexyl ist; R₁₅ Isobutyl ist und R₁₆ 1-Yl-n-butylamin ist;
(aa) R₉ 1-Yl-2-(4(benzolsulfonamid)ethyl ist; R₁₀ 1-Yl-2-(4(benzolsulfonamid)ethyl ist; R₁₁ Methylbenzyl ist; R₁₂ Methylbenzyl ist; R₁₃ 1-Yl-n-butylamin ist; R₁₄ 1-Yl-n-butylamin ist; R₁₅ 1-Yl-2-(4(benzolsulfonamid)ethyl ist und R₁₆ 1-Yl-2-(4(benzolsulfonamid)ethyl ist;
(bb) R₉ 1-Yl-n-butylamin ist; R₁₀ 1-Yl-2-methoxyethyl ist; R₁₁ 1-Yl-n-butylamin ist; R₁₂ Isobutyl ist; R₁₃ Cyclohexyl ist; R₁₄ 1-Yl-n-butylamin ist; R₁₅ 1-Yl-n-butylamin ist und R₁₆ Piperonyl ist;
(cc) R₉ Cyclohexyl ist; R₁₀ Methylbenzyl ist; R₁₁ Cyclohexyl ist; R₁₂ Piperonyl ist; R₁₃ 1-Yl-n-butylamin ist; R₁₄ 1-Yl-2-(4(benzolsulfonamid)ethyl ist; R₁₅ 1-Yl-n-butylamin ist und R₁₆ 1-Yl-2-methoxyethyl ist;
(dd) R₉ 1-Yl-2-methoxyethyl ist; R₁₀ 1-Yl-2-(4(benzolsulfonamid)ethyl ist; R₁₁ 1-Yl-n-butylamin ist; R₁₂ 1-Yl-2-methoxyethyl ist; R₁₃ 1-Yl-2-(4(benzolsulfonamid)ethyl ist; R₁₄ 1-Yl-2-methoxyethyl ist; R₁₅ Isobutyl ist und R₁₆ Cyclohexyl ist;
(ee) R₉ 1-Yl-2-methoxyethyl ist; R₁₀ Methylbenzyl ist; R₁₁ 1-Yl-n-butylamin ist; R₁₂ 1-Yl-n-butylamin ist; R₁₃ Piperonyl ist; R₁₄ Isobutyl ist; R₁₅ 1-Yl-2-(4(benzolsulfonamid)ethyl ist und R₁₆ 1-Yl-n-butylamin ist;
(ff) R₉ 1-Yl-n-butylamin ist; R₁₀ Methylbenzyl ist; R₁₁ 1-Yl-2-(4(benzolsulfonamid)ethyl ist; R₁₂ Methylbenzyl ist; R₁₃ 1-Yl-n-butylamin ist; R₁₄ 1-Yl-2-(4(benzolsulfonamid)ethyl ist; R₁₅ 1-Yl-2-(4(benzolsulfonamid)ethyl ist und R₁₆ Cyclohexyl ist;
(gg) R₉ 1-Yl-n-butylamin ist; R₁₀ 1-Yl-2-methoxyethyl ist; R₁₁ 1-Yl-n-butylamin ist; R₁₂ 1-Yl-2-(4(benzolsulfonamid)ethyl ist; R₁₃ 1-Yl-2-methoxyethyl ist; R₁₄ 1-Yl-2-methoxyethyl ist; R₁₅ 1-Yl-n-butylamin ist und R₁₆ Methylbenzyl ist;
(hh) R₉ Cyclohexyl ist; R₁₀ Cyclohexyl ist; R₁₁ Methylbenzyl ist; R₁₂ 1-Yl-n-butylamin ist; R₁₃ Methylbenzyl ist; R₁₄ Cyclohexyl ist; R₁₅ Methylbenzyl ist und R₁₆ 1-Yl-n-butylamin ist;
(ii) R₉ 1-Yl-n-butylamin ist; R₁₀ Furfuryl ist; R₁₁ Methylbenzyl ist; R₁₂ 1-Yl-2-(4(benzolsulfonamid)ethyl ist; R₁₃ Furfuryl ist; R₁₄ Cyclohexyl ist; R₁₅ Methylbenzyl ist und R₁₆ Cyclohexyl ist;
(jj) R₉ 1-Yl-n-butylamin ist; R₁₀ Methylbenzyl ist; R₁₁ 1-Yln-butylamin ist; R₁₂ 1-Yl-2-(4(benzolsulfonamid)ethyl ist; R₁₃ 1-Yl-2-methoxyethyl ist; R₁₄ Methylbenzyl ist; R₁₅ 1-Y1-2-methoxyethyl ist und R₁₆ Isobutyl ist;
(kk) R₉ 1-Yl-2-(4(benzolsulfonamid)ethyl ist; R₁₀ 1-Yl-n-butylamin ist; R₁₁ 1-Yl-n-butylamin ist; R₁₂ Methylbenzyl ist; R₁₃ Methylbenzyl ist; R₁₄ Cyclohexyl ist; R₁₅ 1-Yl-2-(4(benzolsulfonamid)ethyl ist und R₁₆ Methylbenzyl ist;
(11) R₉ 1-Yl-2-(4(benzolsulfonamid)ethyl ist; R₁₀ 1-Yl-2-(4(benzolsulfonamid)ethyl ist; R₁₁ 1-Yl-n-butylamin ist; R₁₂ 1-Yl-n-butylamin ist; R₁₃ Methylbenzyl ist; R₁₄ 1-Yl-n-butylamin ist; R₁₅ Methylbenzyl ist und R₁₆ 1-Yl-n-butylamin ist;
(mm) R₉ 1-Yl-n-butylamin ist; R₁₀ 1-Yl-2-(4(benzolsulfonamid)ethyl ist; R₁₁ 1-Yl-2-(4(benzolsulfonamid)ethyl ist; R₁₂ 1-Yl-2-methoxyethyl ist; R₁₃ 1-Yl-n-butylamin ist; R₁₄ Cyclohexyl ist; R₁₅ 1-Yl-2-(4(benzolsulfonamid)ethyl ist und R₁₆ Methylbenzyl ist;
(nn) R₉ 1-Yl-n-butylamin ist; R₁₀ 1-Yl-n-butylamin ist; R₁₁ Piperonyl ist; R₁₂ 1-Yl-2-(4(benzolsulfonamid)ethyl ist; R₁₃ 1-Yln-butylamin ist; R₁₄ Methylbenzyl ist; R₁₅ Methylbenzyl ist und R₁₆ Cyclohexyl ist;
(oo) R₉ 1-Yl-2-(4(benzolsulfonamid)ethyl ist; R₁₀ Methylbenzyl ist; R₁₁ Methylbenzyl ist; R₁₂ 1-Yl-n-butylamin ist; R₁₃ Methylbenzyl ist; R₁₄ Piperonyl ist; R₁₅ 1-Yl-n-butylamin ist und R₁₆ 1-Yl-2-methoxyethyl ist;
(pp) R₉ Piperonyl ist; R₁₀ 1-Yl-n-butylamin ist; R₁₁ 1-Yl-n-butylamin ist; R₁₂ Methylamin ist; R₁₃ Piperonyl ist; R₁₄ 1-Yl-n-butylamin ist; R₁₅ Piperonyl ist und R₁₆ 1-Yl-2-methoxyethyl ist;
(qq) R₉ Cyclohexyl ist; R₁₀ Cyclohexyl ist; R₁₁ Furfuryl ist; R₁₂ 1-Yl-2-methoxyethyl ist; R₁₃ Isobutyl ist; R₁₄ Cyclohexyl ist; R₁₅ Methylbenzyl ist und R₁₆ Methylbenzyl ist;
(rr) R₉ Piperonyl ist; R₁₀ 1-Yl-n-butylamin ist; R₁₁ Isobutyl ist; R₁₂ Isobutyl ist; R₁₃ 1-Yl-2-methoxyethyl ist; R₁₄ Cyclohexyl ist; R₁₅ 1-Yl-2-(4(benzolsulfonamid)ethyl ist und R₁₆ 1-Yl-2-(4(benzolsulfonamid)ethyl ist;
(ss) R₉ Cyclohexyl ist; R₁₀ Cyclohexyl ist; R₁₁ 1-Yl-n-butylamin ist; R₁₂ Methylbenzyl ist; R₁₃ 1-Yl-n-butylamin ist; R₁₄ Methylbenzyl ist; R₁₅ Cyclohexyl ist und R₁₆ Piperonyl ist;
und pharmazeutisch annehmbare Salze davon.

4. Verfahren nach Anspruch 1, wobei die Bibliothek eine Verbindung der folgenden Formel umfasst: 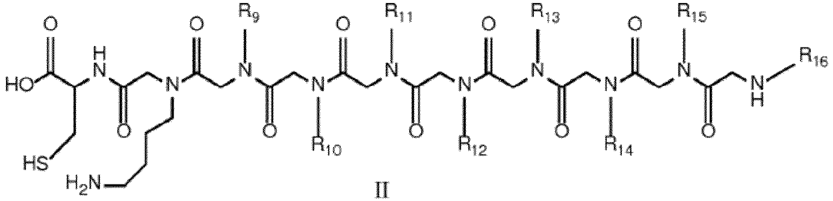 wobei die Verbindung aus der Gruppe bestehend aus einer Verbindung der Formel II ausgewählt ist, wobei
(a) R₉ n-Butylamin ist; R₁₀ 1-Yl-n-butylamin ist; R₁₁ Piperonyl ist; R₁₂ Methylbenzyl ist; R₁₃ Piperonyl ist; R₁₄ Methylbenzyl ist; R₁₅ 1-Yl-2-(4(benzolsulfonamid)ethyl ist und R₁₆ 1-Yl-2-methoxyethyl ist;
(b) R₉ 1-Yl-n-butylamin ist; R₁₀ 1-Yl-2-(4(benzolsulfonamid)ethyl ist; R₁₁ 1-Yl-n-butylamin ist; R₁₂ Cyclohexyl ist; R₁₃ 1-Yl-2-methoxyethyl ist; R₁₄ 1-Yl-2,2-Dimethylethyl(isobutyl) ist; R₁₅ 1-Yl-2-(4(benzolsulfonamid)ethyl ist und R₁₆ Methylbenzyl ist;
(c) R₉ 1-Yl-n-butylamin ist; R₁₀ 1-Yl-n-butylamin ist; R₁₁ Piperonyl ist; R₁₂ 1-Yl-2-(4(benzolsulfonamid)ethyl ist; R₁₃ 1-Yln-butylamin ist; R₁₄ Methylbenzyl ist; R₁₅ Methylbenzyl ist und R₁₆ Cyclohexyl ist;
(d) R₉ Piperonyl ist; R₁₀ 1-Yl-n-butylamin ist; R₁₁ Isobutyl ist; R₁₂ 1-Yl-2-(4(benzolsulfonamid)ethyl ist; R₁₃ Methylbenzyl ist; R₁₄ Cyclohexyl ist; R₁₅ Isobutyl ist und R₁₆ 1-Yl-n-Butylamin ist;
(e) R₉ 1-Yl-n-butylamin ist; R₁₀ 1-Yl-n-butylamin ist; R₁₁ Methylbenzyl ist; R₁₂ 1-Yl-2-methoxyethyl ist; R₁₃ Cyclohexyl ist; R₁₄ Cyclohexyl ist; R₁₅ Methylbenzyl ist und R₁₆ Piperonyl ist;
(f) R₉ Piperonyl ist; R₁₀ 1-Yl-n-butylamin ist; R₁₁ Isopropyl ist; R₁₂ Isopropyl ist; R₁₃ 1-Yl-2-methoxyethyl ist; R₁₄ Cyclohexyl ist; R₁₅ 1-Yl-2-(4(benzolsulfonamid)ethyl ist und R₁₆ 1-Yl-2-(4(benzolsulfonamid)ethyl ist;
(g) R₉ 1-Yl-n-butylamin ist; R₁₀ 1-Yl-n-butylamin ist; R₁₁ Piperonyl ist; R₁₂ Methylbenzyl ist; R₁₃ Piperonyl ist; R₁₄ Methylbenzyl ist; R₁₅ 1-Yl-2-(4(benzolsulfonamid)ethyl ist und R₁₆ Cyclohexyl ist;
(h) R₉ 1-Yl-n-butylamin ist; R₁₀ 1-Yl-n-butylamin ist; R₁₁ Methylbenzyl ist; R₁₂ 1-Yl-2-methoxyethyl ist; R₁₃ Cyclohexyl ist; R₁₄ Cyclohexyl ist; R₁₅ Methylbenzyl ist und R₁₆ Piperonyl ist;
(i) R₉ 1-Yl-n-butylamin ist; R₁₀ Methylbenzyl ist; R₁₁ Methylbenzyl ist; R₁₂ 1-Yl-n-butylamin ist; R₁₃ 1-Yl-n-butylamin ist; R₁₄ Cyclohexyl ist; R₁₅ 1-Yl-2-(4(benzolsulfonamid)ethyl ist und R₁₆ Cyclohexyl ist;
(j) R₉ 1-Yl-2-(4(benzolsulfonamid)ethyl ist; R₁₀ Methylbenzyl ist; R₁₁ Methylbenzyl ist; R₁₂ Cyclohexyl ist; R₁₃ 1-Yl-n-butylamin ist; R₁₄ Methylbenzyl ist; R₁₅ Isobutyl ist und R₁₆ 1-Yl-n-butylamin ist;
(k) R₉ 1-Yl-2-methoxyethyl ist; R₁₀ Isobutyl ist; R₁₁ 1-Yl-2-(4(benzolsulfonamid)ethyl ist; R₁₂ Piperonyl ist; R₁₃ 1-Yl-n-butylamin ist; R₁₄ Cyclohexyl ist; R₁₅ Methylbenzyl ist und R₁₆ 1-Yl-2-methoxyethyl ist;
(1) R₉ 1-Yl-n-butylamin ist; R₁₀ 1-Yl-2-(4(benzolsulfonamid)ethyl ist; R₁₁ 1-Yl-n-butylamin ist; R₁₂ Cyclohexyl ist; R₁₃ 1-Yl-2-methoxyethyl ist; R₁₄ Isobutyl ist; R₁₅ 1-Yl-2-(4(benzolsulfonamid)ethyl ist und R₁₆ Methylbenzyl ist;
(m) R₉ 1-Yl-2-methoxyethyl ist; R₁₀ 1-Yl-n-butylamin ist; R₁₁ 1-Yl-n-butylamin ist; R₁₂ ist 1-Yl-2-methoxyethyl ist; R₁₃ Methylbenzyl ist; R₁₄ 1-Yl-n-butylamin ist; R₁₅ Furfuryl ist und R₁₆ Furfuryl ist;
(n) R₉ Cyclohexyl ist; R₁₀ Cyclohexyl ist; R₁₁ 1-Yl-n-butylamin ist; R₁₂ Furfuryl ist; R₁₃ 1-Yl-2-methoxyethyl ist; R₁₄ 1-Yl-2-methoxyethyl ist; R₁₅ 1-Yl-2-(4(benzolsulfonamid)ethyl ist und R₁₆ Furfuryl ist;
(o) R₉ 1-Yl-n-butylamin ist; R₁₀ Piperonyl ist; R₁₁ 1-Yl-2-(4(benzolsulfonamid)ethyl ist; R₁₂ 1-Yl-2-methoxyethyl ist; R₁₃ Methylbenzyl ist; R₁₄ 1-Yl-n-butylamin ist; R₁₅ 1-Y1-2-methoxyethyl ist und R₁₆ Methylbenzyl ist;
(p) R₉ Cyclohexyl ist; R₁₀ Cyclohexyl ist; R₁₁ Piperonyl ist; R₁₂ 1-Yl-n-butylamin ist; R₁₃ 1-Yl-n-butylamin ist; R₁₄ 1-Yl-n-butylamin ist; R₁₅ 1-Yl-n-butylamin ist und R₁₆ Isobutyl ist;
(q) R₉ Piperonyl ist; R₁₀ 1-Yl-n-butylamin ist; R₁₁ 1-Yl-2-methoxyethyl ist; R₁₂ 1-Yl-2-(4(benzolsulfonamid)ethyl ist; R₁₃ Piperonyl ist; R₁₄ 1-Yl-n-butylamin ist; R₁₅ Methylbenzyl ist und R₁₆ Methylbenzyl ist;
(r) R₉ Methylbenzyl ist; R₁₀ Methylbenzyl ist; R₁₁ Methylbenzyl ist; R₁₂ 1-Yl-n-butylamin ist; R₁₃ Piperonyl ist; R₁₄ 1-Yl-n-butylamin ist; R₁₅ Piperonyl ist und R₁₆ 1-Yl-N-butylamin ist;
(s) R₉ 1-Yl-n-butylamin ist; R₁₀ 1-Yl-n-butylamin ist; R₁₁ Methylbenzyl ist; R₁₂ 1-Yl-butylamin ist; R₁₃ Methylbenzyl ist; R₁₄ Methylbenzyl ist; R₁₅ 1-Yl-2-methoxyethyl ist und R₁₆ Piperonyl ist;
(t) R₉ Methylbenzyl ist; R₁₀ 1-Yl-2-(4(benzolsulfonamid)ethyl ist; R₁₁ 1-Yl-n-butylamin ist; R₁₂ 1-Yl-2-(4(benzolsulfonamid)ethyl ist; R₁₃ Isobutyl ist; R₁₄ 1-Yl-n-butylamin ist; R₁₅ Methylbenzyl ist und R₁₆ 1-Y1-2-methoxyethyl ist;
(u) R₉ 1-Y1-2-methoxyethyl ist; R₁₀ 1-Yl-n-butylamin ist; R₁₁ Isobutyl ist; R₁₂ Isobutyl ist; R₁₃ Cyclohexyl ist; R₁₄ 1-Yl-n-butylamin ist; R₁₅ 1-Y1-2-(4(benzolsulfonamid)ethyl ist und R₁₆ Cyclohexyl ist;
(v) R₉ Isobutyl ist; R₁₀ 1-Yl-n-butylamin ist; R₁₁ 1-Yl-n-butylamin ist; R₁₂ Methylbenzyl ist; R₁₃ 1-Yl-n-butylamin ist; R₁₄ 1-Yl-n-butylamin ist; R₁₅ Piperonyl ist und R₁₆ Piperonyl ist;
(w) R₉ 1-Y1-2-(4(benzolsulfonamid)ethyl ist; R₁₀ Isobutyl ist; R₁₁ Methylbenzyl ist; R₁₂ 1-Y1-2-methoxyethyl ist; R₁₃ 1-Yl-2-(4(benzolsulfonamid)ethyl ist; R₁₄ Isobutyl ist; R₁₅ 1-Yl-2-methoxyethyl ist und R₁₆ Cyclohexyl ist;
(x) R₉ Furfuryl ist; R₁₀ Furfuryl ist; R₁₁ Piperonyl ist; R₁₂ Cyclohexyl ist; R₁₃ Piperonyl ist; R₁₄ 1-Yl-n-butylamin ist; R₁₅ 1-Y1-2-(4(benzolsulfonamid)ethyl ist und R₁₆ Cyclohexyl ist;
(y) R₉ Piperonyl ist; R₁₀ Piperonyl ist; R₁₁ 1-Yl-2-methoxyethyl ist; R₁₂ 1-Y1-2-methoxyethyl ist; R₁₃ 1-Yl-n-butylamin ist; R₁₄ 1-Yl-n-butylamin ist; R₁₅ 1-Yl-n-butylamin ist und R₁₆ 1-Y1-2-methoxyethyl ist;
(z) R₉ Piperonyl ist; R₁₀ 1-Yl-n-butylamin ist; R₁₁ Isobutyl ist; R₁₂ 1-Y1-2-(4(benzolsulfonamid)ethyl ist; R₁₃ Methylbenzyl ist; R₁₄ Cyclohexyl ist; R₁₅ Isobutyl ist und R₁₆ 1-Yl-n-butylamin ist;
(aa) R₉ 1-Y1-2-(4(benzolsulfonamid)ethyl ist; R₁₀ 1-Yl-2-(4(benzolsulfonamid)ethyl ist; R₁₁ Methylbenzyl ist; R₁₂ Methylbenzyl ist; R₁₃ 1-Yl-n-butylamin ist; R₁₄ 1-Yl-n-butylamin ist; R₁₅ 1-Y1-2-(4(benzolsulfonamid)ethyl und R₁₆ 1-Yl-2-(4(benzolsulfonamid)ethyl ist;
(bb) R₉ 1-Yl-n-butylamin ist; R₁₀ 1-Y1-2-methoxyethyl ist; R₁₁ 1-Yl-n-butylamin ist; R₁₂ Isobutyl ist; R₁₃ Cyclohexyl ist; R₁₄ 1-Yl-n-butylamin ist; R₁₅ 1-Yl-n-butylamin ist und R₁₆ Piperonyl ist;
(cc) R₉ Cyclohexyl ist; R₁₀ Methylbenzyl ist; R₁₁ Cyclohexyl ist; R₁₂ Piperonyl ist; R₁₃ 1-Yl-n-butylamin ist; R₁₄ 1-Yl-2-(4(benzolsulfonamid)ethyl ist; R₁₅ 1-Yl-n-butylamin ist und R₁₆ 1-Yl-2-methoxyethyl ist;
(dd) R₉ 1-Y1-2-methoxyethyl ist; R₁₀ 1-Yl-2-(4(benzolsulfonamid)ethyl ist; R₁₁ 1-Yl-n-butylamin ist; R₁₂ 1-Yl-2-methoxyethyl ist; R₁₃ 1-Y1-2-(4(benzolsulfonamid)ethyl ist; R₁₄ 1-Y1-2-methoxyethyl ist; R₁₅ Isobutyl ist und R₁₆ Cyclohexyl ist;
(ee) R₉ 1-Y1-2-methoxyethyl ist; R₁₀ Methylbenzyl ist; R₁₁ 1-Yl-n-butylamin ist; R₁₂ 1-Yl-n-butylamin ist; R₁₃ Piperonyl ist; R₁₄ Isobutyl ist; R₁₅ 1-Yl-2-(4(benzolsulfonamid)ethyl ist und R₁₆ 1-Yl-n-butylamin ist;
(ff) R₉ 1-Yl-n-butylamin ist; R₁₀ Methylbenzyl ist; R₁₁ 1-Yl-2-(4(benzolsulfonamid)ethyl ist; R₁₂ Methylbenzyl ist; R₁₃ 1-Yl-n-butylamin ist; R₁₄ 1-Y1-2-(4(benzolsulfonamid)ethyl ist; R₁₅ 1-Yl-2-(4(benzolsulfonamid)ethyl ist und R₁₆ Cyclohexyl ist;
(gg) R₉ 1-Yl-n-butylamin ist; R₁₀ 1-Y1-2-methoxyethyl ist; R₁₁ 1-Yl-n-butylamin ist; R₁₂ 1-Y1-2-(4(benzolsulfonamid)ethyl ist; R₁₃ 1-Y1-2-methoxyethyl ist; R₁₄ 1-Y1-2-methoxyethyl ist; R₁₅ 1-Yl-n-butylamin ist und R₁₆ Methylbenzyl ist;
(hh) R₉ Cyclohexyl ist; R₁₀ Cyclohexyl ist; R₁₁ Methylbenzyl ist; R₁₂ 1-Yl-n-butylamin ist; R₁₃ Methylbenzyl ist; R₁₄ Cyclohexyl ist; R₁₅ Methylbenzyl ist und R₁₆ 1-Yl-n-butylamin ist;
(ii) R₉ 1-Yl-n-butylamin ist; R₁₀ Furfuryl ist; R₁₁ Methylbenzyl ist; R₁₂ 1-Y1-2-(4(benzolsulfonamid)ethyl ist; R₁₃ Furfuryl ist; R₁₄ Cyclohexyl ist; R₁₅ Methylbenzyl ist und R₁₆ Cyclohexyl ist;
(jj) R₉ 1-Yl-n-butylamin ist; R₁₀ Methylbenzyl ist; R₁₁ 1-Yln-butylamin ist; R₁₂ 1-Y1-2-(4(benzolsulfonamid)ethyl ist; R₁₃ 1-Yl-2-methoxyethyl ist; R₁₄ Methylbenzyl ist; R₁₅ 1-Yl-2-methoxyethyl ist und R₁₆ Isobutyl ist;
(kk) R₉ 1-Y1-2-(4(benzolsulfonamid)ethyl ist; R₁₀ 1-Yl-n-butylamin ist; R₁₁ 1-Yl-n-butylamin ist; R₁₂ Methylbenzyl ist; R₁₃ Methylbenzyl ist; R₁₄ Cyclohexyl ist; R₁₅ 1-Yl-2-(4(benzolsulfonamid)ethyl ist und R₁₆ Methylbenzyl ist;
(11) R₉ 1-Y1-2-(4(benzolsulfonamid)ethyl ist; R₁₀ 1-Yl-2-(4(benzolsulfonamid)ethyl ist; R₁₁ 1-Yl-n-butylamin ist; R₁₂ 1-Yln-butylamin ist; R₁₃ Methylbenzyl ist; R₁₄ 1-Yl-n-butylamin ist; R₁₅ Methylbenzyl ist und R₁₆ 1-Yl-n-butylamin ist;
(mm) R₉ 1-Yl-n-butylamin ist; R₁₀ 1-Yl-2-(4(benzolsulfonamid)ethyl ist; R₁₁ 1-Yl-2-(4(benzolsulfonamid)ethyl ist; R₁₂ 1-Y1-2-methoxyethyl ist; R₁₃ 1-Yl-n-butylamin ist; R₁₄ Cyclohexyl ist; R₁₅ 1-Yl-2-(4(benzolsulfonamid)ethyl ist und R₁₆ Methylbenzyl ist;
(nn) R₉ 1-Yl-n-butylamin ist; R₁₀ 1-Yl-n-butylamin ist; R₁₁ Piperonyl ist; R₁₂ 1-Y1-2-(4(benzolsulfonamid)ethyl ist; R₁₃ 1-Yln-butylamin ist; R₁₄ Methylbenzyl ist; R₁₅ Methylbenzyl ist und R₁₆ Cyclohexyl ist;
(oo) R₉ 1-Y1-2-(4(benzolsulfonamid)ethyl ist; R₁₀ Methylbenzyl ist; R₁₁ Methylbenzyl ist; R₁₂ 1-Yl-n-butylamin ist; R₁₃ Methylbenzyl ist; R₁₄ Piperonyl ist; R₁₅ 1-Yl-n-butylamin ist und R₁₆ 1-Y1-2-methoxyethyl ist;
(pp) R₉ Piperonyl ist; R₁₀ 1-Yl-n-butylamin ist; R₁₁ 1-Yl-n-butylamin ist; R₁₂ Methylamin ist; R₁₃ Piperonyl ist; R₁₄ 1-Yl-n-butylamin ist; R₁₅ Piperonyl ist und R₁₆ 1-Y1-2-methoxyethyl ist;
(qq) R₉ Cyclohexyl ist; R₁₀ Cyclohexyl ist; R₁₁ Furfuryl ist; R₁₂ 1-Y1-2-methoxyethyl ist; R₁₃ Isobutyl ist; R₁₄ Cyclohexyl ist; R₁₅ Methylbenzyl ist und R₁₆ Methylbenzyl ist;
(rr) R₉ Piperonyl ist; R₁₀ 1-Yl-n-butylamin ist; R₁₁ Isobutyl ist; R₁₂ Isobutyl ist; R₁₃ 1-Y1-2-methoxyethyl ist; R₁₄ Cyclohexyl ist; R₁₅ 1-Y1-2-(4(benzolsulfonamid)ethyl ist und R₁₆ 1-Yl-2-(4(benzolsulfonamid)ethyl ist;
(ss) R₉ Cyclohexyl ist; R₁₀ Cyclohexyl ist; R₁₁ 1-Yl-n-butylamin ist; R₁₂ Methylbenzyl ist; R₁₃ 1-Yl-n-butylamin ist; R₁₄ Methylbenzyl ist; R₁₅ Cyclohexyl ist und R₁₆ Piperonyl ist;
und pharmazeutisch annehmbare Salze davon.

5. Verfahren nach Anspruch 1, wobei der Arzneistoff mindestens einer der in Tabelle 1 aufgeführten Arzneistoffe ist.

6. Verfahren nach Anspruch 1, ferner umfassend den Schritt des Bestimmens, ob das Subjekt auf eine Behandlung oder Therapie mit dem Arzneistoff anspricht oder nicht Anspricht.

7. Verfahren nach Anspruch 1, wobei die arzneistoffinduzierte Reaktion mit einer Erkrankung oder einem Stadium der Erkrankung assoziiert ist, wobei die Erkrankung gegebenenfalls eine Krebserkrankung, eine Autoimmunerkrankung, eine entzündliche Erkrankung, eine Infektionserkrankung, eine neurodegenerative Erkrankung oder eine kardiovaskuläre Erkrankung ist.

8. Verfahren nach Anspruch 7, wobei die Krebserkrankung Brustkrebs, Lungenkrebs, Prostatakrebs, Gebärmutterhalskrebs, Kopf-Hals-Krebs, Hodenkrebs, Eierstockkrebs, Hautkrebs, Hirnkrebs, Bauchspeicheldrüsenkrebs, Leberkrebs, Magenkrebs, Darmkrebs, Enddarmkrebs, Speiseröhrenkrebs, ein Lymphom oder Leukämie ist,
oder wobei die Autoimmunerkrankung Lupus, Myestenia gravis, Multiple Sklerose, Narkolepsie, Rheumatoide Arthritis, Nephritis, Chagas-Krankheit, Sklerodermie oder Sjögren-Krankheit ist,
oder wobei die Infektionserkrankung ein Ergebnis einer Infektion mit Viren, Bakterien oder Pilzen ist,
oder wobei die neurodegenerative Erkrankung die Alzheimer-Krankheit, Demenz oder Creutzfeld-Jakob-Erkrankung ist.

9. Verfahren nach Anspruch 1, wobei die Ligandenbibliothek eine Vielzahl von Liganden umfasst, die in einem früheren anfänglichen Screening identifiziert oder basierend auf einer vorbekannten Reaktivität mit den Ligandenbindungsmarkern vorausgewählt wurden.

10. Verfahren nach Anspruch 1, wobei die Ligandenbibliothek eine perlenbasierte Ligandenbibliothek ist, die eine Vielzahl von Perlen mit Liganden, die an die Perlen gebunden sind, umfasst.

11. Verfahren nach Anspruch 1, wobei das Screening durch ein Array durchgeführt wird, und wobei das Array gegebenenfalls ein Mikroarray, ein Mikroskopobjektträger, eine Platte, ein Chip oder eine Population von Perlen ist.

12. Verfahren nach Anspruch 11, wobei das Array ein festes Substrat mit einer Oberfläche umfasst; wobei eine Vielzahl von Liganden an das Substrat gebunden sind, wobei jeder der Liganden ein Ankersegment, das stabil an die Substratoberfläche gebunden ist, ein Peptoidsegment und ein Linkersegment, das die Anker- und Peptoidsegmente verbindet und trennt, umfasst oder wobei das Array zwischen 1000 und 100000 verschiedene Liganden umfasst.

## Revendications

1. Procédé pour diagnostiquer une réponse induite par un médicament chez un sujet, le procédé comprenant: le criblage d'une banque de ligands contre un échantillon biologique préalablement obtenu à partir dudit sujet ; identifier des caractéristiques de liaison d'un ou plusieurs marqueurs dans ledit échantillon avec un ou plusieurs ligands dans la banque ; et déterminer si lesdits un ou plusieurs marqueurs sont associés à ladite réponse induite par un médicament,
dans lequel la banque comprend un composé de formule I, 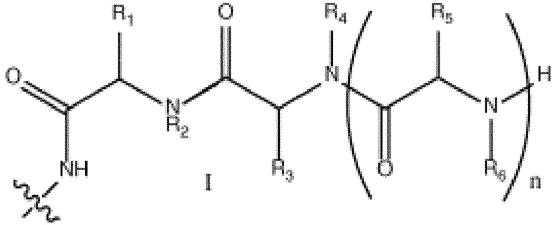 dans lequel
R₁ est -(C₁-C₆)SCH₃ ;
R₂ est H; et
R₃-R₆ sont indépendamment sélectionnés à partir du groupe consistant en H, -C₁-C₆alkyl, -C₁-C₆alkylSCH₃, -C₀-C₆alkylC₂-C₆alkényl, -C₀-C₆alkyl C₂-C₆alkynyl, -C₁-C₆ COOH, -C₁-C₆alkylOH, -C₁-C₆alkylN(R)₂, -C₃-C₈cycloalkyl, -C₁-C₆alkylaryl, -C₁-C₆alkylhétéroaryl, -C₁-C₆alkylNC(O)-C₁-C₆alkyl, -C₁-C₆alkylcycloamide dans lequel un quelconque des groupes aryl ou hétéroaryl peut être indépendamment substitué par - OH, Cl, F, Br, -OCH₃, -SO₂NH₂ ou -O-CH₂-O-,
dans lequel ladite réponse induite par un médicament est une réaction indésirable audit médicament, un effet secondaire dudit médicament ou une résistance audit médicament, diagnostiquant ainsi ladite réponse induite par un médicament chez ledit sujet, et
dans lequel n va de 3 à 11.

2. Procédé selon la revendication 1, dans lequel la banque comprend un composé de formule I, 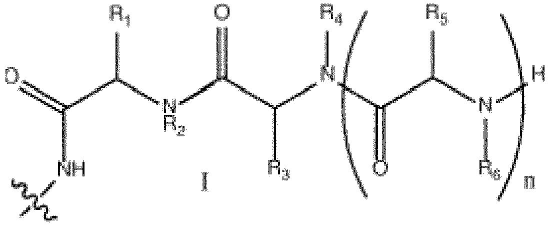 dans lequel les composés sont produits par un procédé qui comprend l'utilisation d'un réactif choisi dans le groupe formé de
(A) furfurylamine ; 3,4-diméthoxyéthanolamine ; benzylamine ; N-(2- aminoéthyl)acétamide ; N-(3-aminopropyl)-2-pyrrolidinone ; éthanolamine ; glycine ; diaminobutane ; allylamine ; pipéronylamine ; méthylbenzylamine ; isobutylamine ;4-(2- aminoéthyl)benzènesulfonamide or cyclohexylamine ; ou
(B) méthoxyéthylamine ; pipéronylamine ; cyclohexylamine ; diaminobutane ; méthylbenzylamine ; isobutylamine ; furfurylamine ou 4-(2-aminoéthyl)benzènesulfonamide ; ou
(C) furfurylamine, éthanolamine ; glycine ; diaminobutane ; allylamine ; pipéronylamine ; méthylbenzylamine ; isobutylamine or 4-(2-aminoéthyl)benzènesulfonamide ; ou
(D) furfurylamine ; N-(2- aminoéthyl)acétamide ; N-(3-aminoéthyl)-2-pyrrolidinone ; éthanolamine ; glycine ; diaminobutane ; allylamine ; pipéronylamine ; méthylbenzylamine ; isobutylamine ;4-(2-aminoéthyl)benzènesulfonamide ; ou
(E) Cystéine, glycine, allylamine, éthanolamine, isobutylamine, méthylbenzylamine, pipéronylamine, méthionine, cyclohexylamine, 3,4-diméthoxyphénéthylamine, benzylamine, N-(2-aminoéthyl)acétamide, N-(3-aminopropyl)-2-pyrrolidone, 4-(2-aminoéthyl)benzènesulfonamide et furfurylamine ; et
dans lequel,
R₁ est-(C₁C₆)SCH₃ ;
R₂ est H;
R₃-R₅ sont indépendamment sélectionnés à partir du groupe consistant en H, -C₁-C₆alkyl, -C₁-C₆alkylSCH₃, -C₀-C₆alkylC₂-C₆alkényl, -C₀-C₆alkyl C₂-C₆alkynyl, -C₁-C₆ COOH, -C₁-C₆alkylOH, -C₁-C₆alkylN(R)₂, -C₃-C₈cycloalkyl, -C₁-C₆alkylaryl, -C₁-C₆alkylhétéroaryl, -C₁-C₆alkylNC(O)C₁-C₆alkyl, -C₁-C₆alkylcycloamide dans lequel un quelconque des groupes aryl ou hétéroaryl peut être indépendamment substitué par-OH, Cl, F, Br, -OCH₃, -SO₂NH₂ ou -O-CH₂-O-,
R₄ est sélectionné à partir du groupe consistant en furfuryl ou -(C₁-C₆alkyl)NR₇R₈, et
R₆ est sélectionné à partir du groupe consistant en H, 1-yl-allyl, 1-yl-2-hydroxyéthyl, isobutyl, 1-yl-n-butylamine, méthylbenzyl, pipéronyl, cyclohexyl, 1-yl-2-(3,4-diméthoxyphényl)éthyl, benzyl, 1-yl-2-(acétamide)éthyl, 1-yl-3-2-pyrrolidinone, 1-yl-2-(4-benzènesulfonamide)éthyl ou furfuryl.

3. Procédé selon la revendication 1, dans lequel la banque comprend un composé ayant une formule la 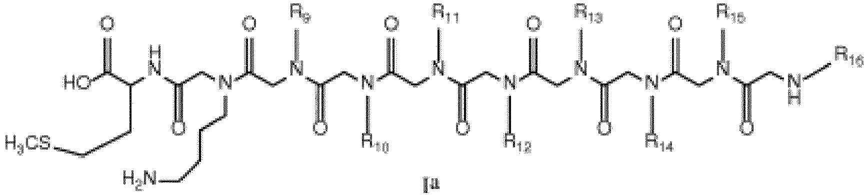 dans lequel le composé est choisi dans le groupe constitué d'un composé de formule la dans lequel,
(a) R₉ est n-butylamine ; R₁₀ est 1-yl-n-butylamine ; R₁₁ est pipéronyl ; R₁₂ est méthylbenzyl ; R₁₃ est pipéronyl ; R₁₄ est méthylbenzyl ; R₁₅ est 1-yl-2-(4(benzènesulfonamide)éthyl et R₁₆ est 1-yl-2-méthoxyéthyl ;
(b) R₉ est 1-yl-n-butylamine ; R₁₀ est 1-yl-2-(4(benzènesulfonamide)éthyl ; R₁₁ est 1-yl-n-butylamine ; R₁₂ est cyclohexyl ; R₁₃ est 1-yl-2-méthyléthyl ; R₁₄ est 1-yl-2,2-diméthyléthyl (isobutyl); R₁₅ est 1-yl-2-(4(benzènesulfonamide)éthyl et R₁₆ est méthylbenzyl ;
(c) R₉ est n-butylamine ; R₁₀ est 1-yl-n-butylamine ; R₁₁ est pipéronyl ; R₁₂ est 1-yl-2-(4(benzènesulfonamide)éthyl ; R₁₃ est 1-yl-n-butylamine ; R₁₄ est méthylbenzyl ; R₁₅ est méthylbenzyl et R₁₆ est cyclohexyl ;
(d) R₉ est pipéronyl ; R₁₀ est 1-yl-n-butylamine ; R₁₁ est isobutyl ; R₁₂ est 1-yl-2-(4(benzènesulfonamide)éthyl ; R₁₃ est méthylbenzyl ; R₁₄ est cyclohexyl ; R₁₅ est isobutyl et R₁₆ est 1-yl-n-butylamine ;
(e) R₉ est 1-yl-n-butylamine ; R₁₀ est 1-yl-n-butylamine ; R₁₁ est méthylbenzyl ; R₁₂ est 1-yl-2-méthoxyéthyl ; R₁₃ est cyclohexyl ; R₁₄ est cyclohexyl ; R₁₅ est méthylbenzyl et R₁₆ est pipéronyl ;
(f) R₉ est pipéronyl ; R₁₀ est 1-yl-n-butylamine ; R₁₁ est isopropyl ; R₁₂ est isopropyl ; R₁₃ est 1-yl-2-méthoxyéthyl ; R₁₄ est cyclohexyl ; R₁₅ est 1-yl-2-(4(benzènesulfonamide)éthyl et R₁₆ est 1-yl-2-(4(benzènesulfonamide)éthyl ;
(g) R₉ est 1-yl-n-butylamine ; R₁₀ est 1-yl-n-butylamine ; R₁₁ est pipéronyl ; R₁₂ est méthylbenzyl ; R₁₃ est pipéronyl ; R₁₄ est méthylbenzyl ; R₁₅ est 1-yl-2-(4(benzènesulfonamide)éthyl et R₁₆ est cyclohexyl ;
(h) R₉ est 1-yl-n-butylamine ; R₁₀ est 1-yl-n-butylamine ; R₁₁ est méthylbenzyl ; R₁₂ est 1-yl-2-méthoxyéthyl ; R₁₃ est cyclohexyl ; R₁₄ est cyclohexyl ; R₁₅ est méthylbenzyl et R₁₆ est pipéronyl ;
(i) R₉ est 1-yl-n-butylamine ; R₁₀ est méthylbenzyl ; R₁₁ est méthylbenzyl ; R₁₂ est 1-yl--n-butylamine ; R₁₃ est 1-yl-n-butylamine ; R₁₄ est cyclohexyl ; R₁₅ est 1-yl-2-(4(benzènesulfonamide)éthyl et R₁₆ est cyclohexyl ;
(j) R₉ est 1-yl-2-(4(benzènesulfonamide)éthyl ; R₁₀ est méthylbenzyl ; R₁₁ est méthylbenzyl ; R₁₂ est cyclohexyl ; R₁₃ est 1-yl-n-butylamine ; R₁₄ est méthylbenzyl ; R₁₅ est isobutyl et R₁₆ est 1-yl-n-butylamine ;
(k) R₅ est 1-yl-2-méthoxyéthyl ; R₁₀ est isobutyl ; R₁₁ est 1-yl-2-(4(benzènesulfonamide)éthyl ; R₁₂ est pipéronyl ; R₁₃ est 1-yl-n-butylamine ; R₁₄ est cyclohexyl ; R₁₅ est méthylbenzyl et R₁₆ est 1-yl-2-méthoxyéthyl ;
(I) R₉ est 1-yl-n-butylamine ; R₁₀ est 1-yl-2-(4(benzènesulfonamide)éthyl ; R₁₁ est 1-yl-n-butylamine ; R₁₂ est cyclohexyl ; R₁₃ est 1-yl-2-méthoxyéthyl ; R₁₄ est isobutyl ; R₁₅ est 1-yl-2-(4(benzènesulfonamide)éthyl et R₁₆ est méthylbenzyl ;
(m) R₉ est 1-yl-2-méthoxyéthyl ; R₁₀ est 1-yl-n-butylamine ; R₁₁ est 1-yl-n-butylamine ; R₁₂ est 1-yl-2-méthoxyéthyl ; R₁₃ est méthylbenzyl ; R₁₄ est 1-yl-n-butylamine ; R₁₅ est furfuryl et R₁₆ est furfuryl ;
(n) R₉ est cyclohexyl ; R₁₀ est cyclohexyl ; R₁₁ est 1-yl-n-butylamine ; R₁₂ est furfuryl ; R₁₃ est 1-yl-2-méthoxyéthyl ; R₁₄ est 1-yl-2-méthoxyéthyl ; R₁₅ est 1-yl-2-(4(benzènesulfonamide)éthyl et R₁₆ est furfuryl ;
(o) R₉ est 1-yl-n-butylamine ; R₁₀ est pipéronyl ; R₁₁ est 1-yl-2-(4(benzènesulfonamide)éthyl ; R₁₂ est 1-yl-2-méthoxyéthyl ; R₁₃ est méthylbenzyl ; R₁₄ est 1-yl-n-butylamine ; R₁₅ est 1-yl-2-méthoxyéthyl et R₁₆ est méthylbenzyl ;
(p) R₉ est cyclohexyl ; R₁₀ est cyclohexyl ; R₁₁ est pipéronyl ; R₁₂ est 1-yl-n-butylamine ; R₁₃ est 1-yl-n-butylamine ; R₁₄ est 1-yl-n-butylamine ; R₁₅ est 1-yl-n-butylamine et R₁₆ est isobutyl ;
(q) R₉ est pipéronyl ; R₁₀ est 1-yl-n-butylamine ; R₁₁ est 1-yl-2-méthoxyéthyl ; R₁₂ est 1-yl-2-(4(benzènesulfonamide)éthyl ; R₁₃ est pipéronyl ; R₁₄ est 1-yl-n-butylamine ; R₁₅ est méthylbenzyl et R₁₆ est méthylbenzyl ;
(r) R₉ est méthylbenzyl ; R₁₀ est méthylbenzyl ; R₁₁ est méthylbenzyl ; R₁₂ est 1-yl-n-butylamine ; R₁₃ est pipéronyl ; R₁₄ est 1-yl-n-butylamine ; R₁₅ est pipéronyl et R₁₆ est 1-yl-n-butylamine ;
(s) R₉ est 1-yl-n-butylamine ; R₁₀ est 1-yl-n-butylamine ; R₁₁ est méthylbenzyl ; R₁₂ est 1-yl-n-butylamine ; R₁₃ est méthylbenzyl ; R₁₄ est méthylbenzyl ; R₁₅ est 1-yl-2-méthoxyéthyl et R₁₆ est pipéronyl;
(t) R₉ est méthylbenzyl ; R₁₀ est 1-yl-2-(4(benzènesulfonamide)éthyl ; R₁₁ est 1-yl-n-butylamine ; R₁₂ est 1-yl-2-(4(benzènesulfonamide)éthyl ; R₁₃ est isobutyl ; R₁₄ est 1-yl-n-butylamine ; R₁₅ est méthylbenzyl et R₁₆ est 1-yl-2-méthoxyéthyl ;
(u) R₉ est 1-yl-2-méthoxyéthyl ; R₁₀ est 1-yl-n-butylamine ; R₁₁ est isobutyl ; R₁₂ est isobutyl ; R₁₃ est cyclohexyl ; R₁₄ est 1-yl-n-butylamine ; R₁₅ est 1-yl-2-(4(benzènesulfonamide)éthyl et R₁₆ est cyclohexyl ;
(v) R₉ est isobutyl ; R₁₀ est 1-yl-n-butylamine ; R₁₁ est 1-yl-n-butylamine ; R₁₂ est méthylbenzyl ; R₁₃ est 1-yl-n-butylamine ; R₁₄ est 1-yl-n-butylamine ; R₁₅ est pipéronyl et R₁₆ est pipéronyl ;
(w) R₉ est 1-yl-2-(4(benzènesulfonamide)éthyl ; R₁₀ est isobutyl ; R₁₁ est méthylbenzyl ; R₁₂ est 1-yl-2-méthoxyéthyl ; R₁₃ est 1-yl-2-(4(benzènesulfonamide)éthyl ; R₁₄ est isobutyl ; R₁₅ est 1-yl-2-méthoxyéthyl et R₁₆ est cyclohexyl ;
(x) R₉ est furfuryl ; R₁₀ est furfuryl ; R₁₁ est pipéronyl ; R₁₂ est cyclohexyl ; R₁₃ est pipéronyl ; R₁₄ est 1-yl-n-butylamine ; R₁₅ est 1-yl-2-(4(benzènesulfonamide)éthyl et R₁₆ est cyclohexyl ;
(y) R₉ est pipéronyl ; R₁₀ est pipéronyl ; R₁₁ est 1-yl-2-méthoxyéthyl ; R₁₂ est 1-yl-2-méthoxyéthyl ; R₁₃ est 1-yl-n-butylamine ; R₁₄ est 1-yl-n-butylamine ; R₁₅ est 1-yl-n-butylamine et R₁₆ est 1-yl-2-méthoxyéthyl ;
(z) R₉ est pipéronyl ; R₁₀ est 1-yl-n-butylamine ; R₁₁ est isobutyl ; R₁₂ est 1-yl-2-(4(benzènesulfonamide)éthyl ; R₁₃ est méthylbenzyl ; R₁₄ est cyclohexyl ; R₁₅ est isobutyl et R₁₆ est 1-yl-n-butylamine ;
(aa) R₉ est 1-yl-2-(4(benzènesulfonamide)éthyl ; R₁₀ est 1-yl-2- (4(benzènesulfonamide)éthyl ; R₁₁ est méthylbenzyl ; R₁₂ est méthylbenzyl ; R₁₃ est 1-yl-n-butylamine ; R₁₄ est 1-yl-n-butylamine ; R₁₅ est 1-yl-2-(4(benzènesulfonamide)éthyl et R₁₆ est 1-yl-2- (4(benzènesulfonamide)éthyl ;
(bb) R₉ est 1-yl-n-butylamine ; R₁₀ est 1-yl-2-méthoxyéthyl ; R₁₁ est 1-yl-n-butylamine ; R₁₂ est isobutyl ; R₁₃ est cyclohexyl ; R₁₄ est 1-yl-n-butylamine ; R₁₅ est 1-yl-n-butylamine et R₁₆ est pipéronyl ;
(cc) R₉ est cyclohexyl ; R₁₀ est méthylbenzyl ; R₁₁ est cyclohexyl ; R₁₂ est pipéronyl ; R₁₃ est 1-yl-n-butylamine ; R₁₄ est 1-yl-2-(4(benzènesulfonamide)éthyl ; R₁₅ est 1-yl-n-butylamine et R₁₆ est 1-yl-2-méthoxyéthyl ;
(dd) R₉ est 1-yl-2-méthoxyéthyl ; R₁₀ est 1-yl-2-(4(benzènesulfonamide)éthyl ; R₁₁ est 1-yl-n-butylamine ; R₁₂ est 1-yl-2-méthoxyéthyl ; R₁₃ est 1-yl-2-(4(benzènesulfonamide)éthyl ; R₁₄ est 1-yl-2-méthoxyéthyl ; R₁₅ est isobutyl et R₁₆ est cyclohexyl ;
(ee) R₉ est 1-yl-2-méthoxyéthyl ; R₁₀ est méthylbenzyl ; R₁₁ est 1-yl-n-butylamine ; R₁₂ est 1-yl-n-butylamine ; R₁₃ est pipéronyl ; R₁₄ est isobutyl ; R₁₅ est 1-yl-2-(4(benzènesulfonamide)éthyl et R₁₆ est 1-yl-n-butylamine
(ff) R₉ est 1-yl-n-butylamine ; R₁₀ est méthylbenzyl ; R₁₁ est 1-yl-2-(4(benzènesulfonamide)éthyl ; R₁₂ est méthylbenzyl ; R₁₃ est 1-yl-n-butylamine ; R₁₄ est 1-yl-2-(4(benzènesulfonamide)éthyl ; R₁₅ est 1-yl-2-(4(benzènesulfonamide)éthyl et R₁₆ est cyclohexyl ;
(gg) R₉ est 1-yl-n-butylamine ; R₁₀ est 1-yl-2-méthoxyéthyl ; R₁₁ est 1-yl-n-butylamine ; R₁₂ est 1-yl-2-(4(benzènesulfonamide)éthyl ; R₁₃ est 1-yl-2-méthoxyéthyl ; R₁₄ est 1-yl-2-méthoxyéthyl ; R₁₅ est 1-yl-n-butylamine et R₁₆ est méthylbenzyl ;
(hh) R₉ est cyclohexyl ; R₁₀ est cyclohexyl ; R₁₁ est méthylbenzyl ; R₁₂ est 1-yl-n-butylamine ; R₁₃ est méthylbenzyl ; R₁₄ est cyclohexyl ; R₁₅ est méthylbenzyl et R₁₆ est 1-yl-n-butylamine ;
(ii) R₉ est 1-yl-n-butylamine ; R₁₀ est furfuryl ; R₁₁ est méthylbenzyl ; R₁₂ est 1-yl-2-(4(benzènesulfonamide)éthyl ; R₁₃ est furfuryl ; R₁₄ est cyclohexyl ; R₁₅ est méthylbenzyl et R₁₆ est cyclohexyl ;
(jj) R₉ est 1-yl-n-butylamine ; R₁₀ est méthylbenzyl ; R₁₁ est 1-yl-n-butylamine ; R₁₂ est 1-yl-2-(4(benzènesulfonamide)éthyl ; R₁₃ est 1-yl-2-méthoxyéthyl ; R₁₄ est méthylbenzyl ; R₁₅ est 1-yl-2-méthoxyéthyl et R₁₆ est isobutyl ;
(kk) R₉ est 1-yl-2-(4(benzènesulfonamide)éthyl ; R₁₀ est 1-yl-n-butylamine ; R₁₁ est 1-yl-n-butylamine ; R₁₂ est méthylbenzyl ; R₁₃ est méthylbenzyl ; R₁₄ est cyclohexyl ; R₁₅ est 1-yl-2-(4(benzènesulfonamide)éthyl et R₁₆ est méthylbenzyl ;
(ll) R₉ est 1-yl-2-(4(benzènesulfonamide)éthyl ; R₁₀ est 1-yl-2- (4(benzènesulfonamide)éthyl ; R₁₁ est 1-yl-n-butylamine ; R₁₂ est 1-yl-n-butylamine ; R₁₃ est méthylbenzyl ; R₁₄ est 1-yl-n-butylamine ; R₁₅ est méthylbenzyl et R₁₆ est 1-yl-n-butylamine ;
(mm) R₉ est 1-yl-n-butylamine ; R₁₀ est 1-yl-2-(4(benzènesulfonamide)éthyl ; R₁₁ est 1-yl-2-(4(benzènesulfonamide)éthyl ; R₁₂ est 1-yl-2-méthoxyéthyl ; R₁₃ est 1-yl-n-butylamine ; R₁₄ est cyclohexyl ; R₁₅ est 1-yl-2-(4(benzènesulfonamide)éthyl et R₁₆ est méthylbenzyl ;
(nn) R₉ est n-butylamine ; R₁₀ est 1-yl-n-butylamine ; R₁₁ est pipéronyl ; R₁₂ est 1-yl-2-(4(benzènesulfonamide)éthyl ; R₁₃ est 1-yl-n-butylamine ; R₁₄ est méthylbenzyl ; R₁₅ est méthylbenzyl et R₁₆ est cyclohexyl ;
(oo) R₉ est 1-yl-2-(4(benzènesulfonamide)éthyl ; R₁₀ est méthylbenzyl ; R₁₁ est méthylbenzyl ; R₁₂ est 1-yl-n-butylamine ; R₁₃ est méthylbenzyl ; R₁₄ est pipéronyl ; R₁₅ est 1-yl-n-butylamine et R₁₆ est 1-yl-2-méthoxyéthyl ;
(pp) R₉ est pipéronyl ; R₁₀ est 1-yl-n-butylamine ; R₁₁ est 1-yl-n-butylamine ; R₁₂ est méthylamine ; R₁₃ est pipéronyl ; R₁₄ est 1-yl-n-butylamine ; R₁₅ est pipéronyl et R₁₆ est 1-yl-2-méthoxyéthyl ;
(qq) R₉ est cyclohexyl ; R₁₀ est cyclohexyl ; R₁₁ est furfuryl ; R₁₂ est 1-yl-2-méthoxyéthyl ; R₁₃ est isobutyl ; R₁₄ est cyclohexyl ; R₁₅ est méthylbenzyl et R₁₆ est méthylbenzyl ;
(rr) R₉ est pipéronyl ; R₁₀ est 1-yl-n-butylamine ; R₁₁ est isobutyl ; R₁₂ est isobutyl ; R₁₃ est 1-yl-2-méthoxyéthyl ; R₁₄ est cyclohexyl ; R₁₅ est 1-yl-2-(4(benzènesulfonamide)éthyl et R₁₆ est 1-yl-2-(4(benzènesulfonamide)éthyl ;
(ss) R₉ est cyclohexyl ; R₁₀ est cyclohexyl ; R₁₁ est 1-yl-n-butylamine ; R₁₂ est méthylbenzyl ; R₁₃ est 1-yl-n-butylamine ; R₁₄ est méthylbenzyl ; R₁₅ est cyclohexyl et R₁₆ est pipéronyl ;
et les sels de ceux-ci acceptables d'un point de vue pharmaceutique.

4. Procédé selon la revendication 1, dans lequel la banque comprend un composé de formule : 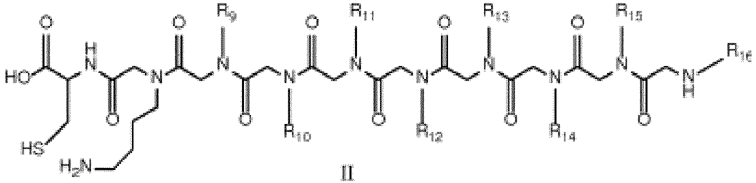 dans lequel le composé est choisi dans le groupe constitué d'un composé de formule II dans lequel,
(a) R₉ est n-butylamine ; R₁₀ est 1-yl-n-butylamine ; R₁₁ est pipéronyl ; R₁₂ est méthylbenzyl ; R₁₃ est pipéronyl ; R₁₄ est méthylbenzyl ; R₁₅ est 1-yl-2-(4(benzènesulfonamide)éthyl et R₁₆ est 1-yl-2-méthoxyéthyl ;
(b) R₉ est 1-yl-n-butylamine ; R₁₀ est 1-y|-2-(4(benzènesulfonamide)éthyl ; R₁₁ est 1-yl-n-butylamine ; R₁₂ est cyclohexyl ; R₁₃ est 1-yl-2-méthoxyéthyl ; R₁₄ est 1-yl-2,2-diméthyléthyl (isobutyl); R₁₅ est 1-yl-2-(4(benzènesulfonamide)éthyl et R₁₆ est méthylbenzyl ;
(c) R₉ est n-butylamine ; R₁₀ est 1-yl-n-butylamine ; R₁₁ est pipéronyl ; R₁₂ est 1-yl-2-(4(benzènesulfonamide)éthyl ; R₁₃ est 1-yl-n-butylamine ; R₁₄ est méthylbenzyl ; R₁₅ est méthylbenzyl et R₁₆ est cyclohexyl ;
(d) R₉ est pipéronyl ; R₁₀ est 1-yl-n-butylamine ; R₁₁ est isobutyl ; R₁₂ est 1-yl-2-(4(benzènesulfonamide)éthyl ; R₁₃ est méthylbenzyl ; R₁₄ est cyclohexyl ; R₁₅ est isobutyl et R₁₆ est 1-yl-n-butylamine ;
(e) R₉ est 1-yl-n-butylamine ; R₁₀ est 1-yl-n-butylamine ; R₁₁ est méthylbenzyl ; R₁₂ est 1-yl-2-méthoxyéthyl ; R₁₃ est cyclohexyl ; R₁₄ est cyclohexyl ; R₁₅ est méthylbenzyl et R₁₆ est pipéronyl ;
(f) R₉ est pipéronyl ; R₁₀ est 1-yl-n-butylamine ; R₁₁ est isopropyl ; R₁₂ est isopropyl ; R₁₃ est 1-yl-2-méthoxyéthyl ; R₁₄ est cyclohexyl ; R₁₅ est 1-yl-2-(4(benzènesulfonamide)éthyl et R₁₆ est 1-yl-2-(4(benzènesulfonamide)éthyl ;
(g) R₉ est 1-yl-n-butylamine ; R₁₀ est 1-yl-n-butylamine ; R₁₁ est pipéronyl ; R₁₂ est méthylbenzyl ; R₁₃ est pipéronyl ; R₁₄ est méthylbenzyl ; R₁₅ est 1-yl-2-(4(benzènesulfonamide)éthyl et R₁₆ est cyclohexyl ;
(h) R₉ est 1-yl-n-butylamine ; R₁₀ est 1-yl-n-butylamine ; R₁₁ est méthylbenzyl ; R₁₂ est 1-yl-2-méthoxyéthyl ; R₁₃ est cyclohexyl ; R₁₄ est cyclohexyl ; R₁₅ est méthylbenzyl et R₁₆ est pipéronyl ;
(i) R₉ est 1-yl-n-butylamine ; R₁₀ est méthylbenzyl ; R₁₁ est méthylbenzyl ; R₁₂ est 1-yl--n-butylamine ; R₁₃ est 1-yl-n-butylamine ; R₁₄ est cyclohexyl ; R₁₅ est 1-yl-2-(4(benzènesulfonamide)éthyl et R₁₆ est cyclohexyl ;
(j) R₉ est 1-yl-2-(4(benzènesulfonamide)éthyl ; R₁₀ est méthylbenzyl ; R₁₁ est méthylbenzyl ; R₁₂ est cyclohexyl ; R₁₃ est 1-yl-n-butylamine ; R₁₄ est méthylbenzyl ; R₁₅ est isobutyl et R₁₆ est 1-yl-n-butylamine ;
(k) R₉ est 1-yl-2-méthoxyéthyl ; R₁₀ est isobutyl ; R₁₁ est 1-yl-2-(4(benzènesulfonamide)éthyl ; R₁₂ est pipéronyl ; R₁₃ est 1-yl-n-butylamine ; R₁₄ est cyclohexyl ; R₁₅ est méthylbenzyl et R₁₆ est 1-yl-2-méthoxyéthyl ;
(I) R₉ est 1-yl-n-butylamine ; R₁₀ est 1-yl-2-(4(benzènesulfonamide)éthyl ; R₁₁ est 1-yl-n-butylamine ; R₁₂ est cyclohexyl ; R₁₃ est 1-yl-2-méthoxyéthyl ; R₁₄ est isobutyl ; R₁₅ est 1-yl-2-(4(benzènesulfonamide)éthyl et R₁₆ est méthylbenzyl ;
(m) R₉ est 1-yl-2-méthoxyéthyl ; R₁₀ est 1-yl-n-butylamine ; R₁₁ est 1-yl-n-butylamine ; R₁₂ est 1-yl-2-méthoxyéthyl ; R₁₃ est méthylbenzyl ; R₁₄ est 1-yl-n-butylamine ; R₁₅ est furfuryl et R₁₆ est furfuryl ;
(n) R₉ est cyclohexyl ; R₁₀ est cyclohexyl ; R₁₁ est 1-yl-n-butylamine ; R₁₂ est furfuryl ; R₁₃ est 1-yl-2-méthoxyéthyl ; R₁₄ est 1-yl-2-méthoxyéthyl ; R₁₅ est 1-yl-2-(4(benzènesulfonamide)éthyl et R₁₆ est furfuryl ;
(o) R₉ est 1-yl-n-butylamine ; R₁₀ est pipéronyl ; R₁₁ est 1-yl-2-(4(benzènesulfonamide)éthyl ; R₁₂ est 1-yl-2-méthoxyéthyl ; R₁₃ est méthylbenzyl ; R₁₄ est 1-yl-n-butylamine ; R₁₅ est 1-yl-2-méthoxyéthyl et R₁₆ est méthylbenzyl ;
(p) R₉ est cyclohexyl ; R₁₀ est cyclohexyl ; R₁₁ est pipéronyl ; R₁₂ est 1-yl-n-butylamine ; R₁₃ est 1-yl-n-butylamine ; R₁₄ est 1-yl-n-butylamine ; R₁₅ est 1-yl-n-butylamine et R₁₆ est isobutyl ;
(q) R₉ est pipéronyl ; R₁₀ est 1-yl-n-butylamine ; R₁₁ est 1-yl-2-méthoxyéthyl ; R₁₂ est 1-yl-2-(4(benzènesulfonamide)éthyl ; R₁₃ est pipéronyl ; R₁₄ est 1-yl-n-butylamine ; R₁₅ est méthylbenzyl et R₁₆ est méthylbenzyl ;
(r) R₉ est méthylbenzyl ; R₁₀ est méthylbenzyl ; R₁₁ est méthylbenzyl ; R₁₂ est 1-yl-n-butylamine ; R₁₃ est pipéronyl ; R₁₄ est 1-yl-n-butylamine ; R₁₅ est pipéronyl et R₁₆ est 1-yl-n-butylamine ;
(s) R₉ est 1-yl-n-butylamine ; R₁₀ est 1-yl-n-butylamine ; R₁₁ est méthylbenzyl ; R₁₂ est 1-yl-n-butylamine ; R₁₃ est méthylbenzyl ; R₁₄ est méthylbenzyl ; R₁₅ est 1-yl-2-méthoxyéthyl et R₁₆ est pipéronyl ;
(t) R₉ est méthylbenzyl ; R₁₀ est 1-yl-2-(4(benzènesulfonamide)éthyl ; R₁₁ est 1-yl-n-butylamine ; R₁₂ est 1-yl-2-(4(benzènesulfonamide)éthyl ; R₁₃ est isobutyl ; R₁₄ est 1-yl-n-butylamine ; R₁₅ est méthylbenzyl et R₁₆ est 1-yl-2-méthoxyéthyl ;
(u) R₉ est 1-yl-2-méthoxyéthyl ; R₁₀ est 1-yl-n-butylamine ; R₁₁ est isobutyl ; R₁₂ est isobutyl ; R₁₃ est cyclohexyl ; R₁₄ est 1-yl-n-butylamine ; R₁₅ est 1-yl-2-(4(benzènesulfonamide)éthyl et R₁₆ est cyclohexyl ;
(v) R₉ est isobutyl ; R₁₀ est 1-yl-n-butylamine ; R₁₁ est 1-yl-n-butylamine ; R₁₂ est méthylbenzyl ; R₁₃ est 1-yl-n-butylamine ; R₁₄ est 1-yl-n-butylamine ; R₁₅ est pipéronyl et R₁₆ est pipéronyl ;
(w) R₉ est 1-yl-2-(4(benzènesulfonamide)éthyl ; R₁₀ est isobutyl ; R₁₁ est méthylbenzyl ; R₁₂ est 1-yl-2-méthoxyéthyl ; R₁₃ est 1-yl-2-(4(benzènesulfonamide)éthyl ; R₁₄ est isobutyl ; R₁₅ est 1-yl-2-méthoxyéthyl et R₁₆ est cyclohexyl ;
(x) R₉ est furfuryl ; R₁₀ est furfuryl ; R₁₁ est pipéronyl ; R₁₂ est cyclohexyl ; R₁₃ est pipéronyl ; R₁₄ est 1-yl-n-butylamine ; R₁₅ est 1-yl-2-(4(benzènesulfonamide)éthyl et R₁₆ est cyclohexyl ;
(y) R₉ est pipéronyl ; R₁₀ est pipéronyl ; R₁₁ est 1-yl-2-méthoxyéthyl ; R₁₂ est 1-yl-2-méthoxyéthyl ; R₁₃ est 1-yl-n-butylamine ; R₁₄ est 1-yl-n-butylamine ; R₁₅ est 1-yl-n-butylamine et R₁₆ est 1-yl-2-méthoxyéthyl ;
(z) R₉ est pipéronyl ; R₁₀ est 1-yl-n-butylamine ; R₁₁ est isobutyl ; R₁₂ est 1-yl-2-(4(benzènesulfonamide)éthyl ; R₁₃ est méthylbenzyl ; R₁₄ est cyclohexyl ; R₁₅ est isobutyl et R₁₆ est 1-yl-n-butylamine ;
(aa) R₉ est 1-yl-2-(4(benzènesulfonamide)éthyl ; R₁₀ est 1-yl-2-(4(benzènesulfonamide)éthyl ; R₁₁ est méthylbenzyl ; R₁₂ est méthylbenzyl ; R₁₃ est 1-yl-n-butylamine ; R₁₄ est 1-yl-n-butylamine ; R₁₅ est 1-yl-2-(4(benzènesulfonamide)éthyl et R₁₆ est 1-yl-2-(4(benzènesulfonamide)éthyl ;
(bb) R₉ est 1-yl-n-butylamine ; R₁₀ est 1-yl-2-méthoxyéthyl ; R₁₁ est 1-yl-n-butylamine ; R₁₂ est isobutyl ; R₁₃ est cyclohexyl ; R₁₄ est 1-yl-n-butylamine ; R₁₅ est 1-yl-n-butylamine et R₁₆ est pipéronyl ;
(cc) R₉ est cyclohexyl ; R₁₀ est méthylbenzyl ; R₁₁ est cyclohexyl ; R₁₂ est pipéronyl ; R₁₃ est 1-yl-n-butylamine ; R₁₄ est 1-yl-2-(4(benzènesulfonamide)éthyl ; R₁₅ est 1-yl-n-butylamine et R₁₆ est 1-yl-2-méthoxyéthyl ;
(dd) R₉ est 1-yl-2-méthoxyéthyl ; R₁₀ est 1-yl-2-(4(benzènesulfonamide)éthyl ; R₁₁ est 1-yl-n-butylamine ; R₁₂ est 1-yl-2-méthoxyéthyl ; R₁₃ est 1-yl-2-(4(benzènesulfonamide)éthyl ; R₁₄ est 1-yl-2-méthoxyéthyl ; R₁₅ est isobutyl et R₁₆ est cyclohexyl ;
(ee) R₉ est 1-yl-2-méthoxyéthyl ; R₁₀ est méthylbenzyl ; R₁₁ est 1-yl-n-butylamine ; R₁₂ est 1-yl-n-butylamine ; R₁₃ est pipéronyl ; R₁₄ est isobutyl ; R₁₅ est 1-yl-2-(4(benzènesulfonamide)éthyl et R₁₆ est 1-yl-n-butylamine ;
(ff) R₉ est 1-yl-n-butylamine ; R₁₀ est méthylbenzyl ; R₁₁ est 1-yl-2-(4(benzènesulfonamide)éthyl ; R₁₂ est méthylbenzyl ; R₁₃ est 1-yl-n-butylamine ; R₁₄ est 1-yl-2-(4(benzènesulfonamide)éthyl ; R₁₅ est 1-yl-2-(4(benzènesulfonamide)éthyl et R₁₆ est cyclohexyl ;
(gg) R₉ est 1-yl-n-butylamine ; R₁₀ est 1-yl-2-méthoxyéthyl ; R₁₁ est 1-yl-n-butylamine ; R₁₂ est 1-yl-2-(4(benzènesulfonamide)éthyl ; R₁₃ est 1-yl-2-méthoxyéthyl ; R₁₄ est 1-yl-2-méthoxyéthyl ; R₁₅ est 1-yl-n-butylamine et R₁₆ est méthylbenzyl ;
(hh) R₉ est cyclohexyl ; R₁₀ est cyclohexyl ; R₁₁ est méthylbenzyl ; R₁₂ est 1-yl-n-butylamine ; R₁₃ est méthylbenzyl ; R₁₄ est cyclohexyl ; R₁₅ est méthylbenzyl et R₁₆ est 1-yl-n-butylamine ;
(ii) R₉ est 1-yl-n-butylamine ; R₁₀ est furfuryl ; R₁₁ est méthylbenzyl ; R₁₂ est 1-yl-2-(4(benzènesulfonamide)éthyl ; R₁₃ est furfuryl ; R₁₄ est cyclohexyl ; R₁₅ est méthylbenzyl et R₁₆ est cyclohexyl ;
(jj) R₉ est 1-yl-n-butylamine ; R₁₀ est méthylbenzyl ; R₁₁ est 1-yl-n-butylamine ; R₁₂ est 1-yl-2-(4(benzènesulfonamide)éthyl ; R₁₃ est 1-yl-2-méthoxyéthyl ; R₁₄ est méthylbenzyl ; R₁₅ est 1-yl-2-méthoxyéthyl et R₁₆ est isobutyl ;
(kk) R₉ est 1-yl-2-(4(benzènesulfonamide)éthyl ; R₁₀ est 1-yl-n-butylamine ; R₁₁ est 1-yl-n-butylamine ; R₁₂ est méthylbenzyl ; R₁₃ est méthylbenzyl ; R₁₄ est cyclohexyl ; R₁₅ est 1-yl-2-(4(benzènesulfonamide)éthyl et R₁₆ est méthylbenzyl ;
(II) R₉ est 1-yl-2-(4(benzènesulfonamide)éthyl ; R₁₀ est 1-yl-2-(4(benzènesulfonamide)éthyl ; R₁₁ est 1-yl-n-butylamine ; R₁₂ est 1-yl-n-butylamine ; R₁₃ est méthylbenzyl ; R₁₄ est 1-yl-n-butylamine ; R₁₅ est méthylbenzyl et R₁₆ est 1-yl-n-butylamine ;
(mm) R₉ est 1-yl-n-butylamine ; R₁₀ est 1-yl-2-(4(benzènesulfonamide)éthyl ; R₁₁ est 1-yl-2-(4(benzènesulfonamide)éthyl ; R₁₂ est 1-yl-2-méthoxyéthyl ; R₁₃ est 1-yl-n-butylamine ; R₁₄ est cyclohexyl ; R₁₅ est 1-yl-2-(4(benzènesulfonamide)éthyl et R₁₆ est méthylbenzyl ;
(nn) R₉ est n-butylamine ; R₁₀ est 1-yl-n-butylamine ; R₁₁ est pipéronyl ; R₁₂ est 1-yl-2-(4(benzènesulfonamide)éthyl ; R₁₃ est 1-yl-n-butylamine ; R₁₄ est méthylbenzyl ; R₁₅ est méthylbenzyl et R₁₆ est cyclohexyl ;
(oo) R₉ est 1-yl-2-(4(benzènesulfonamide)éthyl ; R₁₀ est méthylbenzyl ; R₁₁ est méthylbenzyl ; R₁₂ est 1-yl-n-butylamine ; R₁₃ est méthylbenzyl ; R₁₄ est pipéronyl ; R₁₅ est 1-yl-n-butylamine et R₁₆ est 1-yl-2-méthoxyéthyl ;
(pp) R₉ est pipéronyl ; R₁₀ est 1-yl-n-butylamine ; R₁₁ est 1-yl-n-butylamine ; R₁₂ est méthylamine ; R₁₃ est pipéronyl ; R₁₄ est 1-yl-n-butylamine ; R₁₅ est pipéronyl et R₁₆ est 1-yl-2-méthoxyéthyl ;
(qq) R₉ est cyclohexyl ; R₁₀ est cyclohexyl ; R₁₁ est furfuryl ; R₁₂ est 1-yl-2-méthoxyéthyl ; R₁₃ est isobutyl ; R₁₄ est cyclohexyl ; R₁₅ est méthylbenzyl et R₁₆ est méthylbenzyl ;
(rr) R₉ est pipéronyl ; R₁₀ est 1-yl-n-butylamine ; R₁₁ est isobutyl ; R₁₂ est isobutyl ; R₁₃ est 1-yl-2-méthoxyéthyl ; R₁₄ est cyclohexyl ; R₁₅ est 1-yl-2-(4(benzènesulfonamide)éthyl et R₁₆ est 1-yl-2-(4(benzènesulfonamide)éthyl ;
(ss) R₉ est cyclohexyl ; R₁₀ est cyclohexyl ; R₁₁ est 1-yl-n-butylamine ; R₁₂ est méthylbenzyl ; R₁₃ est 1-yl-n-butylamine ; R₁₄ est méthylbenzyl ; R₁₅ est cyclohexyl et R₁₆ est pipéronyl ;
et les sels de ceux-ci acceptables d'un point de vue pharmaceutique.

5. Procédé selon la revendication 1, dans lequel ledit médicament est au moins l'un des médicaments énumérés dans le tableau 1.

6. Procédé selon la revendication 1, comprenant en outre l'étape consistant à déterminer si ledit sujet répond ou non à un traitement ou à une thérapie par ledit médicament.

7. Procédé selon la revendication 1, dans lequel ladite réponse induite par un médicament est associée à une maladie ou à un stade de ladite maladie, ladite maladie étant facultativement un cancer, une maladie auto-immune, une maladie inflammatoire, une maladie infectieuse, une maladie neurodégénérative ou une maladie cardiovasculaire.

8. Procédé selon la revendication 7, dans lequel le cancer est un cancer du sein, un cancer du poumon, un cancer de la prostate, un cancer du col de l'utérus, un cancer des testicules, un cancer de l'ovaire, un cancer de la peau, un cancer du cerveau, un cancer du pancréas, un cancer du foie, un cancer de l'estomac, un cancer du côlon, un cancer du rectum, un cancer de l'oesophage, un lymphome ou une leucémie,
ou dans lequel la maladie auto-immune est un lupus, une myasthénie gravis, une sclérose en plaques, une narcolepsie, une polyarthrite rhumatoïde, une néphrite, une maladie de Chagas, une sclérodermie ou une maladie de Sjogren,
ou dans lequel la maladie infectieuse résulte d'une infection par des virus, des bactéries ou des champignons,
ou dans lequel la maladie neurodégénérative est une maladie d'Alzheimer, une démence ou une maladie de Creutzfeld-Jacob.

9. Procédé selon la revendication 1, dans lequel ladite banque de ligands comprend une pluralité de ligands identifiés dans un criblage initial ou présélectionnés antérieurement sur la base d'une réactivité connue auxdits marqueurs de liaison au ligand.

10. Procédé selon la revendication 1, dans lequel ladite banque de ligands est une banque de ligands à base de billes comprenant une pluralité de billes ayant des ligands liés aux billes.

11. Procédé selon la revendication 1, dans lequel ledit criblage est effectué par un réseau, et dans lequel ledit réseau est éventuellement un microréseau, une lame de microscope, une plaque, une puce ou une population de billes.

12. Procédé selon la revendication 11, dans lequel ledit réseau comprend un substrat solide ayant une surface ; une pluralité de ligands liés audit substrat, chacun desdits ligands comprenant un segment d'ancrage lié de manière stable à la surface du substrat, un segment peptoïde et un segment de liaison reliant et séparant les segments d'ancrage et peptoïde, ou dans lequel ledit réseau comprend environ 1000 et 100 000 ligands distincts.
